# EUROPEAN PATENT SPECIFICATION

(11) **EP 4 310 083 B1**
(45) Date of publication and mention of the grant of the patent: **14.10.2026**
(21) Application number: 22770574.6
(22) Date of filing: 16.03.2022
(51) Int. Cl.: C07D 405/14, C07D 405/04, C07D 493/00, A61K 31/445, A61K 31/4523, A61P 35/00

(54) **FURAN FUSED RING-SUBSTITUTED GLUTARIMIDE COMPOUND**
RINGSUBSTITUIERTE GLUTARIMIDVERBINDUNG MIT KONDENSIERTEM FURAN
COMPOSÉ DE GLUTARIMIDE SUBSTITUÉ PAR UN CYCLE FUSIONNÉ À UN FURANE

(30) Priority: 17.03.2021 CN 202110286500; 25.06.2021 CN 202110712765; 08.11.2021 CN 202111314330; 28.02.2022 CN 202210187905
(43) Date of publication of application: 24.01.2024
(73) Proprietor: Shanghai Qilu Pharmaceutical Research and Development Centre Ltd., Shanghai 201203 (CN)
(72) Inventor: LEI, Maoyi, Shanghai 200131 (CN); LI, Junmiao, Shanghai 200131 (CN); CHEN, Shuhui, Shanghai 200131 (CN)
(74) Representative: Dehns
(86) International application number: PCT/CN2022/081261
(87) International publication number: WO 2022/194221

(56) References cited:
- WO-A1-2020/048546
- WO-A1-2020/048547
- WO-A1-2021/185291
- CN-A- 110 506 039

## Description

### TECHNICAL FIELD

The present disclosure relates to a series of furan fused ring-substituted glutarimide compounds and to the compounds for use in treating prostate cancer, specifically to a compound of formula (II) and a pharmaceutically acceptable salt thereof.

### BACKGROUND

As androgen receptor signaling is known to play a crucial role in the pathogenesis of prostate cancer and is involved in the development of other androgen receptor-positive cancers, inhibition of androgen receptor signaling with an anti-androgen that antagonizes the androgen receptor has been used, or is proposed to be used, in the treatment of prostate cancer.

AR gene encodes the androgen receptor protein, whose ligands are mainly testosterone and dihydrotestosterone. The receptor is widely distributed in most organs and tissues of the body, mediating the biological effects of androgen. Androgen receptor can bind to heat shock protein (Hsp) in the cytoplasm. When androgen binds to an androgen receptor, the receptor is activated, and the heat shock protein dissociates. The androgen receptor forms a dimer which enters the nucleus, binds to androgen response element (ARE) on the DNA, and initiates the transcription and expression of a series of genes downstream of the element, including genes such as prostate-specific antigen (PSA), prostatic acid phosphatase (PAP), and cyclindependent kinase (CDK) inhibitor p21WAF1/CIPI, which eventually leads to cell differentiation and promotes the development of tissues and organs. Androgen has the function of maintaining prostate growth and development. In an androgen-free environment, prostate cells will undergo spontaneous apoptosis, whereas in an environment with normal androgen level, prostate cells can continue to grow and differentiate. Therefore, it has been suggested that the excessive secretion of androgen and the over-responsiveness of androgen receptor contribute to the uninhibited growth of prostate cells, which are risk factors for the development of prostate cancer.

Prostate cancer (PCa) is one of the most commonly diagnosed non-skin cancers among men in the United States. It is the second most common cause of cancer death, with more than 200,000 new cases and more than 30,000 deaths in the United States each year. Androgen deprivation therapy (ADT) is the standard treatment for advanced PCa. Patients with advanced PCa undergo ADT via luteinizing hormone-releasing hormone (LHRH) agonists, LHRH antagonists, or bilateral orchiectomy. Despite an initial response to ADT, disease progression is inevitable and the cancer emerges as castration-resistant prostate cancer (CRPC). Up to 30% of patients with prostate cancer who receive primary treatment with radiation or surgery will develop metastatic diseases within 10 years of primary treatment. Each year, about 50,000 patients will develop metastatic disease, called metastatic CRPC (mCRPC).

Proteolysis targeting chimera (PROTAC) is a technique that applies the ubiquitin-proteasome system to target specific proteins and induce their intracellular degradation. The ubiquitin-proteasome system is the main pathway for intracellular protein degradation, mainly responsible for the removal of denatured, mutated, or harmful proteins from the cell as its normal physiological functions. 80% or more of intracellular protein degradation is dependent on the ubiquitin-proteasome system. PROTAC utilizes the cell's own protein destruction mechanism to remove specific targeted proteins from the cell.

The present disclosure describes compounds, including compositions comprising the same, whose function is to recruit endogenous proteins to E3 ubiquitin ligases such as cereblon (CRBN) E3 ubiquitin ligases for ubiquitination and subsequent degradation, as well as methods of use of the same. In particular, the present disclosure provides a bi-functional or proteolysis targeting chimera (PROTAC) compound, which has been found to act as a modulator of targeted ubiquitination and androgen receptor (AR) degradation.

WO 2021/185291A1 discloses proteolysis targeting chimera (PROTAC), and an application thereof in preparation of a drug for treating a related disease. CN 110506039A discloses bifunctional compounds which find utility to degrade and (inhibit) Androgen Receptor.

### CONTENT OF THE PRESENT INVENTION

The present disclosure provides a compound of formula (II) or a pharmaceutically acceptable salt thereof, 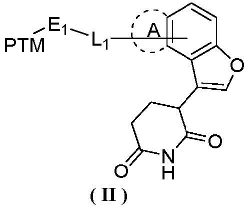 wherein
PTM is selected from 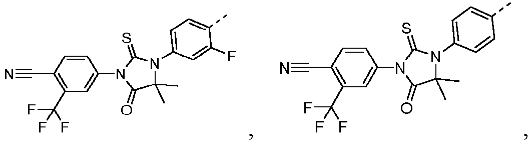 and 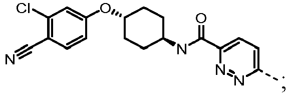
the structural moiety -E₁-L₁- is selected from 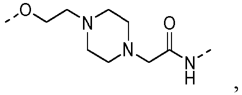 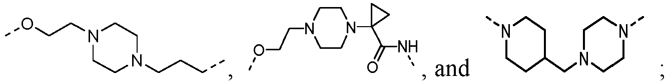
ring A is absent;
or ring A is selected from phenyl and 5-membered heteroaryl;
the 5-membered heteroaryl contains 1, 2, or 3 heteroatoms or heteroatom groups independently selected from -NH-, -O-, -S-, and N, respectively.

In some embodiments of the present disclosure, the structural moiety 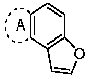 is selected from 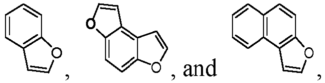 and other variables are as defined in the present disclosure.

There are still some embodiments of the present disclosure which are obtained by any combination of the above variables.

The present disclosure also provides the following compounds or pharmaceutically acceptable salts thereof: 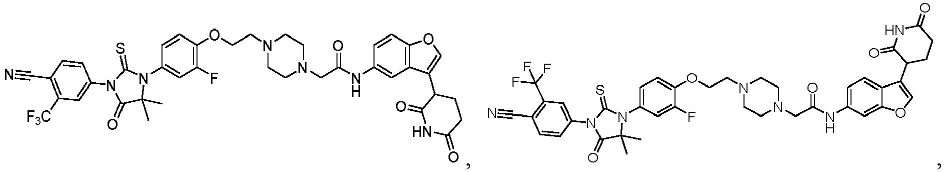 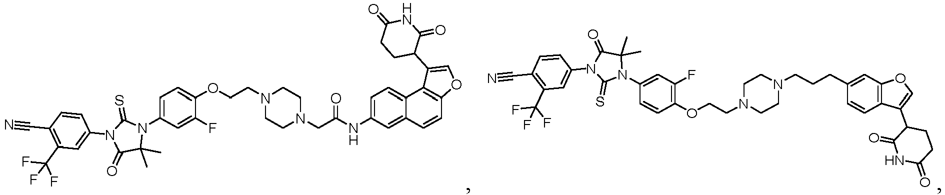 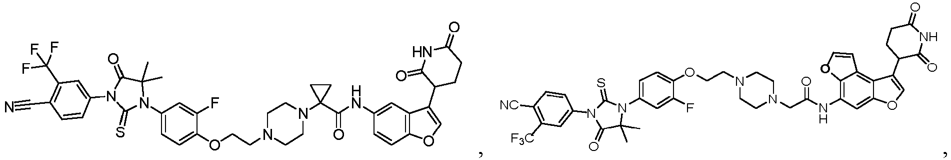 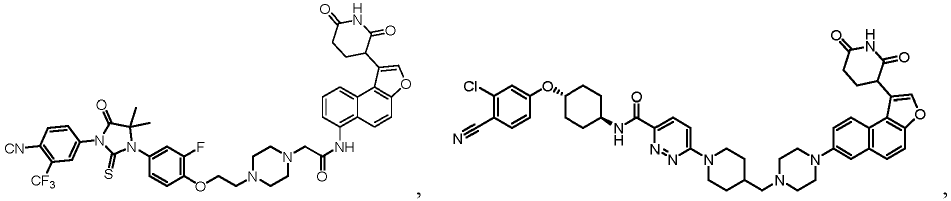 and

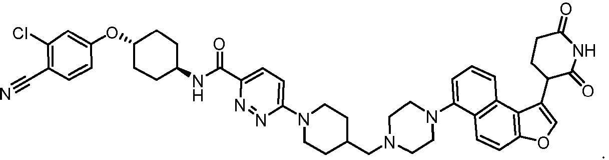

The present disclosure also provides the following compounds or pharmaceutically acceptable salts thereof:

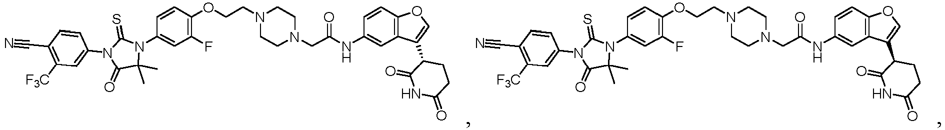

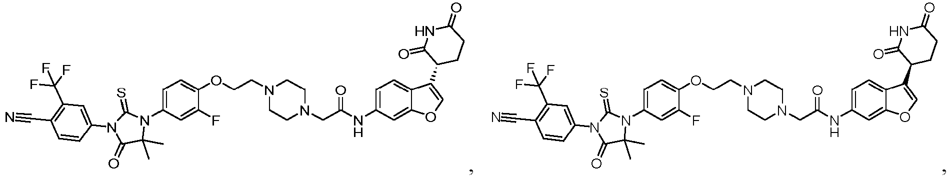

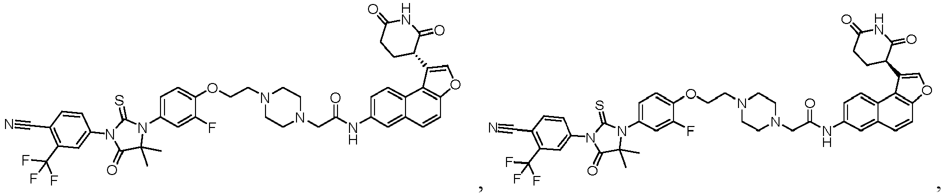

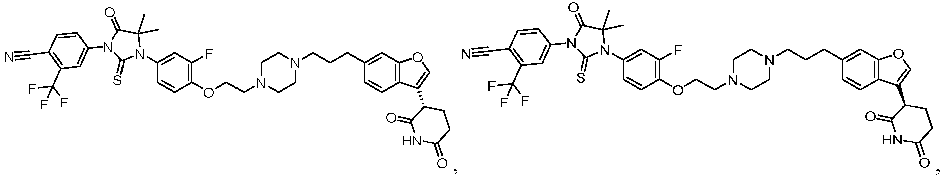

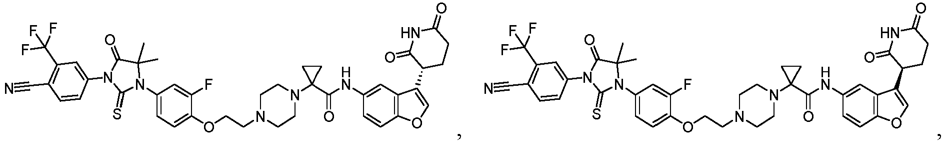

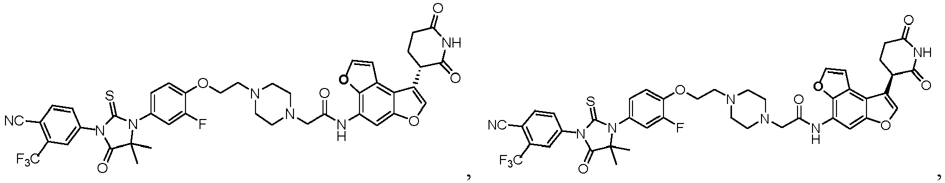

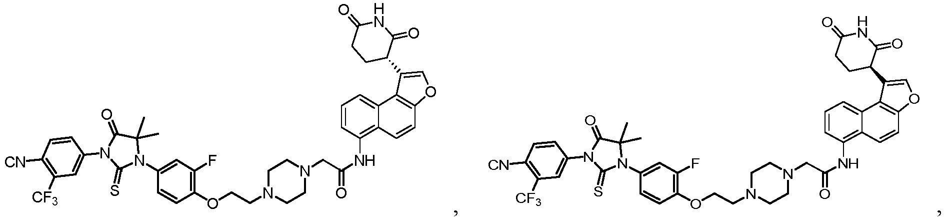

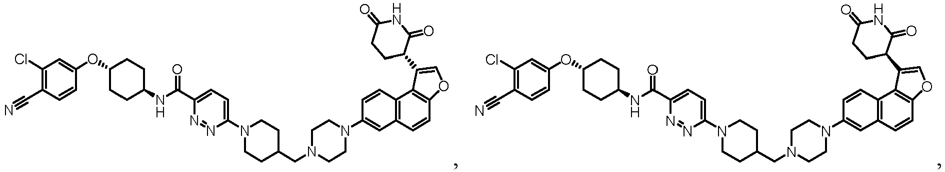

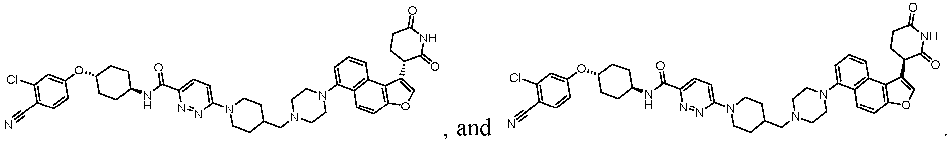

The present disclosure also provides the compound or the pharmaceutically acceptable salt thereof as herein described for use in treating prostate cancer.

### Definition and description

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereomers, (*D*)-isomers, (*L*)-isomers, racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 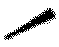 ) and a wedged dashed bond ( 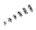 ); in the absence of a stereogenic center in the structure, the relative configuration is represented by the simultaneous occurrence of a wedged solid bond ( 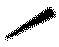 ) and a wedged dashed bond ( 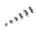 ), for example, trans-1,4-dimethylcyclohexane is represented by

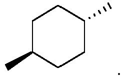

Unless otherwise specified, when a group has one or more linkable sites, any one or more sites of the group can be linked to other groups through chemical bonds. The chemical bond between the site and other groups can be represented by a straight solid bond ( 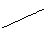 ), a straight dashed bond ( 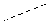 ), or a wavy line ( 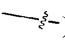 ). For example, the straight solid bond in - OCH₃ means that it is linked to other groups through the oxygen atom in the group; the straight dashed bond in 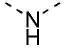 means that it is linked to other groups through the two ends of nitrogen atom in the group; the wave line in 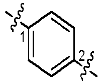 means that the phenyl group is linked to other groups through carbon atoms at position 1 and position 2.

The compounds of the present disclosure may exist in specific forms. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers and *D*- and *L*-isomers can be prepared using chiral synthesis or chiral reagents or other conventional techniques. If one kind of enantiomer of certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine).

The compound of the present disclosure may contain an unnatural proportion of atomic isotope at one or more atoms that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

The term "replaced" means that a specified atom or group can be replaced by another atom or group as specified. For example, CH₂ in CH₃CH₂CH₃ can be replaced by O, S and NH to obtain CH₃OCH₃, CH₃SCH₃, and CH₃NHCH₃.

When any variable (such as R) occurs in the constitution or structure of the compound more than once, the definition of the variable at each occurrence is independent. Thus, for example, if a group is substituted by 0 to 2 R, the group can be optionally substituted with up to two R, wherein the definition of R at each occurrence is independent. Moreover, a combination of the substituent and/or the variant thereof is allowed only when the combination results in a stable compound.

When a substituent is vacant, it means that the substituent is absent, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

Unless otherwise specified, the number of atoms in a ring is usually defined as the number of ring members, for example, "5- to 7-membered ring" refers to a "ring" in which 5 to 7 atoms are arranged around.

Unless otherwise specified, the terms "5-membered heteroaryl ring" and "5-membered heteroaryl" in the present disclosure can be used interchangeably, and the term "5-membered heteroaryl" refers to a monocyclic group consisting of 5 ring atoms with a conjugated π-electron system, wherein 1, 2 or 3 ring atoms are heteroatoms independently selected from O, S and N, and the rest are carbon atoms. Where nitrogen atoms are optionally quaternized, and nitrogen and sulfur heteroatoms can be optionally oxidized (i.e., NO and S(O)ₚ, p is 1 or 2). The 5-membered heteroaryl can be connected to the rest of the molecule through a heteroatom or a carbon atom. Examples of the 5-membered heteroaryl include, but are not limited to, pyrrolyl (including N-pyrrolyl, 2-pyrrolyl, 3-pyrrolyl, etc.), pyrazolyl (including 2-pyrazolyl, 3-pyrazolyl, etc.), imidazolyl (including N-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1H-1,2,3-triazolyl, 2H-1,2,3-triazolyl, 1H-1,2,4-triazolyl, 4H-1,2,4-triazolyl, etc.), tetrazolyl, isoxazolyl (3-isoxazolyl, 4-isoxazolyl, 5-isoxazolyl, etc.), thiazolyl (including 2-thiazolyl , 4-thiazolyl, 5-thiazolyl, etc.), furyl (including 2-furyl, 3-furyl, etc.), thienyl (including 2-thienyl, 3-thienyl, etc.).

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond ( 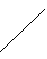 ), a straight dashed bond ( 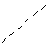 ), or a wavy line ( 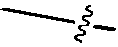 ). For example, the straight solid bond in -OCH₃ means that it is linked to other groups through the oxygen atom in the group; the straight dashed bond in 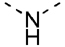 means that it is linked to other groups through the two ends of nitrogen atom in the group; the wave line in 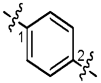 means that the phenyl group is linked to other groups through carbon atoms at position 1 and position 2; 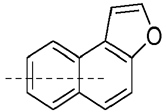 means that it can be linked to other groups through any linkable sites on the naphthalene ring by one chemical bond, including at least six types of linkage, including

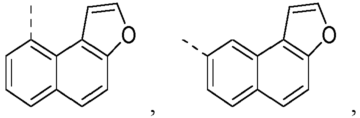

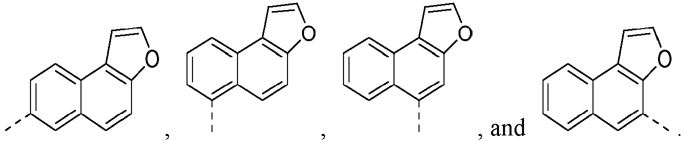

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

The solvent used in the present disclosure is commercially available. The following abbreviations are used in the present disclosure: aq refers to water; eq refers to equivalent or equivalent weight; CDI refers to carbonyldiimidazole; DCM refers to dichloromethane; PE refers to petroleum ether; DMF refers to N,N-dimethylformamide; DMSO refers to dimethyl sulfoxide; EtOAc refers to ethyl acetate; EtOH refers to ethanol; MeOH refers to methanol; Boc refers to tert-butoxycarbonyl, which is a protective group for amino; r.t. refers to room temperature; O/N refers to overnight; THF refers to tetrahydrofuran; Boc₂O refers to di-tert-butyl dicarbonate; M refers to mol/L; HPLC refers to high performance liquid chromatography.

The compounds of the present disclosure are named according to the conventional naming principles in the art or by ChemDraw^{®} software, and the commercially available compounds use the supplier catalog names.

### Technical effect

The compounds of the present disclosure have excellent AR protein degradation effect, cell proliferation inhibition effect, and significant antitumor effect.

### BRIEF DESCRIPTION OF THE DRAWINGS

Figure 1 shows the AR protein degradation activity of enzalutamide and WX001 in cell line LNCaP.
Figure 2 shows the AR protein degradation activity of WX002 in cell line LNCaP.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed.

In the following Reference Examples and Examples, the compounds WX001-WX005, WX008-WX009 and WX014-WX0017 are according to the invention, whereas the other compounds are not according to the invention and are present for illustration purposes only.

### Reference example 1

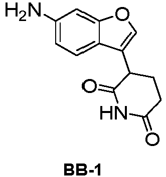

### Synthetic route:

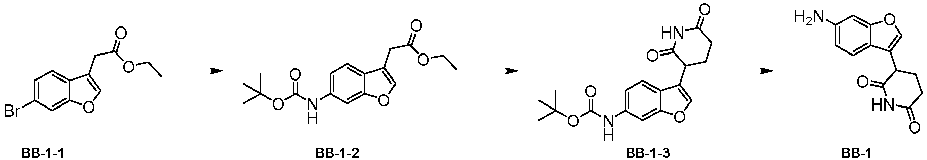

### Step 1: Synthesis of intermediate BB-1-2

To toluene (15 mL) and water (1.5 mL) were added compound BB-1-1 (1 g, 3.53 mmol) and tert-butyl carbamate (2.07 g, 17.66 mmol). To the reaction were slowly added tris(dibenzylideneacetone)dipalladium (226.41 mg, 247.25 µmol), 2-di-tert-butylphosphino-2',4',6'-triisopropylbiphenyl (209.98 mg, 494.49 µmol), and potassium phosphate (3.00 g, 14.13 mmol) successively. The mixture was replaced with nitrogen three times, then heated to 100°C, and stirred for 12 hours. The reaction mixture was cooled to room temperature and evaporated to dryness by rotary evaporation under reduced pressure to remove most of the organic solvent. The residue was diluted with ethyl acetate (30 mL). The organic phase was washed twice with water (30 mL × 2), dried over anhydrous sodium sulfate, filtered, and evaporated to dryness by rotary evaporation under reduced pressure. The resulting residue was separated by column chromatography (eluent: petroleum ether/ethyl acetate = 1/0 to 3/2, v/v) to obtain intermediate BB-1-2. ¹H NMR(400MHz, CDCl₃)8:7.83 - 7.73(m, 1H), 7.57(s, 1H), 7.47 - 7.40(m, 1H), 7.14 - 7.05(m, 1H), 6.81(br d, J=1.0 Hz, 1H), 4.19(q, J=7.0 Hz, 2H), 3.67(d, J=1.3 Hz, 2H), 1.49 - 1.42(m, 9H), 1.32 - 1.23(m, 3H).

### Step 2: Synthesis of intermediate BB-1-3

To tetrahydrofuran (5 mL) were added compound BB-1-2 (300 mg, 939.40 µmol) and acrylamide (73.45 mg, 1.03 mmol, 71.31 µL) at 0°C. To the reaction was slowly added potassium tert-butoxide (158.12 mg, 1.41 mmol). The mixture was replaced with nitrogen three times, and then stirred at 0°C for 1 hour. The reaction mixture was slowly added with saturated ammonium chloride aqueous solution (20 mL) and extracted twice with ethyl acetate (20 mL × 2). The organic phase was washed once with saturated brine (20 mL), dried over anhydrous sodium sulfate, filtered, and evaporated to dryness by rotary evaporation under reduced pressure. The resulting residue was separated by column chromatography (eluent: petroleum ether/ethyl acetate = 1/0 to 3/1, v/v) to obtain intermediate BB-1-3.

### Step 3: Synthesis of hydrochloride of intermediate BB-1

To ethyl acetate (2 mL) was added compound BB-1-3 (40 mg, 116.16 µmol), and then hydrochloric acid/dioxane (4 M, 1 mL) was slowly added dropwise to the reaction. The mixture was replaced with nitrogen three times, and then stirred at 25°C for 12 hours. The reaction mixture was directly evaporated to dryness by rotary evaporation to obtain hydrochloride of intermediate BB-1.

### Reference example 2

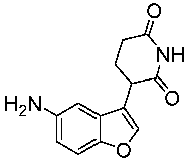

### Synthetic route:

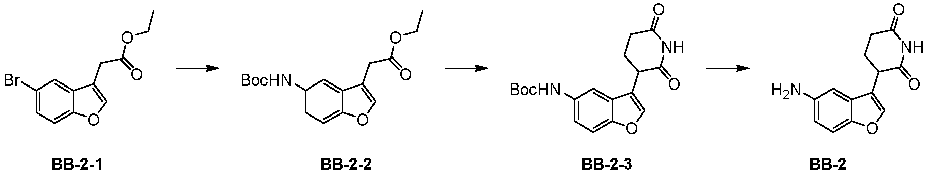

### Step 1: Synthesis of intermediate BB-2-2

To a mixture of toluene (70 mL) and water (10 mL) were added intermediate BB-2-1 (4 g, 14.13 mmol) and tert-butyl carbamate (4.97 g, 42.39 mmol) at room temperature under nitrogen atmosphere, and then added potassium phosphate (12.00 g, 56.51 mmol), tris(dibenzylideneacetone)dipalladium (905.63 mg, 988.99 µmol), and 2-di-tert-butylphosphino-2',4',6'-triisopropylbiphenyl (839.93 mg, 1.98 mmol) successively. The reaction mixture was stirred and reacted at 110°C for 12 hours under nitrogen atmosphere. After the reaction was completed, the reaction mixture was cooled to room temperature and filtered through diatomite. The filtrate was added with water (100 mL) and extracted with ethyl acetate (500 mL × 2). The organic phases were combined, washed with saturated brine (300 mL × 2), dried over anhydrous sodium sulfate, and filtered. The filtrate was concentrated under reduced pressure to remove the solvent, and the resulting residue was separated by column chromatography (eluent: petroleum ether/ethyl acetate = 0/1 to 10/1, v/v) to obtain intermediate BB-2-2. ¹H NMR(400 MHz, CDCl₃)8: 7.71(br s, 1H), 7.63(s, 1H), 7.37(d, J=8.8 Hz, 1H), 7.14(dd, J=2.0, 8.8 Hz, 1H), 6.59(br s, 1H), 4.20(q, J=7.2 Hz, 2H), 3.67(d, J=0.8 Hz, 2H), 1.53(s, 9H), 1.29 (t, J=7.2 Hz, 3H).

### Step 2: Synthesis of intermediate BB-2-3

To tetrahydrofuran (8 mL) were added compound BB-2-2 (0.75 g, 1.69 mmol) and potassium tert-butoxide (284.42 mg, 2.53 mmol). To the reaction mixture was slowly added acrylamide (312.60 mg, 2.03 mmol). The mixture was replaced with nitrogen three times and then stirred at 0 to 5°C for 2 hours. After the reaction was completed, the reaction mixture was slowly added with saturated ammonium chloride aqueous solution (20 mL), and extracted twice with ethyl acetate (20 mL × 2). The organic phases were combined, dried over anhydrous sodium sulfate, filtered, and evaporated to dryness by rotary evaporation under reduced pressure. The resulting residue was separated by column chromatography (eluent: petroleum ether/ethyl acetate = 1/0 to 1/1, v/v) to obtain intermediate BB-2-3. MS-ESI m/z: 245.0 [M+H]⁺.¹H NMR (400MHz, CDCl₃) δ: 8.13 (br s, 1H), 7.65 (br s, 1H), 7.46 (s, 1H), 7.32 (d, J=8.8 Hz, 1H), 7.04 (dd, J=2.1, 8.8 Hz, 1H), 6.57 (br s, 1H), 3.91 (t, J=7.6 Hz, 1H), 2.80 - 2.53 (m, 2H), 2.35 - 2.20 (m, 2H), 1.45 (s, 9H).

### Step 3: Synthesis of hydrochloride of intermediate BB-2

Intermediate BB-2-3 (300 mg, 871.18 µmol) was dissolved in hydrochloric acid/ethyl acetate solution (4 M, 5 mL) at room temperature, and the reaction mixture was stirred and reacted at room temperature for 2 hours. After the reaction was completed, the reaction mixture was concentrated under reduced pressure to remove the solvent to obtain hydrochloride of intermediate BB-2.

¹H NMR (400MHz, DMSO-d6) δ: 10.97 (s, 1H), 10.34 (br s, 2H), 8.05 (s, 1H), 7.72 (d, J=8.8 Hz, 1H), 7.58 (d, J=2.0 Hz, 1H), 7.35 (dd, J=2.1, 8.7 Hz, 1H), 4.20 (dd, J=4.8, 12.3 Hz, 1H), 2.88 - 2.73 (m, 1H), 2.68 - 2.58 (m, 1H), 2.30 (dq, J=4.4, 12.6 Hz, 1H), 2.20 - 2.10 (m, 1H).

### Reference example 3

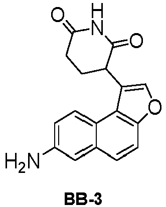

### Synthetic route:

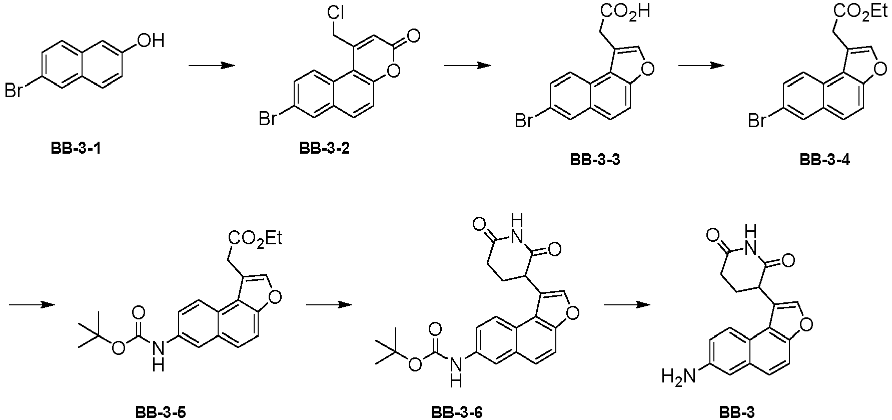

### Step 1: Synthesis of intermediate BB-3-2

To ice water (40 mL) was added dropwise concentrated sulfuric acid (220.80 g, 2.21 mol, 120 mL, purity: 98%) at room temperature under nitrogen atmosphere, then added compound BB-3-1 (10 g, 44.83 mmol), cooled to 5 to 10 °C, and ethyl 4-chloroacetoacetate (7.38 g, 44.83 mmol) was added slowly dropwise thereto. The reaction mixture was warmed to room temperature and stirred for 16 hours, then warmed to 50°C, and continued to be stirred for 16 hours. After the reaction was completed, the reaction mixture was cooled to room temperature and poured into ice water (1 L). A large amount of solid was precipitated and filtered to collect the filter cake. The resulting solid was added with toluene (400 mL), concentrated under reduced pressure to remove the solvent, then added with toluene (400 mL), and concentrated under reduced pressure again to remove the solvent to obtain intermediate BB-3-2.

### Step 2: Synthesis of intermediate BB-3-3

Intermediate BB-3-2 (14.5 g, 44.81 mmol) was dissolved in a solution of sodium hydroxide (8.70 g, 217.52 mmol) in water (150 mL) at room temperature under nitrogen atmosphere. The reaction mixture was heated to 80°C, stirred, and reacted for 5 hours. After the reaction was completed, the reaction mixture was cooled to room temperature and diluted with dichloromethane (150 mL). The organic phase was collected after the phases were separated, and the aqueous phase was extracted with dichloromethane (150 mL × 3). The aqueous phase was added with 2 M dilute hydrochloric acid to adjust the pH to 4 and extracted with ethyl acetate (200 mL × 3). The organic phases were combined, washed with saturated brine (50 mL × 2), dried over anhydrous sodium sulfate, filtered, and the filtrate was concentrated under reduced pressure to remove the solvent to obtain intermediate BB-3-3.

### Step 3: Synthesis of intermediate BB-3-4

Intermediate BB-3-3 (11.3 g, 37.03 mmol) was dissolved in ethanol (300 mL) at room temperature under nitrogen atmosphere, then concentrated sulfuric acid (2.08 g, 20.78 mmol, 1.13 mL, purity: 98%) was added thereto, and the reaction mixture was heated to 80°C and stirred for 12 hours. After the reaction was completed, the reaction mixture was cooled to room temperature, concentrated under reduced pressure to remove the solvent, added with water (150 mL), and extracted with ethyl acetate (150 mL × 1, 100 mL × 3). The organic phases were combined, washed with saturated brine (50 mL × 2), dried over anhydrous sodium sulfate, filtered, and the filtrate was concentrated under reduced pressure to remove the solvent. The resulting residue was separated by column chromatography (eluent: petroleum ether/ethyl acetate = 1/0 to 97/3, v/v) to obtain intermediate BB-3-4. ¹H NMR(400 MHz, DMSO-d6)δ: 8.35(d, J=2.0 Hz, 1H), 8.09(t, J=4.4 Hz, 2H), 7.86(s, 2H), 7.73(dd, J=2.0, 8.8 Hz, 1H), 4.18-4.09(m, 4H), 1.18(t, J=7.2 Hz, 3H).

### Step 4: Synthesis of intermediate BB-3-5

Intermediate BB-3-4 (2.00 g, 6.00 mmol) was dissolved in toluene (60 mL) and water (6 mL) with stirring at room temperature, and then tert-butyl carbamate (3.52 g, 30.01 mmol), tris(dibenzylideneacetone)dipalladium (384.78 mg, 420.20 µmol), 2-di-tert-butylphosphino-2',4',6'-triisopropylbiphenyl (356.87 mg, 840.40 µmol), and potassium phosphate (5.10 g, 20.01 mmol) were added thereto successively. The reaction mixture system was replaced with nitrogen three times, slowly heated to 105°C, and reacted for 12 hours under nitrogen atmosphere. After the reaction was completed, the reaction mixture was cooled to room temperature. The resulting reaction mixture was added with water (50 mL) and extracted with ethyl acetate (50 mL × 3). The organic phases were combined, washed with saturated brine (50 mL × 3) successively, dried over anhydrous sodium sulfate, filtered, and the filtrate was concentrated under reduced pressure to remove the solvent. The resulting residue was purified by column chromatography (eluent: petroleum ether/ethyl acetate = 10/1 to 5/1, v/v) to obtain intermediate BB-3-5.

### Step 5: Synthesis of intermediate BB-3-6

BB-3-5 (4.00 g, 10.83 mmol) was dissolved in N,N-dimethylformamide (60 mL) solvent at 0°C under nitrogen atmosphere, then potassium tert-butoxide (1.22 g, 10.83 mmol) and acrylamide (1.67 g, 10.83 mmol) were added thereto respectively, and the reaction mixture was stirred and reacted at 0°C for 3 hours. After the reaction was completed, the reaction mixture was added with water (50 mL), and extracted with ethyl acetate (100 mL × 3). The organic phases were combined, washed with saturated brine (30 mL × 2) successively, dried over anhydrous sodium sulfate, filtered, and the filtrate was concentrated under reduced pressure to remove the solvent. The resulting residue was purified by column chromatography (eluent: petroleum ether/ethyl acetate = 3/1 to 1/1, v/v) to obtain intermediate BB-3-6. MS-ESI m/z: 338.4 [M+H]⁺. ¹H NMR(400 MHz, DMSO-d6)δ: 10.95(s, 1H), 9.55(s, 1H), 8.25(s, 1H), 7.96(d, J=4.0 Hz, 2H), 7.72(s, 2H), 2.89(s, 3H), 2.74(s, 2H), 1.52(s, 9H).

### Step 6: Synthesis of hydrochloride of intermediate BB-3

BB-3-6 (1.0 g, 2.54 mmol) was dissolved in ethyl acetate (20 mL) at 0°C, then hydrochloric acid/ethyl acetate (2.54 mL) was slowly added thereto, and the reaction mixture was stirred for 4 hours. After the reaction was completed, the reaction mixture was concentrated under reduced pressure to obtain hydrochloride of BB-3. MS-ESI m/z: 294.3 1[M+H]⁺.

### Reference example 4

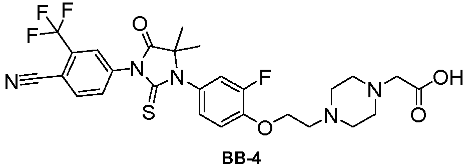

### Synthetic route:

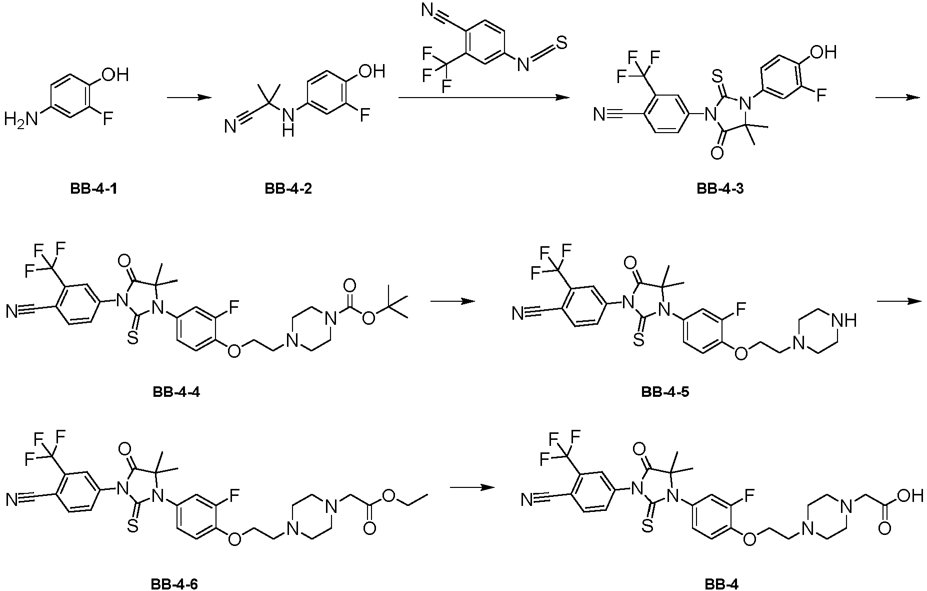

### Step 1: Synthesis of intermediate BB-4-2

Compound BB-4-1 (10 g, 78.67 mmol) was dissolved in dichloromethane (120 mL) and acetone (60 mL) at 0 to 5°C under nitrogen atmosphere, then slowly added dropwise with cyanotrimethylsilane (12.45 g, 125.50 mmol, 15.70 mL) and trimethylsilyl trifluoromethanesulfonate (820.00 mg, 3.69 mmol, 666.67 µL) successively, and the reaction mixture was stirred at 25°C for 2 hours. After the reaction was completed, the reaction mixture was cooled to 0 to 5°C, diluted with water (200 mL), and extracted with ethyl acetate (200 mL × 3). The combined organic phases were washed with saturated brine (100 mL × 2), dried over anhydrous sodium sulfate, filtered, and the filtrate was concentrated under reduced pressure to remove the solvent. The resulting residue was separated by column chromatography (eluent: petroleum ether/ethyl acetate = 10/1 to 5/1, v/v) to obtain compound BB-4-2. H NMR(400MHz, CDCl3)δ: 6.83(t, J=9.0 Hz, 1H), 6.74(dd, J=2.7, 12.1 Hz, 1H), 6.66 - 6.59(m, 1H), 1.65(s, 6H).

### Step 2: Synthesis of compound BB-4-3

Compound BB-4-2 (8 g, 45.40 mmol) was dissolved in *N,N-*dimethylacetamide (150 mL) at room temperature under nitrogen atmosphere. To the reaction mixture was added 4-isothiocyanato-2-(trifluoromethyl)benzonitrile (10.36 g, 45.40 mmol) in batches. The reaction mixture was stirred at 25°C for 12 hours, then added with methanol (60 mL) and dilute hydrochloric acid (2 M, 60 mL), and stirred at 70°C for 3 hours. After the reaction was completed, the reaction mixture was cooled to room temperature, diluted with water (500 mL), and extracted with ethyl acetate (200 mL × 3). The organic phases were combined, washed with saturated brine (100 mL × 2), dried over anhydrous sodium sulfate, filtered, and the filtrate was concentrated under reduced pressure to remove the solvent. The resulting residue was separated by column chromatography (eluent: petroleum ether/ethyl acetate = 3/1 to 1/1, v/v) to obtain compound BB-4-3. ¹H NMR(400MHz, CDCl₃)δ: 8.03 - 7.94(m, 1H), 8.02 - 7.94(m, 1H), 8.05 - 7.92(m, 1H), 8.04 - 7.92(m, 1H), 8.15 - 7.89(m, 1H), 7.86(d, J=1.5 Hz, 1H), 7.82 - 7.79(m, 1H), 7.19 - 7.10(m, 2H), 6.99 - 6.90(m, 2H), 6.06(br s, 1H), 1.58(s, 6H).

### Step 3: Synthesis of compound BB-4-4

Compound BB-4-3 (2 g, 4.72 mmol) was dissolved in N,N-dimethylformamide (50 mL) at room temperature under nitrogen atmosphere, and added with tert-butyl 4-(2-bromoethyl)piperazine-1-carboxylate (1.66 g, 5.67 mmol), potassium carbonate (1.31 g, 9.45 mmol), and potassium iodide (784.17 mg, 4.72 mmol) successively. The reaction mixture was heated to 80°C, stirred, and reacted for 12 hours. After the reaction was completed, the reaction mixture was cooled to room temperature, diluted with water (100 mL), and extracted with ethyl acetate (100 mL × 3). The organic phases were combined, washed with saturated brine (100 mL × 2), dried over anhydrous sodium sulfate, filtered, and the filtrate was concentrated under reduced pressure to remove the solvent. The resulting residue was separated by column chromatography (eluent: petroleum ether/ethyl acetate = 3/1 to 1/1, v/v) to obtain compound BB-4-4. MS-ESI m/z: 636.3 [M+H]⁺.

### Step 4: Synthesis of hydrochloride of compound BB-4-5

Compound BB-4-4 (2.0 g, 3.15 mmol) was dissolved in ethyl acetate (50 mL) at room temperature under nitrogen atmosphere. The reaction mixture was slowly added dropwise with hydrochloric acid/ethyl acetate (4 M, 3.93 mL) and stirred at 25°C for 12 hours. After the reaction was completed, the reaction mixture was concentrated under reduced pressure to obtain hydrochloride of compound BB-4-5. ¹H NMR(400MHz, CD₃OD)δ: 8.23 - 8.13(m, 2H), 8.00(dd, J=1.8, 8.3 Hz, 1H), 7.40(t, J=8.9 Hz, 1H), 7.32(dd, J=2.4, 11.4 Hz, 1H), 7.24(br d, J=8.8 Hz, 1H), 4.69 - 4.60(m, 2H), 3.88 - 3.79(m, 6H), 3.75 - 3.65(m, 4H), 1.58(s, 6H).

### Step 5: Synthesis of compound BB-4-6

Compound BB-4-5 (1.5 g, 2.62 mmol, hydrochloride) was dissolved in acetonitrile (50 mL) at room temperature under nitrogen atmosphere, and then added with ethyl bromoacetate (875.85 mg, 5.24 mmol, 580.04 µL) and potassium carbonate (724.86 mg, 5.24 mmol) successively. The reaction mixture was heated to 80°C, stirred, and reacted for 5 hours. After the reaction was completed, the reaction mixture was cooled to room temperature, diluted with water (100 mL), and extracted with ethyl acetate (100 mL × 3). The organic phases were combined, washed with saturated brine (100 mL × 2), dried over anhydrous sodium sulfate, filtered, and the filtrate was concentrated under reduced pressure to remove the solvent. The resulting residue was separated by column chromatography (eluent: petroleum ether/ethyl acetate = 3/1 to 1/1, v/v) to obtain compound BB-4-6. MS-ESI m/z: 622.2 [M+H]⁺.

### Step 6: Synthesis of compound BB-4

Compound BB-4-6 (1.5 g, 2.42 mmol) was dissolved in ethanol (50 mL) at room temperature under nitrogen atmosphere. The reaction mixture was slowly added dropwise with lithium hydroxide (1 M, 7.25 mL), and stirred at 25°C for 12 hours. After the reaction was completed, the reaction mixture was added with 2 M hydrochloric acid to adjust the pH to 2 to 3, and concentrated under reduced pressure to obtain compound BB-4. MS-ESI m/z: 594.1 [M+H]⁺.

### Reference example 5

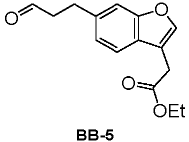

### Synthetic route:

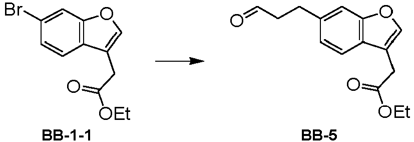

To dioxane (30 mL) were added compound BB-1-1 (3 g, 10.60 mmol) and allyl alcohol (1.34 g, 23.07 mmol, 1.57 mL), and the reaction mixture was slowly added with *N*-cyclohexyl-N-methyl-cyclohexylamine (2.48 g, 12.72 mmol, 2.70 mL), tri-tert-butylphosphine (4.29 g, 2.12 mmol, 4.97 mL, content: 10%), and tris(dibenzylideneacetone)dipalladium (970.32 mg, 1.06 mmol). The mixture was replaced with nitrogen three times, and then stirred at 30°C for 12 hours. The reaction mixture was slowly added with water (50 mL) and extracted three times with ethyl acetate (50 mL × 3). The combined organic phases were dried over anhydrous sodium sulfate, filtered, and evaporated to dryness by rotary evaporation under reduced pressure. The resulting residue was separated by column chromatography (eluent: petroleum ether/ethyl acetate = 0/1 to 3/1, v/v) to obtain compound BB-5. ¹H NMR(400MHz, CDCl₃)δ: 9.84(s, 1H), 7.60(s, 1H), 7.50(d, J=8.0 Hz, 1H), 7.33(s, 1H), 7.12(dd, J=1.0, 8.0 Hz, 1H), 4.24 - 4.16(m, 2H), 3.69(d, J=0.8 Hz, 2H), 3.14 - 3.03(m, 2H), 2.90 - 2.78(m, 2H), 1.29(t, J=7.2 Hz, 3H).

### Reference example 6

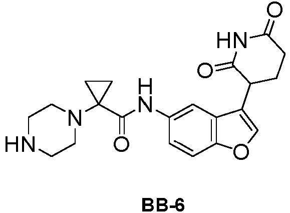

### Synthetic route:

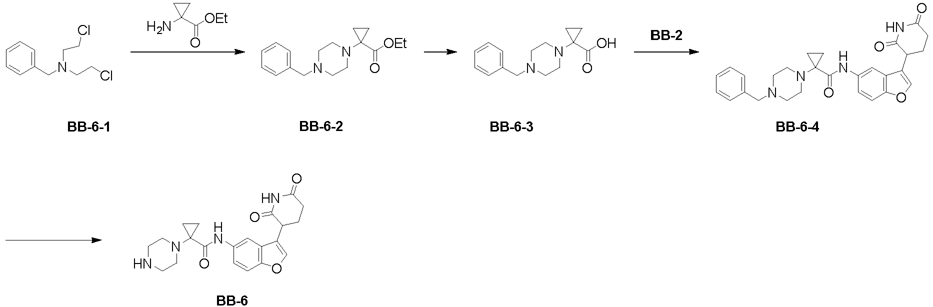

### Step 1: Synthesis of intermediate BB-6-2

To ethanol (10 mL) was added compound ethyl 1-aminocyclopropane-1-carboxylate hydrochloride (1.0 g, 6.04 mmol) at 25°C, and the stirring was started. Compound N-benzyl-2-chloro-N-(2-chloroethyl)ethylamine hydrochloride (1.78 g, 6.64 mmol) and *N,N-*diisopropylethylamine (7.88 g, 60.98 mmol, 10.62 mL) were successively added thereto. The reaction mixture was heated to 80°C under nitrogen atmosphere and stirred for another 12 hours under nitrogen atmosphere. After the reaction was completed, the reaction mixture was cooled to room temperature and then directly evaporated to dryness by rotary evaporation. The crude product was added with water (20 mL) and extracted with ethyl acetate (20 mL × 2). The organic phases were combined, washed with half-saturated brine (10 mL × 2), dried over anhydrous sodium sulfate, and filtered. The filtrate was concentrated under reduced pressure to remove the solvent, and the resulting residue was purified by column chromatography (eluent: petroleum ether/ethyl acetate = 3/1 to 1/1, v/v) to obtain intermediate BB-6-2. ¹H NMR (400 MHz, CDCl₃) δ: 7.40-7.27 (m, 5 H), 4.18 (q, J=7.20 Hz, 2H), 3.55 (s, 2H), 3.02 (s, 4H), 2.40 ( s, 4H), 1.28-1.34 (m, 5H), 0.94 (q, J=3.6 Hz, 2H).

### Step 2: Synthesis of intermediate BB-6-3

To ethanol (20 mL) was added intermediate BB-6-2 (0.3 g, 1.04 mmol) at 25°C, and the stirring was started. Compound potassium hydroxide (583.66 mg, 10.40 mmol) was successively added thereto. The reaction mixture was heated to 120°C, and stirred for another 12 hours. After the reaction was completed, the reaction mixture was cooled to room temperature, and evaporated to dryness by rotary evaporation. The reaction mixture was then added with water (40 mL), and extracted with ethyl acetate (40 mL × 2). The aqueous phase was added with 4 M dilute hydrochloric acid to adjust the pH to 3, and then extracted with ethyl acetate (40 mL). The product was in the aqueous phase, which was collected by lyophilizing. Compound BB-6-3 was obtained. ¹H NMR (400 MHz, DMSO-d6) δ: 10.70 ( s, 1H), 7.59 ( s, 2H), 7.45 (s, 3H), 4.26 ( s, 2H), 3.46 (t, J=12.4 Hz, 2H), 3.21 ( d, J=11.6 Hz, 2H), 2.94 ( d, J=12.8 Hz, 2H), 2.83 ( s, 2H), 1.16 ( s, 2H), 0.94 ( s, 2H).

### Step 3: Synthesis of compound BB-6-4

To N,N-dimethylformamide (20 mL) was added intermediate BB-6-3 at 25°C, and the stirring was started. Compound BB-2 (938.21 mg, 3.84 mmol), 2-(7-azabenzotriazol-1-yl)-*N,N,N',N*'-tetramethyluronium hexafluorophosphate (2.19 g, 5.76 mmol), and triethylamine (1.55 g, 15.37 mmol, 2.14 mL) were successively added thereto. The reaction mixture was stirred for another 12 hours under nitrogen atmosphere. After the reaction was completed, the reaction mixture was added with water (50 mL), and extracted with ethyl acetate (50 mL × 2). The organic phases were combined, washed with half-saturated brine (50 mL), dried over anhydrous sodium sulfate, and filtered. The filtrate was concentrated under reduced pressure to remove the solvent, and the resulting residue was purified by column chromatography (eluent: petroleum ether/ethyl acetate = 1/1 to 0/1, v/v) to obtain intermediate BB-6-4. MS-ESI m/z:: 487.2[M+H]⁺.¹H NMR (400 MHz, DMSO-d6) δ: 10.91 (s, 1H), 9.92 (s, 1H), 7.95 (s, 1H), 7.88 (s, 1H), 7.53 (d, J=8.8Hz, 1H), 7.41 (dd, J=8.8, 1.6Hz, 1H), 7.35-7.28 (m, 4H), 7.27-7.22 (m, 1H), 4.12 (dd, J=12.0, 4.8Hz, 1H), 3.50 (s, 2H), 3.40-3.30 (m, 2H), 2.89 (s, 2H), 2.83-2.74(m, 1H), 2.73 (s, 2H), 2.61-2.60 (m, 1H), 2.56-2.55 (m, 2H), 2.34-2.20 (m, 1H), 2.14-2.10 (m, 1H),1.08 - 1.06( m, 4 H).

### Step 4: Synthesis of compound BB-6

Compound BB-6-4 (50 mg, 97.91 µmol) was dissolved in tetrahydrofuran (10 mL) at 25°C, and added with wet palladium on carbon (5 mg, 10.28 µL) under argon atmosphere. The reaction mixture was replaced with hydrogen three times, and the pressure of the system was kept at 40 Psi. The reaction mixture was heated to 30°C and stirred for 12 hours under hydrogen atmosphere. After the reaction was completed, the reaction mixture was cooled to room temperature, filtered, and the filtrate was concentrated under reduced pressure to remove the solvent to obtain compound BB-6. MS-ESI m/z: 397.1[M+H]⁺.

### Reference example 7

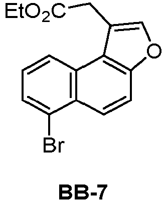

### Synthetic route:

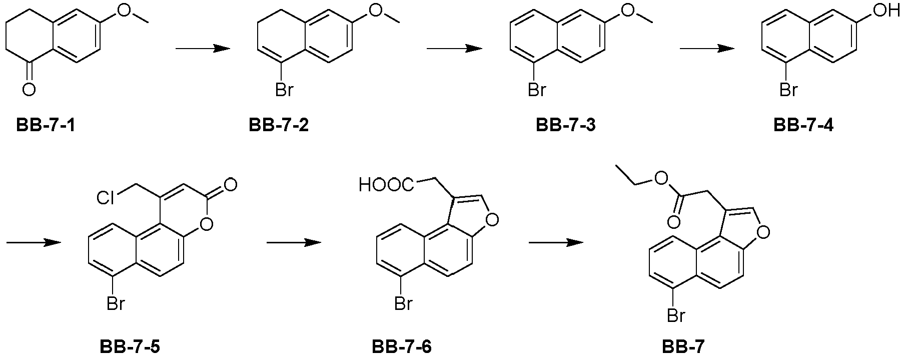

### Step 1: Synthesis of compound BB-7-2

Compound triphenyl phosphite (48.42 g, 156.06 mmol, 41.04 mL) was dissolved in dichloromethane (250 mL) at 25°C, cooled to -70°C under nitrogen atmosphere, and added dropwise with liquid bromine (27.21 g, 170.25 mmol, 8.78 mL). After the addition was completed, the reaction mixture was successively added dropwise with a solution of triethylamine (18.66 g, 184.44 mmol, 25.67 mL) and compound BB-7-1 (25.00 g, 141.88 mmol) in dichloromethane (60 mL). After the addition was completed, the reaction mixture was warmed to 25°C and stirred for 12 hours. After the reaction was completed, the reaction mixture was slowly poured into saturated sodium sulfite aqueous solution (400 mL), stirred for 10 minutes, and extracted with dichloromethane (200 mL × 3). The organic phase was washed with saturated brine (100 mL), dried over anhydrous sodium sulfate, filtered, and evaporated to dryness by rotary evaporation. The crude product was purified by column chromatography (eluent: petroleum ether). Compound BB-7-2 was obtained. ¹H NMR (400 MHz, CDCl₃) δ: 7.48 (d, J=8.78 Hz, 1H), 6.75 (dd, J=8.53, 2.51 Hz, 1H), 6.68 (d, J=2.76 Hz, 1H), 6.30 (t, J=4.77 Hz, 1H), 3.82 (s, 3H), 2.82 (t, J= 8.03Hz, 2H), 2.35 (td, J=8.03, 5.02 Hz, 2H).

### Step 2: Synthesis of compound BB-7-3

Compound BB-7-2 (10.00 g, 41.82 mmol) was dissolved in toluene (100 mL) at 25°C, cooled to 0°C under nitrogen atmosphere, and added with dichlorodicyanobenzoquinone (10.44 g, 46.00 mmol) in batches. After the addition was completed, the reaction mixture was reacted at 25°C for 15 hours. The reaction mixture was added dropwise with saturated sodium sulfite aqueous solution (200 mL) to quench, stirred for 10 minutes, added with 1 N sodium hydroxide aqueous solution (100 mL), and extracted with ethyl acetate (50 mL × 3). The organic phase was washed with saturated brine (100 mL), dried over anhydrous sodium sulfate, filtered, and evaporated to dryness by rotary evaporation. The resulting crude product was slurried with petroleum ether (50 mL) for 10 minutes, filtered, and the filter cake was rinsed with petroleum ether (25 mL × 2). The filtrate was evaporated to dryness by rotary evaporation, and the crude product was purified by column chromatography (eluent: petroleum ether). Compound BB-7-3 was obtained. ¹H NMR (400 MHz, CDCl₃) δ: 8.13 (d, J=9.29 Hz, 1H), 7.67 (d, J=8.28 Hz, 1H), 7.61 (d, J=7.28 Hz, 1H), 7.21-7.26 (m, 2H), 7.11 (d, J=2.51 Hz, 1H), 3.92 (s, 3H).

### Step 3: Synthesis of compound BB-7-4

Compound BB-7-3 (4.90 g, 20.67 mmol) was dissolved in dichloromethane (50 mL) at 25°C, cooled to 0°C, and slowly added dropwise with boron tribromide (6.21 g, 24.80 mmol, 2.39 mL). After the addition was completed, the reaction mixture was stirred at 25°C for 3 hours. The reaction mixture was poured into ice water (250 mL) to quench and extracted with dichloromethane (100 mL). The organic phase was washed with saturated brine (100 mL × 1), dried over anhydrous sodium sulfate, filtered, and evaporated to dryness by rotary evaporation. Compound BB-7-4 was obtained. ¹H NMR (400 MHz, CDCl₃) δ: 8.19 (d, J=9.03 Hz, 1H), 7.63-7.73 (m, 2H), 7.26-7.32 (m, 1H), 7.23 (dd, J=9.03, 2.51 Hz, 1H), 7.19 (d, J=2.51 Hz, 1H).

### Step 4: Synthesis of compound BB-7-5

Compound BB-7-4 (2.50 g, 11.21 mmol) was dissolved in methanesulfonic acid (25 mL) at 20°C, added dropwise with ethyl 4-chloroacetoacetate (2.77 g, 16.81 mmol, 2.27 mL), and stirred at 20°C for 15 hours. The reaction mixture was poured into ice water (200 mL) to quench, stirred for 10 minutes, and filtered. The filter cake was rinsed with water (30 mL × 3), and then evaporated to dryness by rotary evaporation. Compound BB-7-5 was obtained. ¹H NMR (400 MHz, DMSO-d6) δ: 8.56 (d, J=8.78 Hz, 1H), 8.46 (d, J=9.29 Hz, 1 H), 7.71 (d, J=9.29 Hz, 1H), 7.63 (dd, J=8.66, 7.65 Hz, 1H), 6.92 (s, 1H), 5.39 (s, 2H).

### Step 5: Synthesis of compound BB-7-6

To an aqueous solution of sodium hydroxide (2 M, 1.03 mL) was added compound BB-7-5 (0.10 g, 309.05 µmol) at 25°C, and the reaction mixture was stirred at 80°C for 3 hours. The reaction mixture was cooled to 25°C, diluted with water (10 mL), added with dilute hydrochloric acid (2 M, aqueous solution) to adjust the pH to 3, and extracted with ethyl acetate (10 mL × 3). The organic phase was washed with saturated brine (10 mL × 1), dried over anhydrous sodium sulfate, filtered, and the filtrate was evaporated to dryness by rotary evaporation. Compound BB-7-6 was obtained. ¹H NMR (400 MHz, DMSO-d6) δ: 12.59 (br s, 1H), 8.24 (d, J=8.38 Hz, 1H), 7.96 (d, J=9.26 Hz, 1H), 7.90 (d, J=7.50 Hz, 1H), 7.54 (dd, J=2.8 Hz, 1H), 7.53 (s, 1H), 7.41 (s, 1H), 4.08 (s, 2H).

### Step 6: Synthesis of compound BB-7

Compound BB-7-6 (1.40 g, 4.59 mmol) was dissolved in anhydrous ethanol (14 mL) at 25°C, slowly added dropwise with sulfuric acid (413.28 mg, 4.13 mmol, 224.61 µL, concentration: 98%), then heated to 80°C, and stirred for 12 hours. The reaction mixture was concentrated under reduced pressure to remove the solvent, diluted with ethyl acetate (60 mL), added with saturated sodium bicarbonate aqueous solution (60 mL), and extracted with ethyl acetate (60 mL × 2). The organic phase was washed with saturated brine (60 mL × 1), dried over anhydrous sodium sulfate, filtered, and evaporated to dryness by rotary evaporation. The residue was slurried with petroleum ether (5 mL) at room temperature, stirred for 10 minutes, and then filtered. The filter cake was rinsed with petroleum ether (5 mL × 2), collected, and evaporated to dryness by rotary evaporation. Compound BB-7 was obtained. ¹H NMR (400 MHz, CDCl₃) δ: 8.22 (t, J=8.41Hz, 2H), 7.78-7.85 (m, 2H), 7.75 (d, J=9.29Hz, 1H), 7.41 (t, J=7.91Hz, 1H), 4.23 (q, J=7.03 Hz, 2H), 4.06 (s, 2H), 1.26 (t, J=7.15Hz, 3H).

### Reference example 8

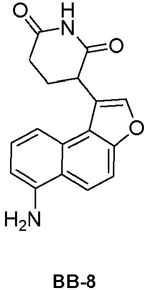

### Synthetic route:

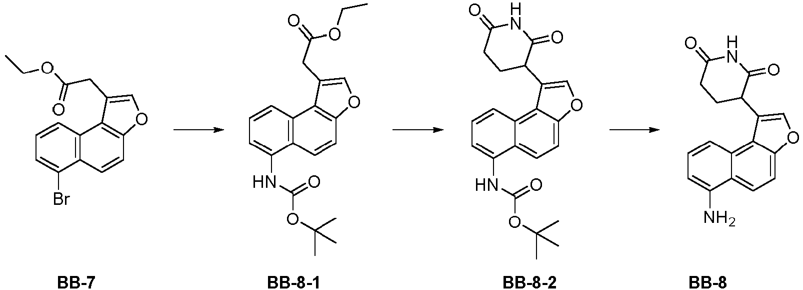

### Step 1: Synthesis of compound 8-1

Compound BB-7 (5 g, 15.01 mmol) was dissolved in a mixed solvent of toluene (50 mL) and water (10 mL), and then added with tert-butyl carbamate (2.64 g, 22.51 mmol), potassium phosphate (12.74 g, 60.03 mmol), tris(dibenzylideneacetone)dipalladium (961.96 mg, 1.05 mmol), and 2-di-tert-butylphosphino-2',4',6'-triisopropylbiphenyl (892.16 mg, 2.10 mmol). The reaction mixture was replaced with nitrogen three times, heated to 100°C, and stirred for 15 hours. The reaction mixture was cooled to room temperature, filtered, and the filter cake was rinsed with ethyl acetate (30 mL × 3). The filtrate was washed with saturated brine (100 mL), dried over anhydrous sodium sulfate, filtered, and evaporated to dryness by rotary evaporation. The crude product was added with methyl tert-butyl ether (50 mL), stirred for 10 minutes, and filtered. The filter cake was rinsed with methyl tert-butyl ether (10 mL × 2) and collected to obtain compound BB-8-1.

### Step 2: Synthesis of compound BB-8-2

Compound BB-8-1 (4.2 g, 11.37 mmol) and acrylamide (888.93 mg, 12.51 mmol, 863.04 µL) were dissolved in *N,N-*dimethylformamide (40 mL), cooled to 0°C under nitrogen atmosphere, added dropwise with a solution of potassium tert-butoxide (2.55 g, 22.74 mmol) in N,N-dimethylformamide (10 mL), warmed to 20°C, and stirred for 2 hours. The reaction mixture was poured into 0.2 N dilute hydrochloric acid (200 mL) and extracted with ethyl acetate (100 mL × 3). The organic phase was washed with saturated brine (100 mL), dried over anhydrous sodium sulfate, filtered, and evaporated to dryness by rotary evaporation. The crude product was added with dichloromethane (20 mL), stirred for 10 minutes, and filtered. The filter cake was rinsed with dichloromethane (10 mL) and collected to obtain compound BB-8-2. ¹H NMR (400 MHz, DMSO-d6) δ:10.94 (s, 1H), 9.28 (s, 1H), 8.04-7.96 (m, 3H), 7.79 (d, J=9.6 Hz, 1H), 7.55-7.51 (m, 2H), 4.67 (dd, J=4.4 Hz, 12.0 Hz, 1H), 2.93-2.84 (m, 1H), 2.66-2.60 (m, 1H), 2.45-2.36 (m, 1H), 2.33-2.26 (m, 1H), 1.49 (s, 9H).

### Step 3: Synthesis of hydrochloride of compound BB-8

Compound BB-8-2 (1 g, 2.54 mmol) was dissolved in ethyl acetate (10 mL), added dropwise with hydrochloric acid/ethyl acetate (4 M, 50.00 mL), and stirred at 20°C for 15 hours. The reaction mixture was concentrated under reduced pressure to obtain hydrochloride of compound BB-8.

### Reference example 9

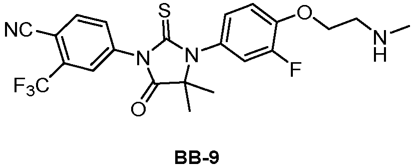

### Synthetic route:

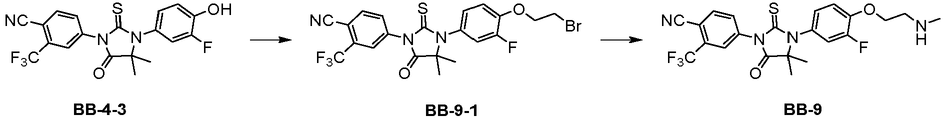

### Step 1: Synthesis of compound BB-9-1

Compound BB-4-3 (13 g, 30.71 mmol) and potassium carbonate (8.49 g, 61.41 mmol) were dissolved in *N,N*-dimethylformamide (300 mL), added with 1,2-dibromoethane (28.84 g, 153.53 mmol, 11.58 mL), heated to 80°C, and stirred for 12 hours. The reaction mixture was cooled to room temperature, added with water (1 L), and extracted with ethyl acetate (300 mL × 3). The organic phase was washed with saturated brine (300 mL × 2), dried over anhydrous sodium sulfate, filtered, and evaporated to dryness by rotary evaporation. The crude product was separated by column chromatography (eluent: petroleum ether/ethyl acetate = 4/1) to obtain compound BB-9-1. ¹H NMR (400 MHz, CDCl₃) δ: 8.00-7.96 (m, 2H), 7.85-7.83 (m, 1H), 7.14-7.04 (m, 3H), 4.44 (t, J=6.0 Hz, 2H), 3.71 (t, J=6.4 Hz, 2H), 1.60 (s, 6H).

### Step 2: Synthesis of compound BB-9

Compound BB-9-1 (3 g, 5.66 mmol) and methylamine hydrochloride (1.91 g, 28.28 mmol) were placed in *N,N*-dimethylformamide (30 mL), added with potassium carbonate (11.73 g, 84.85 mmol), heated to 50°C, and stirred for 12 hours. The reaction mixture was cooled to room temperature, added with water (70 mL), and extracted with ethyl acetate (50 mL × 4). The organic phase was washed with saturated brine (70 mL × 2), dried over anhydrous sodium sulfate, filtered, and evaporated to dryness by rotary evaporation. The crude product was separated by column chromatography (eluent: dichloromethane/methanol = 50/1 to 10/1) to obtain compound BB-9.

### Reference example 10: Fragment BB-10

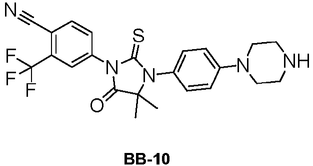

### Synthetic route:

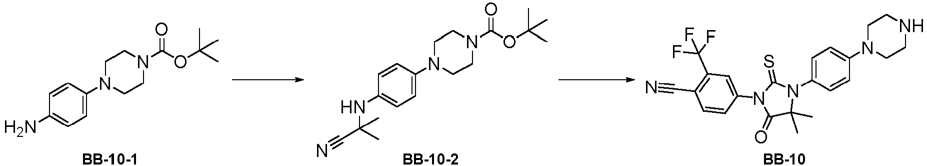

### Step 1: Synthesis of compound BB-10-2

Compound BB-10-1 (5 g, 18.03 mmol) was dissolved in dichloromethane (50 mL) and acetone (25 mL) at 0°C under nitrogen atmosphere, then slowly added dropwise with cyanotrimethylsilane (2.68 g, 27.04 mmol, 3.38 mL) and trimethylsilyl trifluoromethanesulfonate (400.67 mg, 1.80 mmol, 325.74 µL) successively, and the reaction mixture was stirred at 20°C for 2 hours. After the reaction was completed, the reaction mixture was concentrated under reduced pressure to remove the solvent. The resulting residue was separated by column chromatography (eluent: petroleum ether/ethyl acetate = 1/0 to 4/1, v/v) to obtain compound BB-10-2.

### Step 2: Synthesis of compound BB-10

Compound BB-10-2 (10 g, 29.03 mmol) was dissolved in N,N-dimethylacetamide (100 mL) at room temperature under nitrogen atmosphere. To the reaction mixture was added 4-isothiocyanato-2-(trifluoromethyl)benzonitrile (6.62 g, 29.03 mmol) in batches. The reaction mixture was stirred at 20°C for 3 hours, then added with methanol (100 mL) and dilute hydrochloric acid (2 M, 56.32 mL), and stirred at 70°C for 2 hours. After the reaction was completed, the reaction mixture was cooled to room temperature, diluted with water (100 mL), and extracted with ethyl acetate (100 mL × 3). The organic phases were combined, washed with saturated brine (100 mL × 3), dried over anhydrous sodium sulfate, filtered, and the filtrate was concentrated under reduced pressure to remove the solvent. The resulting residue was separated by column chromatography (eluent: dichloromethane/methanol = 20/1 to 10/1, v/v) to obtain compound BB-10. ¹H NMR (400 MHz, CDCl₃) δ:7.99 (d, J=8.8 Hz, 1H), 7.96 (d, J=1.6 Hz, 1H), 7.84 (dd, J=2.0, 8.4 Hz, 1H), 7.22 (d, J=9.2 Hz, 2H), 7.04 (d, J=8.8 Hz, 2H), 3.64-3.57 (m, 4H), 3.45-3.38 (m, 4 H), 1.58 (s, 6H).

### Reference example 11: Fragment BB-11

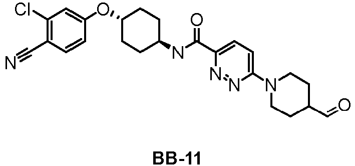

### Synthetic route:

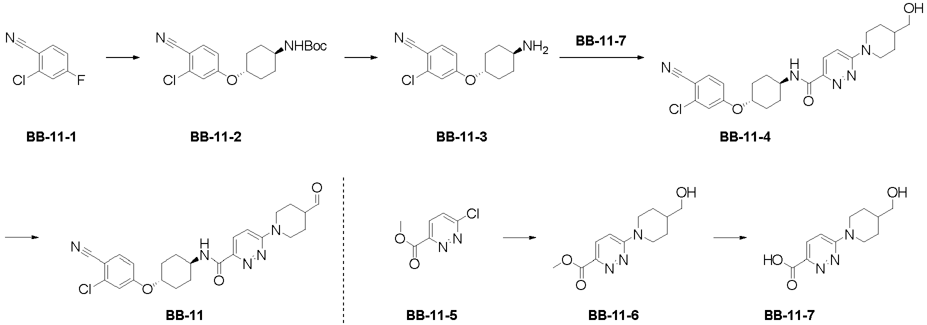

### Step 1: Synthesis of compound BB-11-2

Compound BB-11-1 (0.87 g, 5.57 mmol) and compound tert-butyl [(1R,4R)-4-hydroxycyclohexyl]carbamate (1.00 g, 4.64 mmol) were dissolved in*N,N*-dimethylformamide (20 mL) at 0°C under nitrogen atmosphere, then added with sodium hydride (278.68 mg, 6.97 mmol, 60%), stirred, and reacted at 0°C for 2 hours. After the reaction was completed, the reaction mixture was added with water (50 mL), and extracted with ethyl acetate (50 mL × 3). The organic phases were combined, washed with water (50 mL × 3) and saturated brine (50 mL) successively, dried over anhydrous sodium sulfate, filtered, and the filtrate was concentrated under reduced pressure to remove the solvent. The resulting residue was separated by column chromatography (eluent: petroleum ether/ethyl acetate = 1/0 to 6/1, v/v) to obtain compound BB-11-2. ¹H NMR (400 MHz, CDCl₃) δ: 8.00 (s, 1H), 7.56-7.47 (m, 1H), 6.97-6.92 (m, 1H), 6.84-6.72 (m, 1H), 4.29-4.17 (m, 1H), 4.13-4.08 (m, 1H), 2.14-2.03 (m, 4H), 1.47-1.38 (m, 9H), 1.26-1.20 (m, 4H).

### Step 2: Synthesis of hydrochloride of compound BB-11-3

Compound BB-11-2 (2.70 g, 7.70 mmol) was dissolved in methanol (5 mL) at room temperature, then added with 4 M hydrogen chloride methanol solution (25 mL), and the reaction mixture was stirred and reacted at room temperature for 12 hours. After the reaction was completed, the reaction mixture was concentrated under reduced pressure to remove the solvent to obtain hydrochloride of compound BB-11-3, which was directly used in the next reaction step.

### Step 3: Synthesis of compound BB-11-6

Compound BB-11-5 (10.00 g, 57.95 mmol) and 4-piperidinemethanol (6.67 g, 57.95 mmol) were dissolved in dimethyl sulfoxide (100 mL) at room temperature, and then added with triethylamine (11.73 g, 115.90 mmol, 16.13 mL). The reaction mixture was heated to 90°C, stirred, and reacted for 12 hours. After the reaction was completed, the reaction mixture was cooled to room temperature, added with water (200 mL), and extracted with dichloromethane (100 mL × 4). The organic phases were combined, washed with water (100 mL × 2) and saturated brine (100 mL) successively, dried over anhydrous sodium sulfate, filtered, and the filtrate was concentrated under reduced pressure to remove the solvent. The resulting residue was separated by column chromatography (eluent: dichloromethane/methanol = 1/0 to 20/1, v/v) to obtain compound BB-11-6. ¹H NMR (400 MHz, DMSO-d6) δ:7.79 (d, J=9.2 Hz, 1H), 7.27 (d, J=9.6 Hz, 1H), 4.57-4.53 (m, 1H), 4.53-4.48 (m, 2H), 3.86 (s, 3H), 3.30-3.24 (m, 2H), 2.99 (td, J=1.9, 12.7 Hz, 2H), 1.81-1.66 (m, 3H), 1.21-1.06 (m, 2H).

### Step 4: Synthesis of compound BB-11-7

BB-11-6 (2.00 g, 7.96 mmol) was dissolved in tetrahydrofuran (15 mL) at room temperature, then added with 2 M sodium hydroxide aqueous solution (15 mL), and the reaction mixture was stirred and reacted at room temperature for 12 hours. After the reaction was completed, the reaction mixture was added with 4 M hydrochloric acid to adjust the pH to 4 to 5, concentrated under reduced pressure to remove the solvent to obtain a crude product of compound BB-11-7, which was directly used in the next reaction step.

### Step 5: Synthesis of compound BB-11-4

Hydrochloride of compound BB-11-3 (7.65 mmol) and compound BB-11-7 (7.65 mmol, crude product) were dissolved in *N,N*-dimethylformamide (46 mL) at room temperature under nitrogen atmosphere, then added with diisopropylethylamine (1.03 g, 7.96 mmol, 1.39 mL) and 2-(7-azabenzotriazol-1-yl)-*N,N,N',N'*-tetramethyluronium hexafluorophosphate (3.03 g, 7.96 mmol), and the reaction mixture was stirred and reacted at room temperature for 12 hours. After the reaction was completed, the reaction mixture was added with water (100 mL) and extracted with ethyl acetate (70 mL × 3). The organic phases were combined, washed with water (100 mL × 2) and saturated brine (100 mL) successively, dried over anhydrous sodium sulfate, filtered, and the filtrate was concentrated under reduced pressure to remove the solvent. The resulting residue was separated by column chromatography (eluent: dichloromethane/methanol = 1/0 to 20/1, v/v) to obtain compound BB-11-4. MS-ESI m/z: 470.2 [M+H]⁺. ¹H NMR (400 MHz, DMSO-d6) δ: 8.58 (d, J=8.4 Hz, 1H), 7.85 (d, J=8.4 Hz, 1H), 7.79 (d, J=9.6 Hz, 1H), 7.38 (d, J=2.4 Hz, 1H), 7.33 (d, J=9.6 Hz, 1H), 7.13 (dd, J=2.4, 8.8 Hz, 1H), 4.60-4.44 (m, 4H), 3.92-3.79 (m, 1H), 3.32-3.27 (m, 2H), 3.07-2.95 (m, 2H), 2.18-2.05 (m, 2H), 1.95-1.84 (m, 2H), 1.81-1.68 (m, 3H), 1.68-1.51 (m, 2H), 1.51-1.48 (m, 2H), 1.20-1.06 (m, 2H).

### Step 6: Synthesis of compound BB-11

Compound BB-11-4 (100.02 mg, 205.06 µmol) was dissolved in dichloromethane (6 mL) at room temperature, then added with Dess-Martin periodinane (130.46 mg, 307.59 µmol), and the reaction mixture was stirred and reacted at room temperature for 1 hour. After the reaction was completed, the reaction mixture was added with water (20 mL) and extracted with ethyl acetate (20 mL × 3). The organic phases were combined, washed with saturated brine (20 mL), dried over anhydrous sodium sulfate, filtered, and the filtrate was concentrated under reduced pressure to remove the solvent. The resulting residue was separated by column chromatography (eluent: petroleum ether/ethyl acetate = 1/0 to 0/1, v/v) to obtain compound BB-11. MS-ESI m/z: 468.2 [M+H]⁺.

### Reference example 12: Fragment BB-12

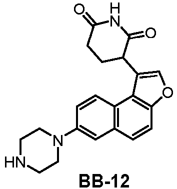

### Synthetic route:

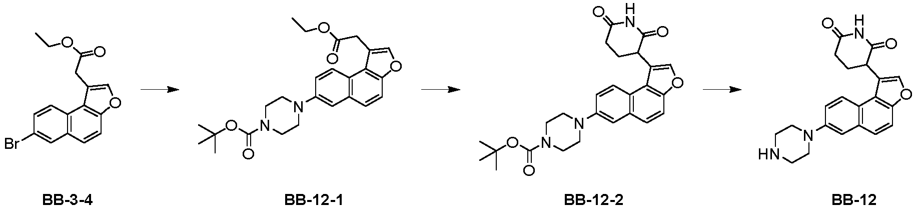

### Step 1: Synthesis of compound BB-12-1

To toluene (150 mL) and water (20 mL) were added compound BB-3-4 (10.00 g, 30.01 mmol) and 1-(tert-butoxycarbonyl)piperazine (8.39 g, 45.02 mmol) at 25°C. To the reaction were slowly added tris(dibenzylideneacetone)dipalladium (1.92 g, 2.10 mmol), potassium phosphate (19.11 g, 90.04 mmol), and 2-di-tert-butylphosphino-2',4',6'-triisopropylbiphenyl (1.78 g, 4.20 mmol). The mixture was replaced with nitrogen three times, then heated to 100°C, and stirred for 12 hours. The reaction mixture was cooled to 25°C, filtered through diatomite, and the filter cake was washed with ethyl acetate (20 mL). The filtrate was added with ethyl acetate (300 mL) and water (300 mL), and the aqueous phase was extracted with ethyl acetate (200 mL × 2). The organic phases were combined, dried over anhydrous sodium sulfate, filtered, and evaporated to dryness by rotary evaporation under reduced pressure. The crude product was separated by column chromatography (eluent: petroleum ether/ethyl acetate = 50/1 to 5/1) to obtain compound BB-12-1.

MS-ESI m/z: 439.0 [M+H]⁺. ¹H NMR (400MHz, CDCl₃) δ: 8.15 (d, J=9.0 Hz, 1H), 7.74 (s, 1H), 7.60 (s, 2H), 7.36 (dd, J=2.5, 9.0 Hz, 1H), 7.31 (d, J=2.5 Hz, 1H), 4.23 (q, J=7.1 Hz, 2H), 4.04 (s, 2H), 3.71 - 3.62 (m, 4H), 3.29 - 3.20 (m, 4H), 1.53 (s, 9H), 1.28 (dt, J=7.2 Hz, 3H).

### Step 2: Synthesis of compound BB-12-2

To *N,N*-dimethylformamide (80 mL) were added compound BB-12-1 (8.6 g, 15.86 mmol) and acrylamide (1.13 g, 15.86 mmol, 1.09 mL) at 0°C, and the stirring was started. To the reaction was added potassium tert-butoxide (2.67 g, 23.80 mmol) in batches. The reaction mixture was replaced with nitrogen three times, and then stirred at 0°C for 1 hour. After the reaction was completed, the reaction mixture was slowly added dropwise with saturated ammonium chloride aqueous solution (480 mL). A large amount of solid was precipitated, and filtered. The filter cake was washed twice with water (20 mL × 2), collected, and evaporated to dryness by rotary evaporation under reduced pressure. The resulting crude product was added with methanol (10 mL), stirred for 30 minutes, and filtered. The filter cake was washed with methanol (5 mL), collected, and evaporated to dryness by rotary evaporation under reduced pressure to obtain compound BB-12-2. ¹H NMR (400MHz, DMSO-d6) δ: 10.96 (br dd, J=2.9, 6.1 Hz, 1H), 8.02 (br d, J=8.8 Hz, 1H), 7.97 - 7.91 (m, 1H), 7.72 - 7.65 (m, 2H), 7.44 - 7.37 (m, 2H), 4.62 (br dd, J=4.3, 11.8 Hz, 1H), 3.53 (br s, 4H), 3.22-3.20 (m, 4H), 2.93 - 2.83 (m, 1H), 2.68 - 2.57 (m, 1H), 2.39 (dq, J=4.3, 12.5 Hz, 1H), 2.26 (br dd, J=3.9, 8.7 Hz, 1H), 1.44 (s, 9H).

### Step 3: Synthesis of hydrochloride of compound BB-12

To ethyl acetate (20 mL) was added compound BB-12-2 (3.4 g, 7.34 mmol), and the stirring was started. A solution of hydrochloric acid/ethyl acetate (4 M, 30 mL) was slowly added thereto. The reaction mixture was replaced with nitrogen three times and stirred at 25°C for 12 hours. After the reaction was completed, the reaction mixture was filtered. The filter cake was washed with ethyl acetate (5 mL × 2), collected, and evaporated to dryness by rotary evaporation under reduced pressure to obtain hydrochloride of compound BB-12. ¹H NMR (400MHz, DMSO-d6) δ: 10.96 (s, 1H), 9.65 (br s, 2H), 8.07 (br d, J=9.3 Hz, 1H), 7.96 (s, 1H), 7.75 - 7.69 (m, 2H), 7.53 (d, J=2.3 Hz, 2H), 7.45 (dd, J=2.4, 9.2 Hz, 1H), 4.64 (br dd, J=4.4, 11.9 Hz, 1H), 3.54 (br d, J=5.0 Hz, 4H), 3.28 (br s, 4H), 2.94 - 2.82 (m, 1H), 2.67 - 2.58 (m, 1H), 2.39 (br dd, J=4.0, 12.5 Hz, 1H), 2.30 - 2.17 (m, 1H).

### Reference example 13: Fragment BB-13

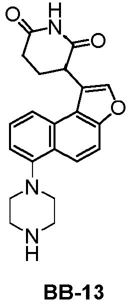

### Synthetic route:

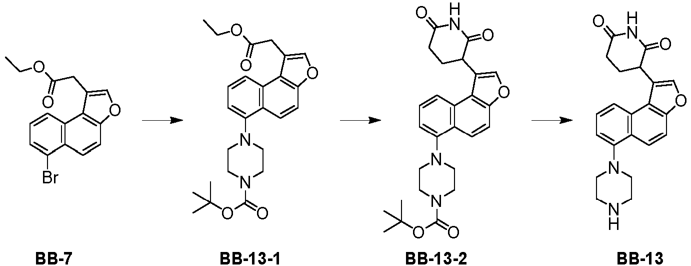

### Step 1: Synthesis of compound BB-13-1

To dioxane (20 mL) and water (4 mL) were added compound BB-7 (1.4 g, 4.20 mmol) and 1-tert-butoxycarbonylpiperazine (1.17 g, 6.30 mmol) at 25°C. To the reaction were slowly added tris(dibenzylideneacetone)dipalladium (269.35 mg, 294.14 µmol), potassium phosphate (2.68 g, 12.61 mmol), and 2-di-tert-butylphosphino-2',4',6'-triisopropylbiphenyl (249.81 mg, 588.28 µmol). The mixture was replaced with nitrogen three times, then heated to 100°C, and stirred for 12 hours. The reaction mixture was cooled to 25°C, filtered through diatomite, and the filter cake was washed with ethyl acetate (20 mL). The filtrate was added with ethyl acetate (100 mL) and water (100 mL), and the aqueous phase was extracted with ethyl acetate (100 mL × 2). The organic phases were combined, dried over anhydrous sodium sulfate, filtered, and evaporated to dryness by rotary evaporation under reduced pressure. The crude product was separated by column chromatography (eluent: petroleum ether/ethyl acetate = 50/1 to 10/1) to obtain compound BB-13-1. MS: ESI m/z:439.0[M+H]⁺. ¹H NMR (400MHz, DMSO-d6) δ: 8.17 (d, J=9.3 Hz, 1H), 8.04 (s, 1H), 7.86 (d, J=8.3 Hz, 1H), 7.77 (d, J=9.3 Hz, 1H), 7.53 (t, J=8.0 Hz, 1H), 7.19 (d, J=7.3 Hz, 1H), 4.20 - 4.09 (m, 4H), 3.36 - 2.59 (m, 8H), 1.45 (s, 9H), 1.20 (t, J=7.0 Hz, 3H).

### Step 2: Synthesis of compound BB-13-2

To *N,N*-dimethylformamide (20 mL) were added compound BB-13-1 (1.5 g, 3.26 mmol) and acrylamide (231.68 mg, 3.26 mmol, 224.93 µL) at 0°C, and the stirring was started. To the reaction was slowly added potassium tert-butoxide (438.91 mg, 3.91 mmol) in batches. The reaction mixture was replaced with nitrogen three times, and then stirred at 0°C for 1 hour. To saturated ammonium chloride aqueous solution (75 mL) was slowly added dropwise the mixture. A solid was precipitated and filtered. The filter cake was washed with water (5 mL × 2), and the collected solid was evaporated to dryness by rotary evaporation under reduced pressure. The crude product was added with methanol (5 mL), stirred for 15 minutes, and filtered. The filter cake was washed with methanol (3 mL × 2), and the collected solid was evaporated to dryness by rotary evaporation under reduced pressure. Compound BB-13-2 was obtained. MS-ESI m/z: 464.0[M+H]⁺. ¹H NMR (400MHz, DMSO-d6) δ: 8.19 (d, J=9.3 Hz, 1H), 7.97 (d, J=16.8 Hz, 2H), 7.78 (d, J=9.0 Hz, 1H), 7.51 (br t, J=8.0 Hz, 1H), 7.18 (br d, J=7.5 Hz, 1H), 4.66 (br d, J=7.8 Hz, 1H), 3.34 (m, 4H), 3.17 (m, 4H), 2.89 - 2.81 (m, 1H), 2.68 - 2.55 (m, 1H), 2.40 (dq, J=3.8, 12.4 Hz, 1H), 2.26 (br s, 1H), 1.44 (s, 9H).

### Step 3: Synthesis of hydrochloride of compound BB-13

To ethyl acetate (6 mL) was added compound BB-13-2 (0.6 g, 1.16 mmol), and the stirring was started. To the reaction was slowly added hydrochloric acid/ethyl acetate (4 M, 10 mL). The reaction mixture was replaced with nitrogen three times and stirred at 25°C for 12 hours. The mixture was evaporated to dryness by rotary evaporation under reduced pressure to obtain hydrochloride of compound BB-13. MS-ESI m/z: 364.0[M+H]⁺.

### Example 1

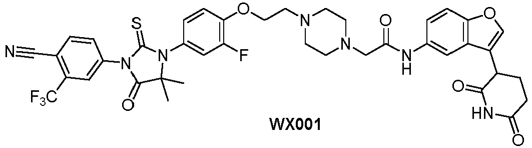

### Synthetic route:

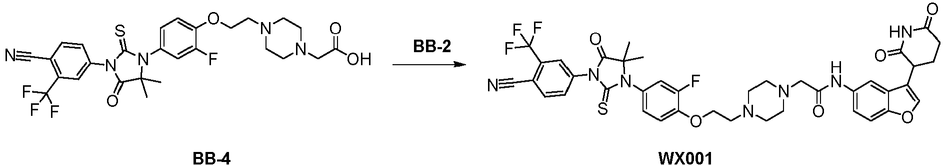

Compound BB-4 (0.3 g, 505.40 µmol) was dissolved in *N,N-*dimethylformamide (10 mL) at room temperature under nitrogen atmosphere, then added with BB-2 (123.44 mg, 505.40 µmol, hydrochloride), triethylamine (255.70 mg, 2.53 mmol, 351.72 µL), and 2-(7-azabenzotriazol-1-yl)-*N,N,N',N'*-tetramethyluronium hexafluorophosphate (288.25 mg, 758.10 µmol), and the reaction mixture was stirred at 20°C for 2 hours. After the reaction was completed, the reaction mixture was added with water (50 mL), and extracted with ethyl acetate (50 mL × 3). The organic phases were combined, washed with saturated brine (50 mL × 2), dried over anhydrous sodium sulfate, filtered, and the filtrate was concentrated under reduced pressure to remove the solvent. The resulting residue was separated by preparative HPLC (mobile phase: acetonitrile/water; acidic system: 0.05% hydrochloric acid), and lyophilized to obtain target compound WX001. MS-ESI m/z: 820.2 [M+H]⁺. ¹H NMR(400 MHz, DMSO-d6)δ: 10.94(s, 1H), 10.61(s, 1H), 8.40(d, J=8.0 Hz, 1H), 8.29(d, J=1.6Hz, 1H), 8.08(m, 1H), 7.93-7.92(m, 2H), 7.57(d, J=8.8Hz, 1H), 7.49-7.43(m, 3H), 7.40(d, J=2.4Hz, 1H), 4.59(s, 2H), 4.15-4.11(m, 1H), 3.62-3.54(m, 6H), 2.90-2.51(m, 8H), 2.25-2.15(m, 2H), 1.52(s, 6H).

### Example 2

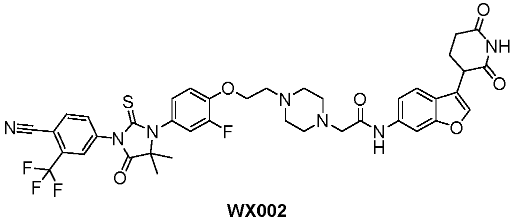

### Synthetic route:

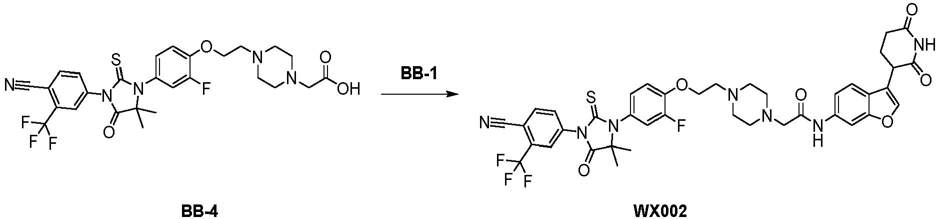

To N,N-dimethylformamide (2 mL) were added compound BB-4 (100 mg, 168.47 µmol) and hydrochloride of intermediate BB-1 (47.29 mg, 168.47 µmol). To the reaction were slowly added 2-(7-azabenzotriazol-1-yl)-*N,N,N',N'*-tetramethyluronium hexafluorophosphate (96.08 mg, 252.70 µmol), and triethylamine (34.09 mg, 336.93 µmol, 46.90 µL). The mixture was replaced with nitrogen three times, and stirred at 25°C for 12 hours. The reaction mixture was poured into water (20 mL) and ethyl acetate (30 mL), and the separated organic phase was washed twice with water (20 mL × 2). The collected organic phase was dried over anhydrous sodium sulfate, filtered, and evaporated to dryness by rotary evaporation under reduced pressure. The resulting residue was separated by preparative HPLC (mobile phase: acetonitrile/water; acidic system: 0.05% hydrochloric acid) to obtain compound WX002. MS-ESI m/z: 820.2 [M+H]⁺. ¹H NMR(400MHz, DMSO-d6)δ: 10.90(s, 1H), 10.47(br s, 1H), 8.40(d, J=8.3 Hz, 1H), 8.28(d, J=1.5 Hz, 1H), 8.13 - 7.97(m, 2H), 7.87(s, 1H), 7.54(d, J=8.5 Hz, 1H), 7.47 - 7.29(m, 3H), 7.26 - 7.13(m, 1H), 4.54(br s, 2H), 4.12(dd, J=4.8, 11.8 Hz, 1H), 3.67 - 3.45(m, 2H), 2.83 - 2.67(m, 2H), 2.63 - 2.43(m, 10H), 2.39 - 2.25(m, 1H), 2.19 - 2.03(m, 1H), 1.52(s, 6H).

### Example 3

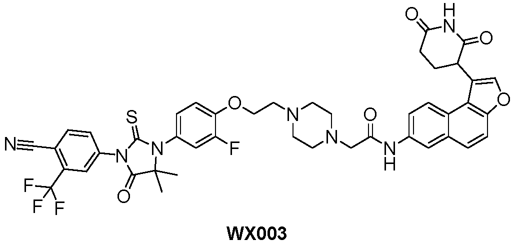

### Synthetic route:

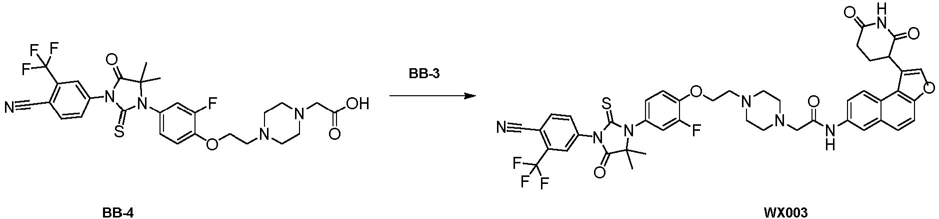

Hydrochloride of BB-3 (378 mg, 1.08 mmol) and BB-4 (767.86 mg, 1.29 mmol) were dissolved in *N,N*-dimethylformamide (5 mL) solvent at room temperature under nitrogen atmosphere, then added with 2-(7-azabenzotriazol-1-yl)-*N,N,N',N'*-tetramethyluronium hexafluorophosphate (614.82 mg, 1.62 mmol) and triethylamine (272.70 mg, 2.69 mmol) respectively, and the reaction mixture was stirred at 25°C for 3 hours. After the reaction was completed, the reaction mixture was added with water (50 mL), and extracted with ethyl acetate (100 mL × 2). The organic phases were combined, washed with saturated brine (100 mL) successively, dried over anhydrous sodium sulfate, filtered, and the filtrate was concentrated under reduced pressure to remove the solvent. The resulting residue was separated by preparative HPLC (mobile phase: acetonitrile/water; acidic system: 0.05% hydrochloric acid) to obtain target compound WX003. MS-ESI m/z: 870.1 [M+H]⁺. ¹H NMR(400 MHz, DMSO-d6)δ: 10.96(s, 1H), 10.73(s, 1H), 8.41-8.40(m, 2H), 8.39(d, J=1.6 Hz, 1H), 8.08(d, J=8.8 Hz, 1H), 8.07(dd, J=1.6, 8.4 Hz, 1H), 8.01(s, 1H), 7.78(s, 3H), 7.75(d, J=2.0 Hz, 1H), 7.46-7.37(m, 2H), 7.23(d, J=8.8 Hz, 1H), 4.68(dd, J=4.0, 11.2 Hz, 1H), 4.58(s, 2H), 3.98(m, 1H), 3.47(s, 9H), 2.95 - 2.58(m, 3H), 2.49 - 2.21(m, 3H), 1.52(s, 6H).

### Example 4

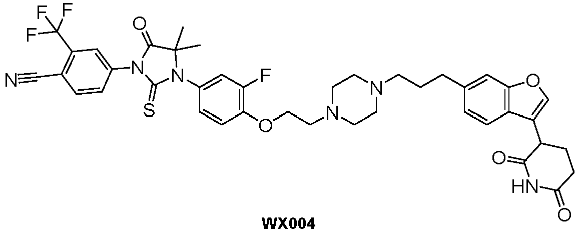

### Synthetic route:

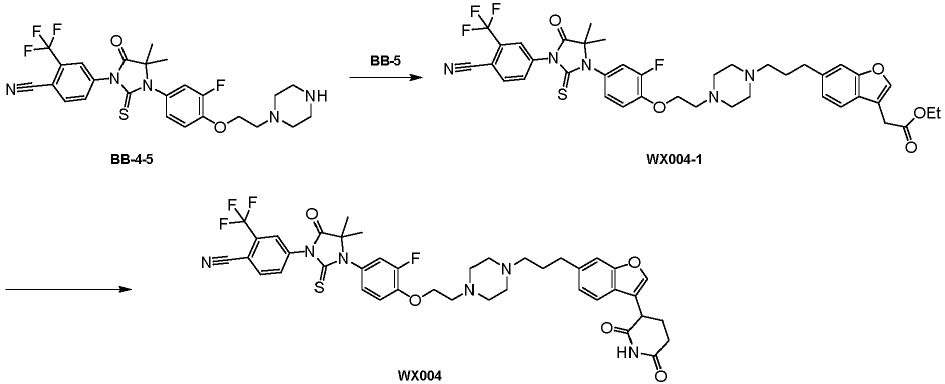

### Step 1: Synthesis of WX004-1

To dichloromethane (10 mL) were added compound BB-5 (102.38 mg, 393.35 µmol) and BB-4-5 (150 mg, 262.23 µmol, 145.82 µL, hydrochloride). The mixture was slowly added with triethylamine (53.07 mg, 524.46 µmol, 73.00 µL), stirred at 25°C for 0.5 hours, slowly added with sodium triacetoxyborohydride (138.94 mg, 655.58 µmol), replaced with nitrogen three times, and then stirred at 25°C for 11.5 hours. The reaction mixture was slowly added with water (50 mL), extracted three times with dichloromethane (50 mL × 3), and the combined organic phases were evaporated to dryness by rotary evaporation under reduced pressure. The resulting residue was purified by column chromatography (eluent: dichloromethane/methanol = 1/0 to 5.7/1, v/v) to obtain compound WX004-1. MS-ESI m/z: 780.1 [M+H]⁺.

### Step 2: Synthesis of WX004

To *N,N*-dimethylformamide (5 mL) were added compound WX004-1 (280 mg, 309.14 µmol) and acrylamide (26.37 mg, 370.97 µmol, 25.60 µL) at 0°C. To the reaction was slowly added potassium tert-butoxide (173.45 mg, 1.55 mmol) in batches. The mixture was replaced with nitrogen three times, and then stirred at 0°C for 1 hour. The reaction mixture was slowly added with saturated ammonium chloride solution, extracted twice with ethyl acetate (20 mL × 2), and the combined organic phases were washed twice with saturated brine (20 mL × 2). The collected organic phases were dried over anhydrous sodium sulfate, filtered, and evaporated to dryness by rotary evaporation under reduced pressure. The resulting residue was separated by preparative HPLC (mobile phase: acetonitrile/water; acidic system: 0.05% HCl) to obtain target compound WX004. MS-ESI m/z: 805.4 [M+H]⁺. ¹H NMR(400MHz, DMSO-d6)δ: 10.89(s, 1H), 8.40(d, J=8.3 Hz, 1H), 8.28(d, J=1.0 Hz, 1H), 8.07(dd, J=1.4, 8.2 Hz, 1H), 7.85(d, J=6.8 Hz, 1H), 7.53(dd, J=4.0, 7.8 Hz, 1H), 7.49 - 7.32(m, 3H), 7.21(br d, J=8.8 Hz, 1H), 7.15(dd, J=5.5, 7.3 Hz, 1H), 4.53(br d, J=1.0 Hz, 2H), 4.12(dd, J=4.9, 11.9 Hz, 1H), 3.83 - 3.62(m, 5H), 3.27 - 3.06(m, 2H), 2.84 - 2.65(m, 3H), 2.57(br dd, J=4.1, 17.2 Hz, 4H), 2.49 - 2.42(m, 2H), 2.40 - 2.26(m, 1H), 2.19 - 2.01(m, 3H), 1.52(s, 6H).

### Example 5

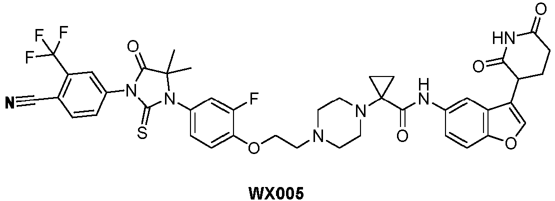

### Synthetic route:

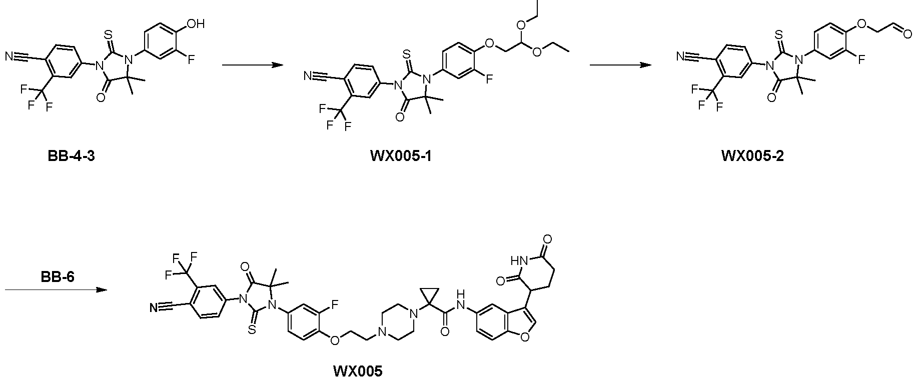

### Step 1: Synthesis of intermediate WX005-1

BB-4-3 (1.00 g, 2.36 mmol) and bromoacetaldehyde diethyl acetal (512.01 mg, 2.60 mmol) were dissolved in N,N-dimethylformamide (10 mL) at room temperature, then potassium carbonate (391.73 mg, 2.83 mmol) was added thereto, and the reaction mixture was stirred and reacted at 80°C for 12 hours under nitrogen atmosphere. After the reaction was completed, the reaction mixture was cooled to room temperature, added with water (50 mL), and extracted with ethyl acetate (50 mL × 3). The organic phases were combined, washed with saturated brine (50 mL × 3) successively, dried over anhydrous sodium sulfate, filtered, and the filtrate was concentrated under reduced pressure to remove the solvent. The resulting residue was separated by column chromatography (eluent: petroleum ether/ethyl acetate = 1/0 to 3/1, v/v) to obtain intermediate WX005-1. ¹H NMR (400 MHz, CDCl₃) δ: 8.01-7.94 (m, 2H), 7.84 (dd, J=2.0, 8.4 Hz, 1H), 7.16-7.10 (m, 1H), 7.09-7.00 (m, 2H), 4.88 (t, J=5.2 Hz, 1H), 4.13 (d, J=5.2 Hz, 2H), 3.86-3.76 (m, 2H), 3.72-3.62 (m, 2H), 1.59 (s, 6H), 1.27 (t, J=7.0 Hz, 6H).

### Step 2: Synthesis of intermediate WX005-2

WX005-1 (500.00 mg, 926.71 µmol) was dissolved in ethanol (20 mL) at room temperature, then acetic acid (5.25 g, 87.42 mmol) and hydrochloric acid (1 M, 2.5 mL) were added thereto, and the reaction mixture was stirred and reacted at 80°C for 12 hours under nitrogen atmosphere. After the reaction was completed, the reaction mixture was cooled to room temperature, concentrated under reduced pressure to remove the solvent (ethanol), added with water (30 mL), and extracted with ethyl acetate (30 mL × 3). The organic phases were combined, washed with saturated brine (30 mL × 3) successively, dried over anhydrous sodium sulfate, filtered, and the filtrate was concentrated under reduced pressure to remove the solvent. The resulting residue was separated by column chromatography (eluent: petroleum ether/ethyl acetate = 1/0 to 1/1, v/v) to obtain intermediate WX005-2. ¹H NMR (400 MHz, CDCl₃) δ: 9.92 (s, 1H), 8.02-7.92 (m, 2H), 7.84 (dd, J=2.0, 8.0 Hz, 1H), 7.19-7.10 (m, 1H), 7.10-7.01 (m, 2H), 4.74 (s, 2H), 1.60 (s, 6H).

### Step 3: Synthesis of compound WX005

To dichloromethane (10 mL) was added compound WX005-2 (40 mg, 85.94 µmol) at 25°C, and the stirring was started. Compound BB-6 (36.80 mg, 77.35 µmol) and sodium triacetoxyborohydride (54.65 mg, 257.83 µmol) were added thereto. The reaction mixture was stirred for another 12 hours under nitrogen atmosphere. After the reaction was completed, the reaction mixture was added with water (50 mL), and extracted with ethyl acetate (50 mL × 2). The organic phases were combined, washed with half-saturated brine (50 mL × 2), dried over anhydrous sodium sulfate, filtered, and evaporated to dryness by rotary evaporation. The resulting residue was separated by preparative HPLC (mobile phase: acetonitrile/water; acidic system: 0.05% HCl) to obtain target compound WX005. ESI m/z: 846.2[M+H]⁺.¹H NMR (400 MHz, DMSO-d6) δ: 10.89 (s, 2H), 9.77 (s, 1H), 8.39 (d, J=8.4 Hz, 1H), 8.27 (d, J=1.25 Hz, 1H), 8.07 (d, J=8.4 Hz, 1 H), 7.96 (d, J=1.2 Hz, 1H), 7.88 (s, 1H), 7.51 - 7.56 (m, 1H), 7.44 - 7.50 (m, 1H), 7.35 - 7.44 (m, 2H), 7.21 -7.23 (m, 1H), 4.61 (s, 2H), 4.11 (dd, J=12.0, 4.8 Hz, 1H), 3.5 -3.60 (m, 4H), 3.50 -3.51 (m, 2H), 2.93 - 3.02 (m, 2H), 2.80 - 2.90 (m, 2H), 2.70 - 2.79 (m, 1H), 2.55 - 2.68 (m, 1H), 2.22 - 2.35 (m, 1H), 2.06 - 2.19 (m, 1H), 1.51 (s, 6H), 1.14 - 1.21 (m, 2H), 1.08-1.10 (m, 2H).

### Example 6

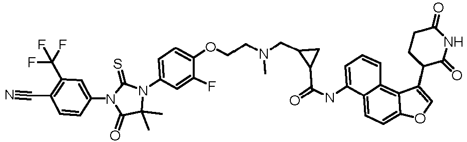

### Synthetic route:

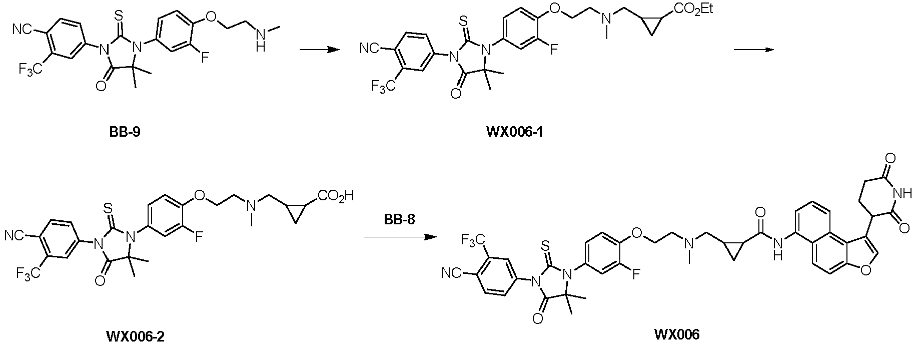

### Step 1: Synthesis of compound WX006-1

Compound BB-9 (71.00 mg, 499.50 µmol, 66.36 µL) and compound ethyl 2-formyl-1-cyclopropanecarboxylate (0.2 g, 416.25 µmol) were dissolved in a mixed solvent of dichloromethane (4 mL) and glacial acetic acid (0.1 mL), then added with sodium triacetoxyborohydride (132.33 mg, 624.38 µmol) at 20°C, and stirred at 20°C for 15 hours. The reaction mixture was added with water (2 mL), stirred for 10 minutes, added with saturated sodium bicarbonate aqueous solution (5 mL), and extracted with dichloromethane (5 mL × 4). The organic phase was washed with saturated brine (10 mL), dried over anhydrous sodium sulfate, filtered, and evaporated to dryness by rotary evaporation. The resulting residue was purified by preparative thin-layer chromatography (developing solvent: dichloromethane/methanol = 10/1) to obtain compound WX006-1.

### Step 2: Synthesis of compound WX006-2

Compound WX006-1 (0.11 g, 181.33 µmol) was dissolved in a mixed solution of tetrahydrofuran (2 mL) and water (0.4 mL), added with lithium hydroxide monohydrate (38.05 mg, 906.65 µmol), and stirred at 20°C for 15 hours. The reaction mixture was added with ethyl acetate (5 mL), and washed with 1 N lithium hydroxide aqueous solution (2 mL × 5). The aqueous phase was added with 1 N dilute hydrochloric acid to adjust the pH to 4 to 5, and extracted with dichloromethane (5 mL × 3). The organic phase was directly dried over anhydrous sodium sulfate, filtered, and evaporated to dryness by rotary evaporation to obtain compound WX006-2.

### Step 3: Synthesis of compound WX006

Compound WX006-2 (90 mg, 155.55 µmol) was dissolved in *N,N-*dimethylformamide (2 mL), added with 2-(7-azabenzotriazol-1-yl)-*N,N,N',N'-*tetramethyluronium hexafluorophosphate (88.72 mg, 233.33 µmol) and *N,N-*diisopropylethylamine (100.52 mg, 777.77 µmol, 135.47 µL), stirred for 10 minutes, then added with hydrochloride of compound BB-8 (54.94 mg, 186.66 µmol), and stirred at 20°C for 60 hours. The reaction mixture was purified by preparative HPLC (mobile phase: acetonitrile/water; neutral system) to obtain compound WX006. ¹H NMR (400 MHz, CD₃OD) δ: 8.15-8.13 (m, 2H), 8.06-8.05 (m, 1H), 7.98 (t, J=10 Hz, 2H), 7.84 (s, 1H), 7.69 (d, J=9.2 Hz, 1H), 7.58 - 7.55 (m, 2H), 7.29 - 7.19 (m, 2H), 7.10- 7.08 (m, 1H), 4.65-4.61 (m, 1H), 4.33 (t, J=5.2 Hz, 2H), 3.07 (t, J=4.8 Hz, 2H), 2.92 - 2.84 (m, 1H), 2.77 - 2.70 (m, 2H), 2.61 - 2.58 (t, 1H), 2.55 (s, 3H) 2.46-2.42 (m, 2H), 1.94-1.91 (m, 1H), 1.67-1.65 (m, 1H), 1.52 (s, 6H), 1.35-1.31 (m, 1H), 0.95-0.90 (m, 1H).

### Example 7

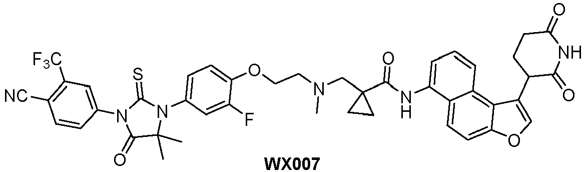

### Synthetic route:

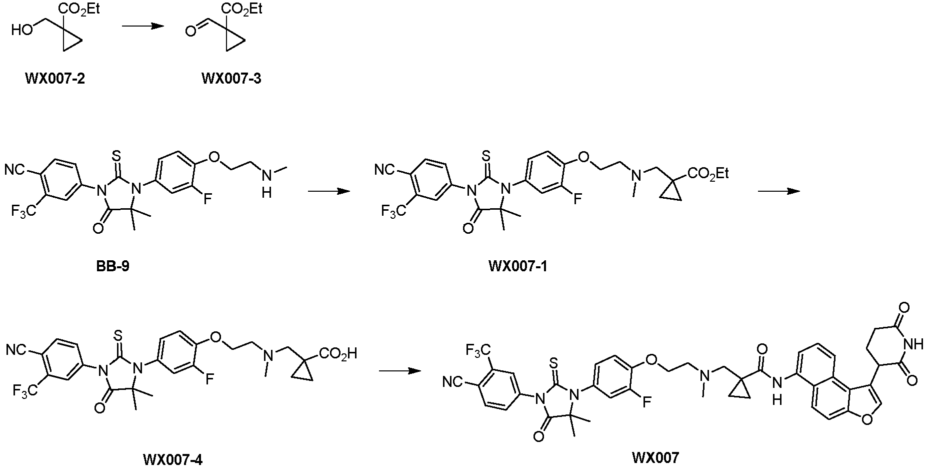

### Step 1: Synthesis of compound WX007-1

Compound WX007-2 (0.5 g, 3.47 mmol) was dissolved in dichloromethane (5 mL), and the reaction mixture was cooled to 0°C, added with Dess-Martin periodinane (2.94 g, 6.94 mmol, 2.15 mL) in batches, slowly warmed to 20°C, and stirred for 2 hours. The reaction mixture was poured into saturated sodium bicarbonate aqueous solution (10 mL) and 10% sodium thiosulfate aqueous solution (30 mL) at 0 to 10°C, and extracted with dichloromethane (10 mL × 3). The organic phase was washed with saturated brine (10 mL), dried over anhydrous sodium sulfate, and filtered to obtain a solution of compound WX007-3. The solution of compound WX007-3 and compound BB-9 (1.4 g, 2.91 mmol) were dissolved in dichloromethane (5 mL) and glacial acetic acid (0.1 mL), then added with sodium triacetoxyborohydride (1.24 g, 5.83 mmol) at 20°C, and stirred for 12 hours. The reaction mixture was added with water (50 mL), and extracted with dichloromethane (30 mL × 3). The organic phase was washed with saturated brine (20 mL × 3), dried over anhydrous sodium sulfate, filtered, and evaporated to dryness by rotary evaporation. The crude product was purified by column chromatography (eluent: petroleum ether/ethyl acetate = 1/1 to 0/1) to obtain compound WX007-1. ¹H NMR (400 MHz, CDCl₃) δ: 8.00-7.96 (m, 2H), 7.85 (dd, J = 1.6 Hz, 8.4 Hz, 1H), 7.14-7.02 (m, 3H), 4.21-4.18 (m, 2H), 4.13 (q, J = 7.2 Hz, 2H), 2.98-2.90 (m, 2H), 2.78 (s, 2H), 2.41 (s, 3H), 1.59 (s, 6H), 1.29-1.23 (m, 5H), 0.88-0.85 (m, 2H).

### Step 2: Synthesis of compound WX007-4

Compound WX007-1 (0.53 g, 873.68 µmol) was dissolved in tetrahydrofuran (5 mL) and water (1 mL), then added with lithium hydroxide monohydrate (183.30 mg, 4.37 mmol), and stirred at 20°C for 24 hours. The reaction mixture was heated to 40°C, and stirred for 24 hours. The reaction mixture was added with 1 N hydrochloric acid to adjust the pH to 6 to 7, evaporated to dryness by rotary evaporation to remove most of tetrahydrofuran, added with methyl tert-butyl ether (30 mL) and 10% potassium carbonate aqueous solution (20 mL × 3), and the phases were separated. The aqueous phase was added with 1 N hydrochloric acid to adjust the pH to 5 to 6, and extracted with dichloromethane (50 mL × 3). The organic phase was dried over anhydrous sodium sulfate, filtered, and evaporated to dryness by rotary evaporation under reduced pressure to obtain compound WX007-4.

### Step 3: Synthesis of compound WX007

Compound WX007-4 (50 mg, 86.42 µmol) and *N,N-*diisopropylethylamine (44.68 mg, 345.68 µmol, 60.21 µL) were dissolved in *N,N*-dimethylformamide (2 mL), added with 2-(7-azabenzotriazol-1-yl)-*N,N,N',N*'-tetramethyluronium hexafluorophosphate (39.43 mg, 103.70 µmol) at 20°C, stirred for 5 minutes, added with hydrochloride of compound BB-8 (34.30 mg, 103.70 µmol), and stirred at 20°C for 12 hours. The reaction mixture was concentrated under reduced pressure to remove the solvent to obtain a crude product, which was purified by preparative HPLC (mobile phase: acetonitrile/water; acidic system: 0.05% HCl) to obtain compound WX007. ¹H NMR (400 MHz, CD₃OD) δ: 8.17-8.15 (m, 3H), 7.98 (d, J=8.4 Hz, 1H), 7.87-7.84 (m, 2H), 7.74 (d, J=9.6 Hz, 1H), 7.62 (t, J=7.6 Hz, 1H), 7.45 (d, J=7.2 Hz, 1H), 7.28-7.25 (m, 2H), 7.12 (d, J=8.8 Hz, 1H), 4.65 (dd, J=5.6 Hz, 11.2 Hz, 1H), 4.52 (t, J=4.4 Hz, 2H), 3.90 (dd, J=4.0 Hz, 13.2 Hz, 1H), 3.83-3.79 (m, 1H), 3.65-3.61 (m, 1H), 3.32-3.29 (m, 1H), 3.08 (s, 3H), 2.94-2.85 (m, 1H), 2.77-2.72 (m, 1H), 2.53-2.43 (m, 2H), 1.92-1.84 (m, 2H), 1.53 (s, 6H), 1.48-1.45 (m, 1H), 1.39-1.35 (m, 1H).

### Example 8

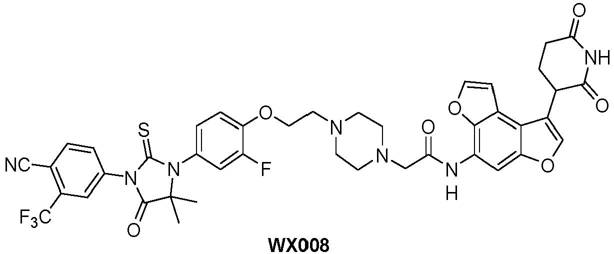

### Synthetic route:

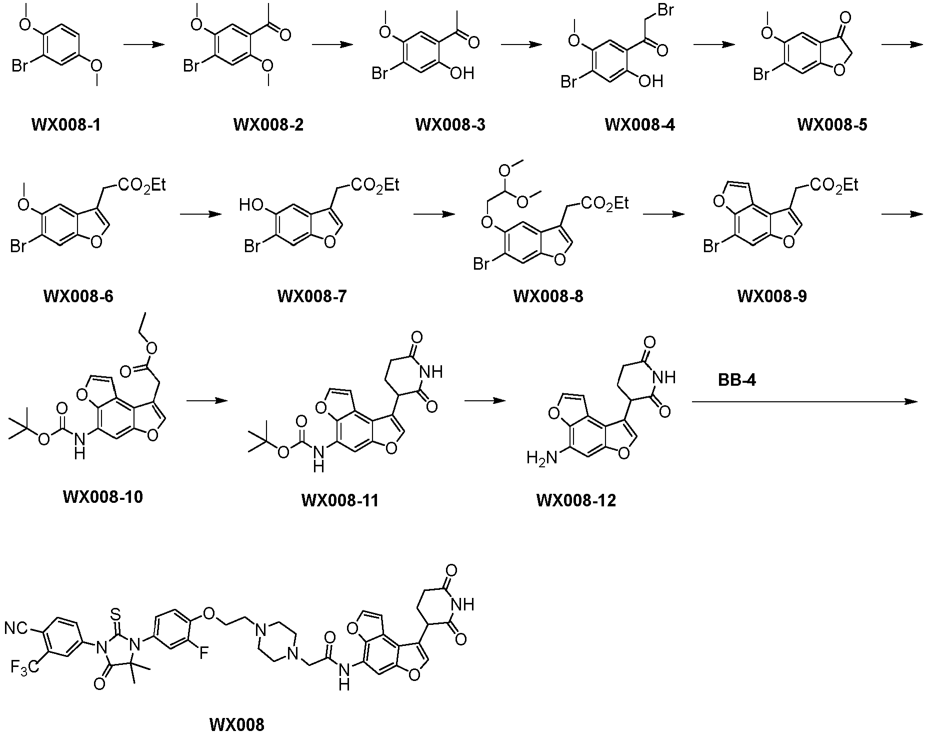

### Step 1: Synthesis of compound WX008-2

Compound WX008-1 (100 g, 460.70 mmol) and acetyl chloride (54.25 g, 691.05 mmol, 49.31 mL) were dissolved in dichloromethane (1000 mL), then the reaction mixture was replaced with nitrogen, added with aluminum chloride (92.15 g, 691.05 mmol, 37.76 mL) in batches at 20°C under nitrogen atmosphere, and stirred for 2 hours. The reaction mixture was poured into 6 N glacial hydrochloric acid (400 mL), and extracted with dichloromethane (700 mL × 3). The organic phase was washed with saturated brine (700 mL × 3), dried over anhydrous sodium sulfate, filtered, and evaporated to dryness by rotary evaporation. The resulting crude product was slurried with methanol (500 mL), stirred for 15 minutes, and filtered. The filter cake was rinsed with methanol (100 mL × 2), and collected to obtain compound WX008-2.

### Step 2: Synthesis of compound WX008-3

WX008-2 (20 g, 77.19 mmol) was dissolved in dichloromethane (200 mL), cooled to -70°C, added dropwise with boron tribromide (17.40 g, 69.47 mmol, 6.69 mL), slowly warmed to 20°C, and stirred for 1 hour. The reaction mixture was poured into 4 N hydrochloric acid (100 mL), and extracted with ethyl acetate (100 mL × 3). The organic phase was washed with saturated brine (100 mL × 3), dried over anhydrous sodium sulfate, filtered, and evaporated to dryness by rotary evaporation. The resulting residue was separated by column chromatography (eluent: petroleum ether/ethyl acetate = 7/1) to obtain compound WX008-3.

### Step 3: Synthesis of compound WX008-4

Compound WX008-3 (13.84 g, 56.47 mmol) was dissolved in chloroform (70 mL) and ethyl acetate (70 mL), added with copper bromide (25.23 g, 112.95 mmol, 5.29 mL), heated to 90°C, and stirred for 16 hours. The reaction mixture was cooled to room temperature, filtered, and the filter cake was rinsed with dichloromethane (110 mL) to obtain a solution of compound WX008-4 in dichloromethane.

### Step 4: Synthesis of compound WX008-5

To the solution of compound WX008-4 in dichloromethane was slowly added dropwise triethylamine (11.43 g, 112.97 mmol, 15.72 mL) at 0°C. After the dropwise addition was completed, the reaction mixture was warmed to 20°C, and stirred for 2 hours. The reaction mixture was washed with saturated ammonium chloride aqueous solution (100 mL × 3), then the organic phase was dried over anhydrous sodium sulfate, and filtered to obtain a solution of compound WX008-5 in dichloromethane.

### Step 5: Synthesis of compound WX008-6

To the solution of WX008-5 in dichloromethane were added toluene (150 mL) and (ethoxycarbonylmethylene)triphenylphosphorane (23.62 g, 67.79 mmol), and heated to 130°C. The low-boiling-point solvent was separated with a water separator, and the reaction mixture was stirred for 12 hours. The reaction mixture was cooled to room temperature, and directly concentrated under reduced pressure to obtain a crude product. The crude product was separated by column chromatography (eluent: petroleum ether/ethyl acetate = 10/1) to obtain compound WX008-6. ¹H NMR (400 MHz, CDCl₃) δ: 7.70 (s, 1H), 7.60 (s, 1H), 7.05 (s, 1H), 4.20 (q, J = 7.2 Hz, 2H), 3.95 (s, 3H), 3.67 (s, 2H), 1.28 (t, J = 7.2 Hz, 3H).

### Step 6: Synthesis of compound WX008-7

Compound WX008-6 (4 g, 12.77 mmol) was dissolved in dichloromethane (40 mL), and the mixture was cooled to -70°C, added dropwise with boron tribromide (3.36 g, 13.41 mmol, 1.29 mL), stirred for 0.5 hours at the same temperature, slowly warmed to 0°C, and stirred for 1 hour. The reaction mixture was cooled to -70°C, added with additional boron tribromide (3.36 g), slowly warmed to 0°C, and stirred for 1 hour. The reaction mixture was poured into 1 N hydrochloric acid (50 mL), and extracted with dichloromethane (50 mL × 3). The organic phase was washed with saturated brine (50 mL × 3), dried over anhydrous sodium sulfate, filtered, and evaporated to dryness by rotary evaporation. The crude product was separated by column chromatography (eluent: petroleum ether/ethyl acetate = 5/1) to obtain compound WX008-7.

### Step 7: Synthesis of compound WX008-8

Compound WX008-7 (1.8 g, 6.02 mmol) and potassium carbonate (1.83 g, 13.24 mmol) were placed in N,N-dimethylformamide (10 mL), added with 2-bromo-1,1-dimethoxyethane (1.22 g, 7.22 mmol, 847.58 µL), heated to 100°C, and stirred for 12 hours. The reaction mixture was cooled to room temperature, added with water (50 mL), and extracted with ethyl acetate (30 mL × 3). The organic phase was washed with saturated brine (30 mL × 2), dried over anhydrous sodium sulfate, filtered, and evaporated to dryness by rotary evaporation. The crude product was separated by column chromatography (eluent: petroleum ether/ethyl acetate = 10/1) to obtain compound WX008-8.

### Step 8: Synthesis of compound WX008-9

Compound WX008-8 (1.84 g, 4.75 mmol) was placed in toluene (20 mL), added with polyphosphoric acid (1.84 g, 8.74 mmol), heated to 100°C, and stirred for 12 hours. The reaction mixture was cooled to room temperature, added with water (30 mL), and extracted with ethyl acetate (30 mL × 3). The organic phase was washed with saturated sodium bicarbonate aqueous solution (30 mL), dried over anhydrous sodium sulfate, filtered, and evaporated to dryness by rotary evaporation. The crude product was separated by column chromatography (eluent: petroleum ether/ethyl acetate = 10/1) to obtain compound WX008-9. ¹H NMR (400 MHz, CDCl₃) δ: 7.81 (d, J = 2.4 Hz, 1H), 7.71 (m, 1H), 7.66 (s, 1H), 7.13 (d, J = 2.0 Hz, 1H), 4.20 (q, J = 7.2 Hz, 2H), 3.85 (d, J = 0.8 Hz, 2H), 1.25 (t, J = 7.2 Hz, 3H).

### Step 9: Synthesis of compound WX008-10

Compound WX008-9 (700 mg, 2.17 mmol), 2-di-tert-butylphosphino-2',4',6'-triisopropylbiphenyl (128.78 mg, 303.28 µmol), tert-butyl carbamate (304.53 mg, 2.60 mmol), potassium phosphate (1.84 g, 8.67 mmol), and tris(dibenzylideneacetone)dipalladium (138.86 mg, 151.64 µmol) were placed in toluene (10 mL) and water (2 mL), then the reaction mixture was replaced with nitrogen, heated to 100°C, and stirred for 6 hours under nitrogen atmosphere. The reaction mixture was cooled to room temperature, added with water (20 mL), and extracted with ethyl acetate (20 mL × 3). The organic phase was washed with saturated brine (20 mL × 2), dried over anhydrous sodium sulfate, filtered, and evaporated to dryness by rotary evaporation. The crude product was added with n-heptane (10 mL), stirred for 15 minutes, and filtered. The filter cake was rinsed with n-heptane (3 mL × 3), and collected to obtain compound WX008-10.

### Step 10: Synthesis of compound WX008-11

Compound WX008-10 (760 mg, 2.11 mmol) and acrylamide (165.35 mg, 2.33 mmol, 160.53 µL) were dissolved in N,N-dimethylformamide (20 mL), added with potassium tert-butoxide (498.34 mg, 4.44 mmol) at 20°C, and stirred for 1 hour at 20°C. The reaction mixture was poured into 1 N hydrochloric acid (20 mL) at 0 to 10°C, and extracted with ethyl acetate (20 mL × 3). The organic phase was washed with saturated brine (20 mL), dried over anhydrous sodium sulfate, filtered, and evaporated to dryness by rotary evaporation. The crude product was separated by column chromatography (eluent: petroleum ether/ethyl acetate = 1/1) to obtain compound WX008-11.

### Step 11: Synthesis of hydrochloride of compound WX008-12

Compound WX008-11 (240 mg, 624.38 µmol) was placed in ethyl acetate (1 mL), added with hydrochloric acid/ethyl acetate (4 M, 10 mL), and stirred at 20°C for 3 hours. The reaction mixture was directly concentrated under reduced pressure to obtain hydrochloride of compound WX008-12. ¹H NMR (400 MHz, DMSO-d6) δ: 10.94 (s, 1H), 8.16 (d, J = 2.0 Hz, 1H), 7.87 (s, 1H), 7.26 (s, 1H), 7.09 (d, J = 2.0 Hz, 1H), 4.29 (dd, J = 4.8 Hz, 12.0 Hz, 1H), 2.86-2.77 (m, 1H), 2.68-2.58 (m, 1H), 2.34-2.22 (m, 1H), 2.19-2.14 (m, 1H).

### Step 12: Synthesis of compound WX008

Compound BB-4 (125.29 mg, 211.07 µmol) and *N,N*-diisopropylethylamine (90.93 mg, 703.57 µmol) were dissolved in *N,N*-dimethylformamide (2 mL), added with 2-(azabenzotriazol-1-yl)-*N,N,N',N*'-tetramethyluronium hexafluorophosphate (86.94 mg, 228.66 µmol) at 20°C, and stirred for 5 minutes. The reaction mixture was added with hydrochloride of compound WX008-12 (50 mg, 175.89 µmol) at 20°C, and stirred for 12 hours. The reaction mixture was directly concentrated under reduced pressure. The crude product was purified by preparative HPLC (mobile phase: acetonitrile/water; acidic system: 0.05% HCl) to obtain compound WX008. ¹H NMR (400 MHz, CD₃OD) δ: 8.19 (s, 1H), 8.17-8.15 (m, 2H), 7.98 (dd, J = 1.6 Hz, 8.0 Hz, 1H), 7.91 (d, J = 2.0 Hz, 1H), 7.81 (s, 1H), 7.40-7.35 (m, 1H), 7.32 (dd, J = 2.4 Hz, 11.6 Hz, 1H), 7.25-7.22 (m, 1H), 7.02 (d, J = 2.0 Hz, 1H), 4.60-4.58 (m, 2H), 4.29 (dd, J = 5.2 Hz, 11.6 Hz, 1H), 3.92 (s, 2H), 3.77-3.69 (m, 6H), 3.42 (m, 4H), 2.92-2.83 (m, 1H), 2.79-2.73 (m, 1H), 2.44-2.30 (m, 2H), 1.57 (s, 6H).

### Example 9

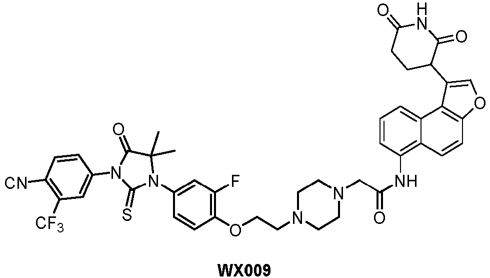

### Synthetic route:

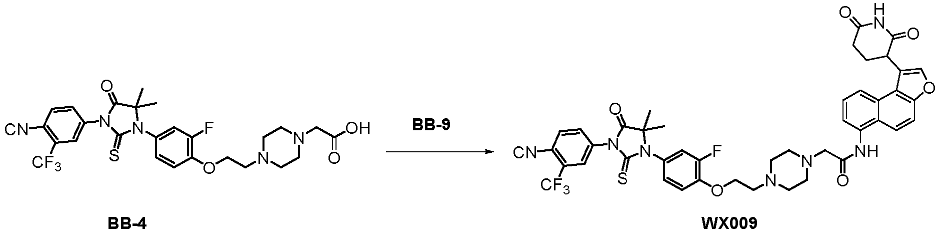

### Synthesis of compound WX009

To N,N-dimethylformamide (10 mL) were added compound BB-4 (200 mg, 336.93 µmol) and hydrochloride of BB-8 (133.73 mg, 404.32 µmol). To the reaction were slowly added 2-(7-azabenzotriazol-1-yl)-*N,N,N',N'*-tetramethyluronium hexafluorophosphate (256.22 mg, 673.86 µmol), and *N,N-*diisopropylethylamine (130.64 mg, 1.01 mmol). The mixture was replaced with nitrogen three times, and stirred at 20°C for 12 hours. The reaction mixture was poured into water (10 mL) and ethyl acetate (10 mL), and the separated organic phase was washed three times with water (10 mL × 3). The collected organic phase was dried over anhydrous sodium sulfate, filtered, and evaporated to dryness by rotary evaporation under reduced pressure. The crude product was purified by preparative HPLC (mobile phase: acetonitrile/water; acidic system: 0.05% HCl) to obtain compound WX009. ¹H NMR (400 MHz, DMSO-d6) δ: 10.95 (s, 1H), 10.69 - 10.37 (m, 1H), 8.39 (d, J=8.0 Hz, 1H), 8.27 (d, J=1.5 Hz, 1H), 8.17 - 7.97 (m, 4H), 7.86 (d, J=9.3 Hz, 1H), 7.68 - 7.54 (m, 2H), 7.49 - 7.33 (m, 2H), 7.22 (br d, J=8.0 Hz, 1H), 4.70 (br dd, J=4.3, 11.8 Hz, 1H), 4.66 - 4.46 (m, 2H), 4.28 - 3.98 (m, 2H), 3.81 - 3.61 (m, 10H), 3.00 - 2.81 (m, 1H), 2.72 - 2.60 (m, 1H), 2.47 - 2.37 (m, 1H), 2.35 - 2.21 (m, 1H), 1.51 (s, 6H).

### Example 10

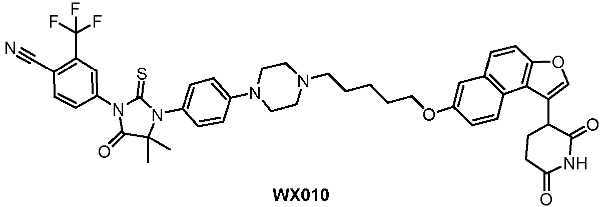

### Synthetic route:

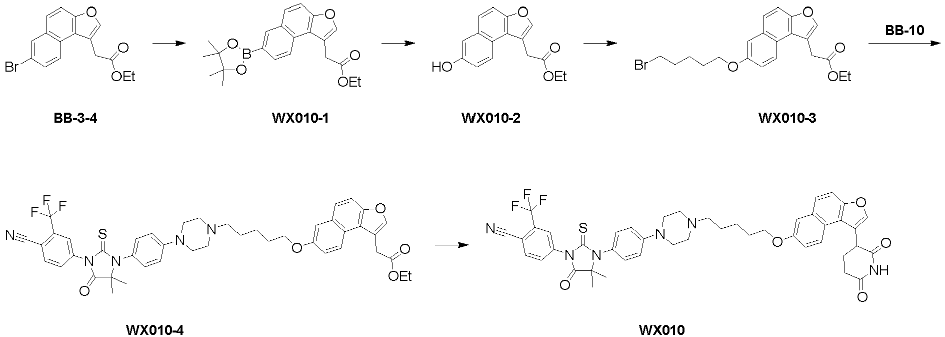

### Step 1: Synthesis of compound WX010-1

To dioxane (20 mL) solvent was added compound BB-3-4 (2 g, 6.00 mmol) at room temperature, and the stirring was started. Compound bis(pinacolato)diboron (2.29 g, 9.00 mmol), [1,1'-bis(diphenylphosphino)ferrocene]palladium dichloride (439.23 mg, 600.28 µmol), and potassium acetate (1.18 g, 12.01 mmol) were added thereto, and the reaction mixture was heated to 100°C, and stirred for 12 hours under nitrogen atmosphere. After the reaction was completed, the reaction mixture was cooled to room temperature, added with water (30 mL), and extracted with ethyl acetate (30 mL × 2). The organic phases were combined, washed with half-saturated brine (30 mL × 2), dried over anhydrous sodium sulfate, filtered, and evaporated to dryness by rotary evaporation. The resulting crude product was purified by column chromatography (eluent: petroleum ether/ethyl acetate = 1/0 to 4/1) to obtain compound WX010-1. MS-ESI m/z: 380.9[M+H]⁺. ¹H NMR (400MHz, CDCl₃) δ: 8.47 (s, 1H), 8.19 (d, J=8.5 Hz, 1H), 7.94 (d, J=8.3 Hz, 1H), 7.83 - 7.75 (m, 2H), 7.65 (d, J=9.0 Hz, 1H), 4.23 (q, J=7.1 Hz, 2H), 4.09 - 4.09 (m, 1H), 4.08 (s, 1H), 1.41 (s, 12H), 1.30 - 1.25 (m, 3H).

### Step 2: Synthesis of compound WX010-2

To a mixed solvent of tetrahydrofuran (40 mL) and water (20 mL) was added compound WX010-1 (2.3 g, 6.05 mmol) at room temperature, and the stirring was started. Compound sodium bicarbonate (1.02 g, 12.10 mmol, 470.52 µL) was added thereto, and the reaction mixture was cooled to 0°C, slowly added dropwise with hydrogen peroxide (5.49 g, 48.39 mmol, 4.65 mL, concentration: 30%), and stirred for another 2 hours under nitrogen atmosphere. The reaction mixture was slowly added dropwise with saturated sodium sulfite aqueous solution (50 mL) under an ice bath. After the dropwise addition was completed, the reaction mixture was stirred for another 10 minutes. The reaction mixture was extracted with ethyl acetate (100 mL × 2). The organic phase was dried over anhydrous sodium sulfate, filtered, and evaporated to dryness by rotary evaporation. The crude product was purified by column chromatography (eluent: petroleum ether/ethyl acetate = 8/1 to 4/1) to obtain compound WX010-2. ¹H NMR (400MHz, CDCl₃) δ: 8.13 (d, J=8.9 Hz, 1H), 7.74 (s, 1H), 7.64 - 7.51 (m, 2H), 7.29 (d, J=2.6 Hz, 1H), 7.18 (dd, J=2.6, 8.9 Hz, 1H), 4.23 (q, J=7.1 Hz, 2H), 4.05 (d, J=0.9 Hz, 2H), 1.31 - 1.22 (m, 3H).

### Step 3: Synthesis of compound WX010-3

To N,N-dimethylformamide (10 mL) were added compound WX010-2 (400 mg, 1.33 mmol) and 1,5-dibromopentane (1.53 g, 6.64 mmol, 897.79 µL), and the stirring was started. To the reaction were slowly added potassium carbonate (550.41 mg, 3.98 mmol) and sodium iodide (198.99 mg, 1.33 mmol). The mixture was replaced with nitrogen three times, and then stirred at 80°C for 10 hours. The reaction mixture was cooled to 25°C, then ethyl acetate (30 mL) and water (30 mL) were added thereto, and the separated organic phase was washed twice with saturated brine (30 mL × 2). The collected organic phase was dried over anhydrous sodium sulfate, filtered, and evaporated to dryness by rotary evaporation under reduced pressure. The resulting crude product was purified by column chromatography (eluent: petroleum ether/ethyl acetate = 50/1 to 10/1) to obtain compound WX010-3. MS-ESI m/z: 418.9[M+H]⁺. ¹H NMR (400MHz,CDCl₃) δ: 8.06 (d, J=9.0 Hz, 1H), 7.66 (s, 1H), 7.53 (s, 2H), 7.25 - 7.10 (m, 3H), 4.17 - 4.08 (m, 3H), 4.03 (t, J=6.3 Hz, 2H), 3.96 (s, 2H), 3.39 (t, J=6.8 Hz, 2H), 1.97 - 1.86 (m, 2H), 1.85 - 1.77 (m, 2H), 1.69 - 1.57 (m, 3H), 1.18 (t, J=7.2 Hz, 3H).

### Step 4: Synthesis of compound WX010-4

To N,N-dimethylformamide (5 mL) were added compound BB-10 (200 mg, 422.37 µmol) and WX010-3 (177.11 mg, 422.37 µmol, 72.61 µL), and the stirring was started. To the reaction were slowly added potassium carbonate (175.12 mg, 1.27 mmol) and potassium iodide (70.12 mg, 422.37 µmol). The reaction mixture was replaced with nitrogen three times, and then stirred at 80°C for 12 hours. The reaction mixture was cooled to room temperature, diluted with ethyl acetate (30 mL), and the organic phase was washed twice with water (20 mL × 2). The collected organic phase was dried over anhydrous sodium sulfate, filtered, and evaporated to dryness by rotary evaporation under reduced pressure. The resulting crude product was purified by column chromatography (eluent: dichloromethane/methanol = 50/1 to 10/1) to obtain compound WX010-4.

MS-ESI m/z: 812.3 [M+H]⁺.

### Step 5: Synthesis of compound WX010

To N,N-dimethylformamide (10 mL) were added compound WX010-4 (300 mg, 369.50 µmol) and acrylamide (26.26 mg, 369.50 µmol, 25.50 µL) at 0°C. To the reaction was slowly added potassium tert-butoxide (82.92 mg, 739.00 µmol) in batches. The mixture was replaced with nitrogen three times, and then stirred at 0°C for 1 hour. The reaction mixture was slowly added with saturated ammonium chloride (50 mL), and extracted twice with ethyl acetate (50 mL × 2). The organic phases were combined, washed three times with saturated brine (30 mL × 3), dried over anhydrous sodium sulfate, filtered, and evaporated to dryness by rotary evaporation under reduced pressure. The crude product was separated by preparative HPLC (mobile phase: acetonitrile/water; acidic system: 0.05% HCl) to obtain compound WX010. MS-ESI m/z: 837.2 [M+H]⁺. ¹H NMR (400MHz, DMSO-d6) δ: 11.23 (br s, 1H), 10.95 (s, 1H), 8.39 (d, J=8.3 Hz, 1H), 8.30 (d, J=1.3 Hz, 1H), 8.15 - 8.04 (m, 2H), 7.98 (d, J=6.8 Hz, 1H), 7.82 - 7.68 (m, 2H), 7.53 (d, J=2.3 Hz, 1H), 7.34 - 7.20 (m, 3H), 7.14 (br d, J=9.0 Hz, 2H), 4.65 (br dd, J=4.1, 11.9 Hz, 1H), 4.18 - 4.09 (m, 2H), 4.03 (s, 1H), 3.93 (br s, 2H), 3.60 (br d, J=11.3 Hz, 2H), 3.38 - 3.23 (m, 2H), 3.22 - 3.04 (m, 4H), 2.98 - 2.79 (m, 1H), 2.71 - 2.53 (m, 1H), 2.41 (br dd, J=3.9, 12.7 Hz, 1H), 2.31 - 2.17 (m, 1H), 1.95 - 1.76 (m, 4H), 1.64 - 1.43 (m, 8H).

### Example 11

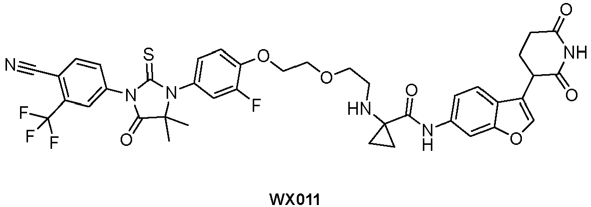

### Synthetic route:

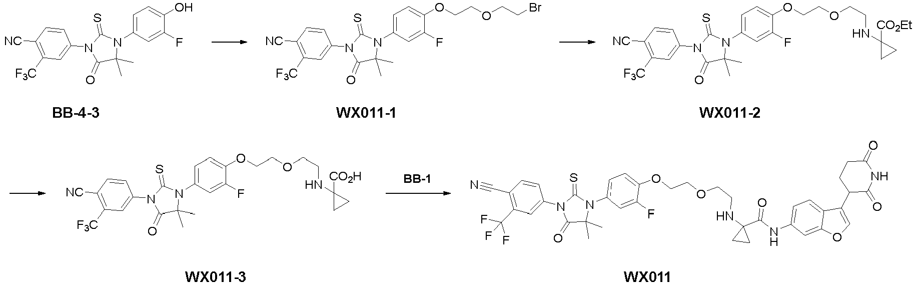

### Step 1: Synthesis of compound WX011-1

Compound BB-4-3 (1 g, 2.36 mmol) was dissolved in N,N-dimethylformamide (30 mL), added with potassium carbonate (652.86 mg, 4.72 mmol) and 2,2'-dibromodiethyl ether (2.74 g, 11.81 mmol, 1.48 mL), heated to 80°C, and stirred for 30 hours. The reaction mixture was added with water (150 mL), and extracted with ethyl acetate (30 mL × 3). The organic phase was washed with saturated brine (50 mL), dried over anhydrous sodium sulfate, filtered, and evaporated to dryness by rotary evaporation. The crude product was purified by column chromatography (eluent: petroleum ether/ethyl acetate = 3/1) to obtain compound WX011-1.

### Step 2: Synthesis of compound WX011-2

Ethyl 1-aminocyclopropanecarboxylate hydrochloride (346.01 mg, 2.09 mmol) and potassium carbonate (577.48 mg, 4.18 mmol) were dissolved in N,N-dimethylformamide (10 mL), added with compound WX011-1 (0.6 g, 1.04 mmol), heated to 75°C, and stirred for 15 hours. The reaction mixture was added with water (50 mL), and extracted with ethyl acetate (15 mL × 2). The organic phase was washed with saturated brine (30 mL), dried over anhydrous sodium sulfate, filtered, and evaporated to dryness by rotary evaporation. The crude product was purified by column chromatography (eluent: petroleum ether/ethyl acetate = 3/1) to obtain compound WX011-2.

### Step 3: Synthesis of compound WX011-3

Compound WX011-2 (0.2 g, 321.22 µmol) was dissolved in a mixed solution of tetrahydrofuran (4 mL) and water (1 mL), added with lithium hydroxide monohydrate (67.40 mg, 1.61 mmol) at 20°C, and stirred for 30 hours. The reaction mixture was added with water (5 mL), then added with 1 N dilute hydrochloric acid to adjust the pH to 5, and extracted with ethyl acetate (10 mL × 4). The organic phase was washed with saturated brine (10 mL), dried over anhydrous sodium sulfate, filtered, and evaporated to dryness by rotary evaporation to obtain compound WX011-3.

### Step 4: Synthesis of compound WX011

Compound WX011-3 (80 mg, 134.55 µmol) was dissolved in*N,N*-dimethylformamide (1 mL), added with 2-(7-azabenzotriazol-1-yl)-*N,N,N',N*'-tetramethyluronium hexafluorophosphate (76.74 mg, 201.82 µmol) and *N,N*-diisopropylethylamine (69.56 mg, 538.20 µmol, 93.74 µL), stirred for 10 minutes, then added with hydrochloride of compound BB-1 (49.10 mg, 174.91 µmol), and stirred at 20°C for 15 hours. The reaction mixture was purified by preparative HPLC (mobile phase: acetonitrile/water; acidic system: 0.05% HCl) to obtain compound WX011. ¹H NMR (400 MHz, CD₃OD) δ: 8.16-8.14 (m, 2H), 7.95 (dd, J=1.6 Hz, 8.4 Hz, 1H), 7.91 (d, J = 1.6 Hz, 1H), 7.71 (s, 1H), 7.50 (d, J = 8.8 Hz, 1H), 7.30-7.22 (m, 3H), 7.16-7.14(m, 1H), 4.35 (t, J = 4.0 Hz, 2H), 4.10 (dd, J=4.2 Hz, 11.6 Hz, 1H), 3.99-3.97 (m, 2H), 3.89 (t, J = 2.8 Hz, 2H), 3.47 (s, 2H), 2.83-2.66 (m, 2H), 2.42-2.32 (m, 1H), 2.29-2.22 (m, 1H), 1.81-1.78 (m, 2H), 1.65-1.63 (m, 2H), 1.54 (s, 9H).

### Example 12

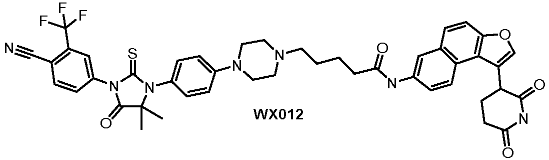

### Synthetic route:

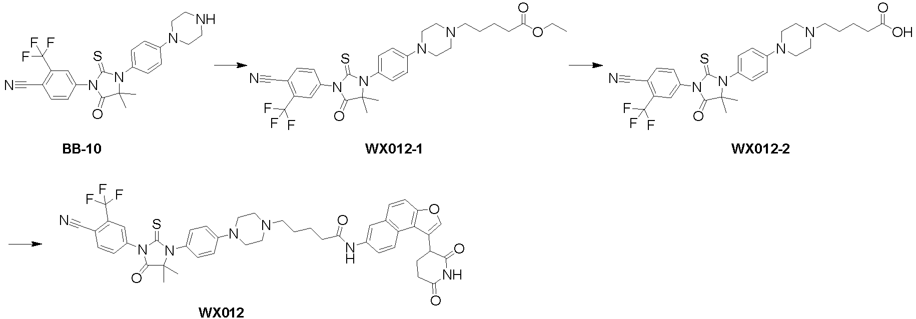

### Step 1: Synthesis of compound WX012-1

To *N,N*-dimethylformamide (5 mL) were added compound BB-10 and ethyl 5-bromovalerate (662.33 mg, 3.17 mmol, 505.59 µL), and the stirring was started. To the reaction were slowly added potassium carbonate (262.69 mg, 1.90 mmol) and sodium iodide (94.97 mg, 633.56 µmol). The reaction mixture was replaced with nitrogen three times, and then stirred at 80°C for 12 hours. After the reaction was completed, the reaction mixture was cooled to 25°C, diluted with ethyl acetate (20 mL). The organic phase was washed twice with water (20 mL × 2), and washed once with saturated brine (20 mL). The collected organic phase was dried over anhydrous sodium sulfate, filtered, and evaporated to dryness by rotary evaporation under reduced pressure. The crude product was purified by column chromatography (eluent: dichloromethane/methanol = 50/1 to 10/1) to obtain compound WX012-1. MS-ESI m/z: 602.1[M+H]⁺.

### Step 2: Synthesis of compound WX012-2

To anhydrous ethanol (2.5 mL) and water (0.5 mL) was added compound WX012-1 (170 mg, 282.54 µmol), and the stirring was started. To the reaction was slowly added lithium hydroxide monohydrate (29.64 mg, 706.35 µmol). The reaction mixture was replaced with nitrogen three times, and stirred at 25°C for 12 hours. After the reaction was completed, the reaction mixture was added with water (10 mL), and evaporated to dryness by rotary evaporation under reduced pressure to remove most of the organic solvent. The aqueous phase was added with 2 N hydrochloric acid to adjust the pH to 3, and directly lyophilized. Compound WX012-2 was obtained. MS-ESI m/z: 574.0[M+H]⁺.

### Step 3: Synthesis of hydrochloride of compound WX012

To N,N-dimethylformamide (5 mL) was added hydrochloride of compound BB-3 (54.30 mg, 139.46 µmol). To the reaction were slowly added 2-(azabenzotriazol-1-yl)-*N,N,N',N*'-tetramethyluronium hexafluorophosphate (165.71 mg, 435.82 µmol) and triethylamine (52.92 mg, 522.99 µmol, 72.79 µL). The mixture was replaced with nitrogen three times, and stirred at 25°C for 3 hours. The reaction mixture was diluted with ethyl acetate (30 mL), and the organic phase was washed twice with water (20 mL × 2). The collected organic phase was dried over anhydrous sodium sulfate, filtered, and evaporated to dryness by rotary evaporation under reduced pressure. The resulting crude product was separated by preparative HPLC (mobile phase: acetonitrile/water; acidic system: 0.05% HCl) to obtain hydrochloride of compound WX012. MS-ESI m/z: 850.4[M+H]⁺. ¹H NMR (400MHz, DMSO-d6) δ:10.95 (s, 1H), 10.54 (br d, J=4.5 Hz, 1H), 10.29 (s, 1H), 8.48 - 8.35 (m, 2H), 8.29 (d, J=1.3 Hz, 1H), 8.18 - 8.04 (m, 2H), 7.99 (s, 1H), 7.79 - 7.69 (m, 3H), 7.28 - 7.21 (m, 2H), 7.14 (d, J=9.0 Hz, 2H), 4.65 (br dd, J=4.4, 12.2 Hz, 1H), 3.94 (br d, J=11.3 Hz, 2H), 3.62 (br d, J=10.8 Hz, 2H), 3.28 - 3.07 (m, 6H), 2.95 - 2.80 (m, 1H), 2.69 - 2.59 (m, 1H), 2.49 - 2.36 (m, 3H), 2.33 - 2.22 (m, 1H), 1.92 - 1.77 (m, 2H), 1.76 - 1.65 (m, 2H), 1.49 (s, 6H).

### Example 13

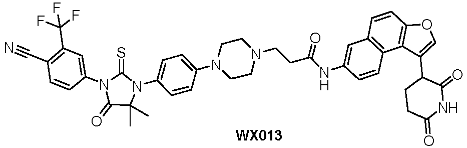

### Synthetic route:

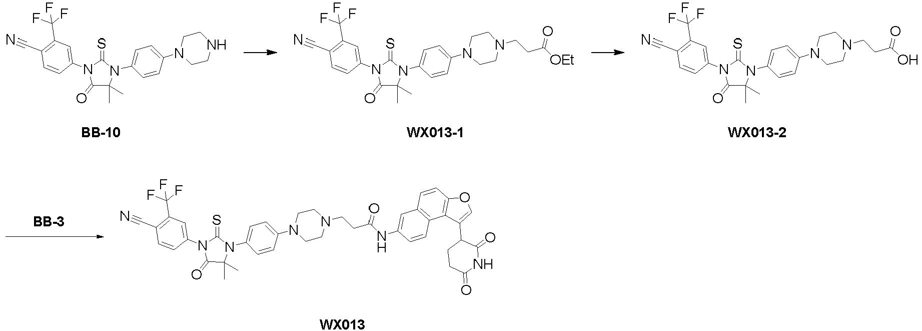

### Step 1: Synthesis of compound WX013-1

To acetonitrile (5 mL) were added compound BB-10 (0.15 g, 284.79 µmol) and ethyl bromopropionate (103.11 mg, 569.57 µmol, 72.61 µL), and the stirring was started. To the reaction were slowly added potassium carbonate (78.72 mg, 569.57 µmol) and potassium iodide (47.28 mg, 284.79 µmol). The reaction mixture was replaced with nitrogen three times, and then stirred at 80°C for 2 hours. The reaction mixture was cooled to room temperature, then ethyl acetate (50 mL) and water (50 mL) were added thereto, and the separated organic phase was washed twice with saturated brine (30 mL × 2). The collected organic phase was dried over anhydrous sodium sulfate, filtered, and evaporated to dryness by rotary evaporation under reduced pressure. The crude product was purified by column chromatography (eluent: dichloromethane/methanol = 50/1 to 10/1, v/v) to obtain compound WX013-1. MS-ESI m/z: 574.0 [M+H]⁺.

### Step 2: Synthesis of compound WX013-2

To anhydrous ethanol (4 mL) and water (1 mL) was added compound WX013-1 (0.2 g, 348.66 µmol), and the stirring was started. To the reaction was slowly added lithium hydroxide monohydrate (43.89 mg, 1.05 mmol). The reaction mixture was replaced with nitrogen three times, and stirred at 25°C for 12 hours. The reaction mixture was evaporated to dryness by rotary evaporation under reduced pressure to remove most of the ethanol, and water (20 mL) was added thereto. The aqueous phase was added with 2 N hydrochloric acid to adjust the pH to 3 to 4, and directly lyophilized. Compound WX013-2 was obtained. MS-ESI m/z: 546.0 [M+H]⁺.

### Step 3: Synthesis of hydrochloride of compound WX013

To N,N-dimethylformamide (3 mL) were added compound WX013-2 (60 mg, 109.98 µmol) and hydrochloride of BB-3. To the reaction were slowly added 2-(7-azabenzotriazol-1-yl)-N,N,N',N'-tetramethyluronium hexafluorophosphate (83.63 mg, 219.95 µmol) and *N,N-*diisopropylethylamine (42.64 mg, 329.93 µmol, 57.47 µL). The mixture was replaced with nitrogen three times, and then stirred at 20°C for 12 hours. After the reaction was completed, the reaction mixture was poured into a mixed solvent of water and ethyl acetate (10 mL:10 mL), and the separated organic phase was washed three times with water (10 mL × 3). The collected organic phase was dried over anhydrous sodium sulfate, filtered, and evaporated to dryness by rotary evaporation under reduced pressure. The resulting crude product was purified by preparative HPLC (mobile phase: acetonitrile/water; acidic system: 0.05% HCl) to obtain hydrochloride of compound WX013. MS-ESI m/z: 822.3 [M+H]⁺.

¹H NMR (400MHz, DMSO-d6) δ: 10.95 (s, 1H), 10.54 (br s, 1H), 10.46 (br s, 1H), 8.47 - 8.35 (m, 2H), 8.29 (br s, 1H), 8.16 (br s, 1H), 8.08 (br d, J=8.5 Hz, 1H), 8.00 (s, 1H), 7.80 - 7.67 (m, 3H), 7.34 - 7.20 (m, 2H), 7.16 (br d, J=8.8 Hz, 2H), 4.68 (br s, 1H), 3.98 (m, 2H), 3.64 (m, 2H), 3.55 (m, 4H), 3.23 (m, 2H), 3.05 (br s, 2H), 2.86 (m, 1H), 2.67 (m, 1H), 2.63 - 2.62 (m, 1H), 2.41 - 2.33 (m, 1H), 1.49 (br s, 6H).

### Example 14

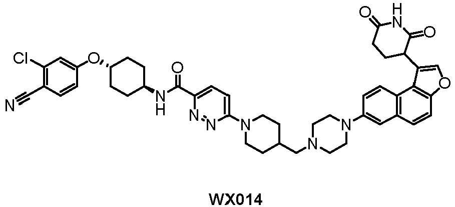

### Synthetic route:

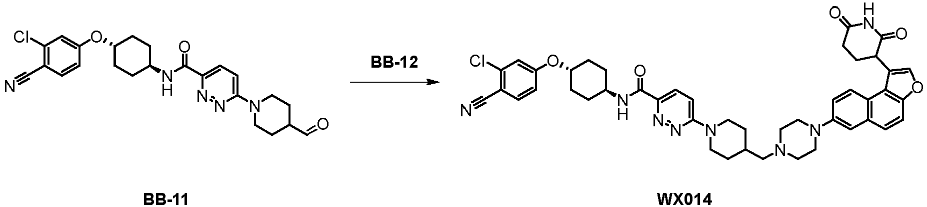

### Synthesis of hydrochloride of compound WX014

Compound BB-11 (400 mg, 854.80 µmol) and hydrochloride of compound BB-12 (403.83 mg, 1.11 mmol) were dissolved in dichloromethane (30 mL), then added with sodium triacetoxyborohydride (543.50 mg, 2.56 mmol), and the reaction mixture was stirred at room temperature for 12 hours under nitrogen atmosphere. After the reaction was completed, the reaction mixture was diluted with dichloromethane (100 mL) and water (100 mL). The organic phase was separated, dried over anhydrous sodium sulfate, filtered, and concentrated under reduced pressure. The resulting crude product was purified by preparative HPLC (mobile phase: acetonitrile/water; acidic system: 0.05% HCl) to obtain hydrochloride of compound WX014. MS-ESI m/z: 815.2 [M+H]⁺.¹H NMR (400MHz, DMSO-d6) δ: 10.97 (s, 1H), 10.83 (br s, 1H), 8.63 (d, J=8.0 Hz, 1H), 8.05 (br d, J=8.5 Hz, 1H), 7.96 (s, 1H), 7.91 - 7.84 (m, 2H), 7.73 (s, 2H), 7.54 - 7.43 (m, 3H), 7.41 - 7.38 (m, 1H), 7.16 - 7.11 (m, 1H), 4.63 (br dd, J=4.0, 12.0 Hz, 1H), 4.58 - 4.46 (m, 3H), 3.98 - 3.81 (m, 3H), 3.66 (br d, J=11.0 Hz, 2H), 3.43 (br t, J=12.2 Hz, 2H), 3.28 - 3.05 (m, 6H), 2.96 - 2.83 (m, 1H), 2.76 - 2.58 (m, 2H), 2.56 - 2.53 (m, 1H), 2.46 - 2.37 (m, 1H), 2.31 - 2.21 (m, 2H), 2.11 (br d, J=9.0 Hz, 2H), 2.02 (br d, J=12.0 Hz, 2H), 1.90 (br d, J=10.5 Hz, 2H), 1.72 - 1.58 (m, 2H), 1.58 - 1.45 (m, 2H).

### Example 15

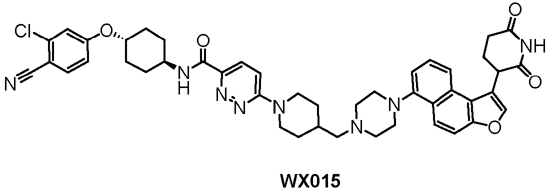

### Synthetic route:

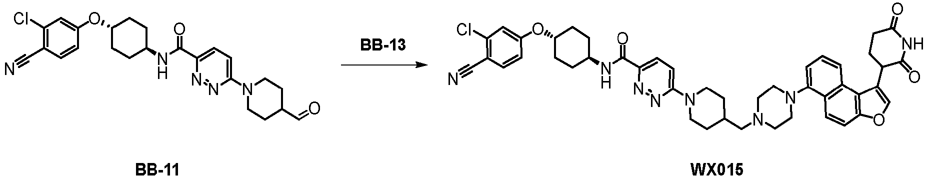

### Synthesis of hydrochloride of compound WX015

Compound BB-11 (400 mg, 854.80 µmol) and hydrochloride of compound BB-13 (403.83 mg, 1.11 mmol) were dissolved in dichloromethane (30 mL), then added with sodium triacetoxyborohydride (181.17 mg, 854.80 µmol), and the reaction mixture was stirred at room temperature for 12 hours under nitrogen atmosphere. After the reaction was completed, the reaction mixture was diluted with dichloromethane (100 mL) and water (100 mL). The organic phase was separated, dried over anhydrous sodium sulfate, filtered, and concentrated under reduced pressure. The resulting crude product was purified by preparative HPLC (mobile phase: acetonitrile/water; acidic system: 0.05% HCl) to obtain hydrochloride of compound WX015. MS-ESI m/z: 815.2 [M+H]⁺.¹H NMR (400MHz, DMSO-d6) δ: 10.96 (s, 1H), 10.82 (br s, 1H), 8.63 (d, J=8.3 Hz, 1H), 8.17 (d, J=9.3 Hz, 1H), 8.03 (s, 1H), 7.96 (br d, J=5.8 Hz, 1H), 7.86 (dt, J=9.4, 15.6 Hz, 3H), 7.60 - 7.51 (m, 2H), 7.39 (d, J=2.5 Hz, 1H), 7.27 - 7.22 (m, 1H), 7.14 (dd, J=2.3, 8.8 Hz, 1H), 4.68 (br dd, J=3.9, 11.9 Hz, 1H), 4.58 - 4.47 (m, 3H), 3.92 - 3.81 (m, 1H), 3.69 (br s, 3H), 3.50 - 3.38 (m, 3H), 3.23 - 3.06 (m, 4H), 2.98 - 2.81 (m, 1H), 2.70 - 2.58 (m, 1H), 2.45 - 2.20 (m, 4H), 2.16 - 1.99 (m, 5H), 1.91 (br d, J=10.3 Hz, 2H), 1.71 - 1.58 (m, 2H), 1.57 - 1.42 (m, 2H), 1.40 - 1.27 (m, 2H).

### Example 16 and example 17

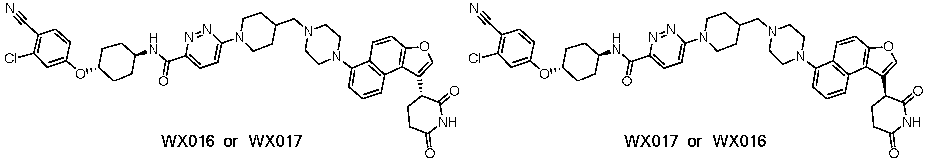

### Synthetic route:

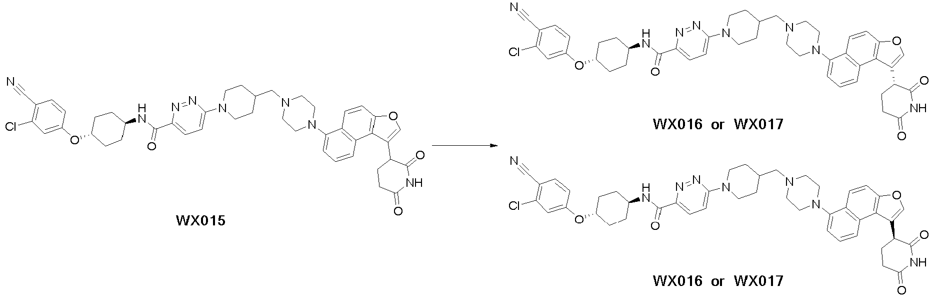

### Synthesis of compound 16 and compound 17

Compound BB-11 (7 g, 14.96 mmol) and hydrochloride of compound BB-13 (7.18 g, 17.95 mmol) were dissolved in dichloromethane (500 mL), stirred at room temperature for 12 hours, then added with sodium triacetoxyborohydride (4.76 g, 22.44 mmol), and the reaction mixture was stirred at room temperature for 12 hours under nitrogen atmosphere. After the reaction was completed, the reaction mixture was diluted with water (200 mL). The organic phase was separated, washed with saturated brine (100 mL), dried over anhydrous sodium sulfate, filtered, and concentrated under reduced pressure. The resulting crude product was separated by column chromatography (eluent: dichloromethane/methanol = 10/1) to obtain compound WX015, which was subjected to chiral resolution by HPLC (column type: DAICEL CHIRALPAK IE (250 mm * 30 mm, 10 µm); mobile phase: A (IPA), B (ACN), B%: 50% to 100%; flow rate: 80 mL/min) to obtain compounds WX016 and WX017.

Analysis method: chromatographic column: Chiralpak IA 100 * 4.6 mm, 3 µm; mobile phase: A: n-hexane (0.1% diethylamine); B: isopropanol: acetonitrile = 2:1, A:B = 40:60; column temperature: 35°C; wavelength: 220 nm.

WX016: The retention time was 4.679 min, ee%: 98.77%; MS-ESI m/z: 815.4 [M+H]⁺. ¹H NMR (400MHz, DMSO-d6) δ: 10.97 (s, 1H), 8.60 (d, J=8.3 Hz, 1H), 8.16 (d, J=9.3 Hz, 1H), 7.99 (s, 1H), 7.91 - 7.74 (m, 4H), 7.51 (t, J=7.9 Hz, 1H), 7.41 - 7.31 (m, 2H), 7.21 - 7.10 (m, 2H), 4.66 (br dd, J=4.1, 11.7 Hz, 1H), 4.58 - 4.45 (m, 3H), 3.94 - 3.79 (m, 1H), 3.223.00 (m, 7H), 2.89 (ddd, J=5.3, 12.2, 17.4 Hz, 2H), 2.69 - 2.56 (m, 2H), 2.47 - 2.33 (m, 2H), 2.29 (br d, J=7.0 Hz, 3H), 2.11 (br d, J=9.3 Hz, 2H), 2.00 - 1.81 (m, 5H), 1.72 - 1.58 (m, 2H), 1.58 - 1.44 (m, 2H), 1.26 - 1.09 (m, 2H).

WX017: The retention time was 5.797 min, ee%: 99.86%; MS-ESI m/z: 815.4 [M+H]⁺. ¹H NMR (400MHz, DMSO-d6) δ = 11.00 (br s, 1H), 8.60 (br d, J=8.3 Hz, 1H), 8.22 - 8.13 (m, 1H), 8.04 - 7.95 (m, 1H), 7.94 - 7.71 (m, 4H), 7.57 - 7.47 (m, 1H), 7.41 - 7.31 (m, 2H), 7.16 (br dd, J=8.0, 19.1 Hz, 2H), 4.66 (br d, J=7.3 Hz, 1H), 4.58 - 4.40 (m, 3H), 3.87 (br d, J=7.8 Hz, 1H), 3.28 - 2.97 (m, 7H), 2.88 (br t, J=12.3 Hz, 2H), 2.69 - 2.56 (m, 2H), 2.49 - 2.35 (m, 2H), 2.28 (br d, J=6.0 Hz, 3H), 2.17 - 2.00 (m, 2H), 2.00 - 1.79 (m, 5H), 1.71 - 1.58 (m, 2H), 1.51 (q, J=10.6 Hz, 2H), 1.26 - 1.01 (m, 2H).

### Biological data

### Test example 1: In vitro test of regulation of AR protein level and downstream effector prostate-specific antigen (PSA) protein in human prostate cancer LNCaP cells

Experimental purpose: To study the regulation of AR protein level and downstream effector prostate-specific antigen (PSA) protein in human prostate cancer LNCaP cells by compounds at different concentrations using WB (Western Blot) method.

### Experimental scheme:

1) LNCaP cells were thawed and passaged at least twice;
2) LNCaP cells were inoculated in a 6-well plate at 3 × 10⁵ cells per well, adherently cultured overnight, and treated with the test compound at a certain concentration;
3) after 24 hours of treatment, the supernatant of cultured cell samples was discarded, and the residue was washed twice with DPBS (Dulbecco's phosphate buffered saline); the cells were then lysed with a certain amount of 2% SDS lysis buffer preheated at 100°C, collected, and denatured at 100°C for 15 minutes;
4) the above lysis buffer was denatured and cooled for protein quantification (Pierce BCA protein assay kit, Thermo), then the volume was fixed with 5-fold loading buffer (containing dithiothreitol (DDT), Beyotime) according to the same protein concentration, and reduced and denatured at 100°C for 10 minutes;
5) the above samples (10 to 20 µg of protein) were separated by SDS-PAGE, and transferred to PVDF membrane (Biorad);
6) the strip was cut according to the molecular weight of the target protein and blocked with a blocking solution (3% bovine serum albumin TBS-T solution, wherein TBS-T solution was Tris-HCl buffer containing 0.2% Tween-20) for 1 hour, then incubated overnight at 4°C with primary antibodies (anti-AR (#5153, CST), anti-PSA/KLK3 (#5365, CST), and anti-β-actin (#4970, CST), diluted 1:1000, 1: 1000, and 1:2000 respectively in a blocking solution);
7) finally, it was incubated at room temperature for 1 hour with HRP-conjugated secondary antibody (anti-rabbit IgG (#7074, CST), diluted 1:2000 in a blocking solution), and the strips on the membrane were detected with chemiluminescent substrate (Clarity ECL, Biorad).

### Experimental results:

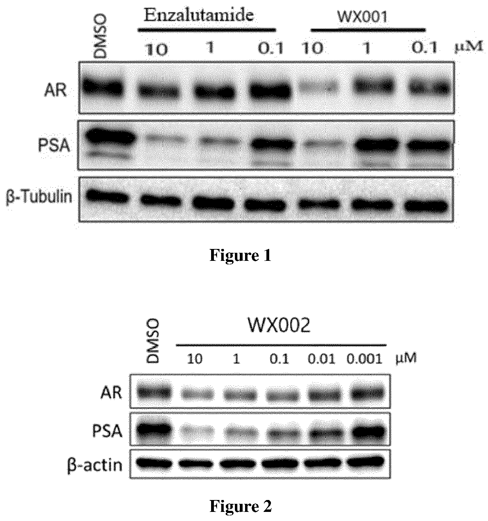

The test results are shown in Figure 1 and Figure 2.

Conclusion: The compounds of the present disclosure exhibit good degradation activity on AR protein, and their degradation ability shows good concentration dependence. The compounds of the present disclosure can significantly affect the corresponding downstream effector PSA changes, and the degree of their effect shows good concentration dependence.

### Test example 2: Evaluation of antiproliferative effects in human prostate cancer LNCaP cells

### Experimental purpose

In this experiment, the inhibitory effect of the test compound on cell proliferation in human prostate cancer LNCaP cells was detected.

### Experimental materials:

### 1. Cell line and culture method

**Table 1. Cell line and culture method**

| Cell line | Tumor type | Growth characteristics | Culture method |
|---|---|---|---|
| LNCaP | Human prostate cancer | Adherent | Phenol red-free 1640 + 10% FBS |

### 2. Culture media and reagents

**Table 2. Culture media and reagents**

| Culture media and reagents | Manufacturers | Cat. No. |
|---|---|---|
| Phenol red-free RPMI 1640 | GIBCO | 11835030 |
| Dulbecco's PBS | CORNING | 21-031-CVC |
| FBS | ExCell Bio | FSP500 |
| Penicillin-Streptomycin solution | HyClone | SV30010 |
| 0.25% Trypsin | GIBCO | 25200072 |

### 3. Multi-well plate

Greiner CELLSTAR^{®} 96-well plate, flat-bottom black plate (with lid and clear bottom), #655090.

### 4. Reagents and instruments for cell viability assay

(1) Promega CellTiter-Glo luminescent cell viability assay kit (Promega-G7573).
(2) 2104 EnVision^{®} plate reader, PerkinElmer.

### Experimental scheme:

### 1. Cell culture

The tumor cell lines were cultured in a 37°C, 5% CO₂ incubator under the above culture conditions. Regular passage, and cells in logarithmic growth phase were taken for plating.

### 2. Cell plating

(1) Cells were stained with trypan blue and viable cells were counted;
(2) the cell concentration was adjusted to an appropriate concentration;

**Table 3. Number of cells plating**

| Cell line | Density (per 96-well) |
|---|---|
| LNCaP | 4500 |

(3) 90 µL of cell suspension per well was added to the culture plate as shown in the table above, and cell-free culture medium was added to the blank control well;
(4) the culture plate was cultured overnight in a 37°C, 5% CO₂ incubator with 100% relative humidity.

### 3. Preparation of compound storage plate

Preparation of stock solution storage plate with 400 times the initial concentration of the compound: The compound was serially diluted with DMSO from the highest concentration to the lowest concentration. The solution was prepared as needed each time.

### Preparation of working solution with 10 times the initial concentration of compounds and cell treatment with compounds

(1) To a V-bottom 96-well plate was added 78 µL of cell culture medium, and 2 µL of compound was pipetted from a stock solution storage plate with 400 times the initial concentration of the compound and added to the cell culture medium of the 96-well plate. To vehicle control and blank control was added 2 µL of DMSO. After the compound or DMSO was added, blowing with a pipette and mixing well were carried out.
(2) Administration: 10 µL of a working solution with 10 times the initial concentration of the compound was added to the cell culture plate. To vehicle control and blank control was added 10 µL of DMSO-cell culture medium mixture.
(3) The 96-well plate was returned to the incubator and cultured for 6 days.

### 5. CellTiter-Glo luminescent cell viability assay

The following steps were performed according to the instructions of Promega CellTiter-Glo luminescent cell viability assay kit (Promega-G7573).
(1) CellTiter-Glo buffer was melted and brought to room temperature;
(2) CellTiter-Glo substrate was brought to room temperature;
(3) CellTiter-Glo working solution was prepared by adding 100 mL of CellTiter-Glo buffer to a vial of CellTiter-Glo substrate to dissolve the substrate;
(4) the mixture was slowly vortexed and shaken to be fully dissolved;
(5) the cell culture plate was taken out and left for 30 minutes to equilibrate to room temperature;
(6) 50 µL of CellTiter-Glo working solution was added per well (equal to half the volume of cell culture medium per well). The cell plate was wrapped in aluminum foil to be protected from light;
(7) the culture plate was shaken on an orbital shaker for 2 minutes to induce cell lysis;
(8) the culture plate was left at room temperature for 10 minutes to stabilize the luminescent signal;
(9) the luminescent signal was detected on a 2104 EnVision plate reader.

### 6. Data analysis

The inhibition rate (IR) of the test compound was calculated using the following formula: IR (%) = (RLU vehicle control - RLU compound) / (RLU vehicle control - RLU blank control) × 100%. The inhibition rates of compounds at different concentrations were calculated in Excel, and then GraphPad Prism software was used to plot inhibition curves and calculate relevant parameters, including minimum inhibition rate, maximum inhibition rate, and IC₅₀.

### Experimental results:

The test results are shown in Table 4.

**Table 4. Inhibitory effect of compounds of the present disclosure on cell proliferation in LNCaP cell lines**

| Compound | LNCaP IC₅₀ (nM) |
|---|---|
| WX001 | 332.6 |
| WX002 | 46 |
| WX003 | 230 |
| WX004 | 25 |
| WX005 | 190 |
| Hydrochloride of WX015 | 45 |
| WX016 | 31 |
| WX017 | 44 |

| | |
|---|---|
| Conclusion: The compounds of the present disclosure exhibit excellent inhibitory effect on cell proliferation in human prostate cancer LNCaP cells. | |

### Test example 3: In vivo pharmacodynamic study of subcutaneous xenograft tumor CB-17 SCID model of human prostate cancer LNCaP cells

### Cell culture

Human prostate cancer LNCaP cells (ECACC-89110211) were cultured *in vitro* in a monolayer under the culture conditions of RPMI-1640 medium with 10% fetal bovine serum, 100 U/mL penicillin, and 100 µg/mL streptomycin, and cultured in a 37°C, 5% CO₂ incubator. Routine digestion with trypsin-EDTA was performed twice a week for passaging. When the cell saturation density was 80% to 90% and the number of cells reached the required level, the cells were collected, counted, and inoculated.

### Experimental animals

CB-17 SCID mice, 6 to 8 weeks old, male, weighing 18 to 22 g.

### Experimental scheme

0.2 mL (1 × 10⁷) of LNCaP cells (with matrix gel, volume ratio of 1:1) were subcutaneously inoculated on the right back of each mouse. The mice were subjected to surgical castration when the average tumor volume reached approximately 80 mm³, and grouping and administration were initiated when the average tumor volume reached 166 mm³. Experimental animal grouping and administration regimen are shown in Table 5.

**Table 5. Experimental animal grouping and administration regimen**

| Group | N1 | Treatment with compound | Dose (mg/kg) | Administration volume parameter (µL/g)2 | Route of administration | Frequency of administration |
|---|---|---|---|---|---|---|
| 1 | 6 | Vehicle group (10% DMSO/10% Solutol | -- | 10 | p.o. | BID |
| 2 | 6 | Enzalutamide | 20 | 10 | p.o. | QD |
| 3 | 6 | WX002 | 50 | 10 | p.o. | BID |

| | | | | | | |
|---|---|---|---|---|---|---|
| Note: 1. N: Number of mice per group 2. Administration volume: 10 µL/g based on mouse body weight. If weight loss exceeds 15%, the administration regimen should be adjusted accordingly. 3. BID interval was 8 hours. | | | | | | |

### Tumor measurement and experimental indicators

Tumor diameters were measured twice a week with a vernier caliper. The formula for calculating tumor volume was: V = 0.5a × b², wherein a and b represented the long and short diameters of the tumor, respectively.

The antitumor efficacy of compounds was evaluated by TGI (%). TGI (%) reflected the tumor growth inhibition rate. TGI (%) = [(1 - (average tumor volume at the end of administration in a treatment group - average tumor volume at the beginning of administration in this treatment group)) / (average tumor volume at the end of treatment in the vehicle control group - average tumor volume at the beginning of treatment in the vehicle control group)] × 100%.

### Experimental results:

The test results are shown in Table 6.

**Table 6. Antitumor effect of compounds on LNCaP xenograft tumor model**

| Group | Tumor volume (mm³) | Tumor volume (mm³) | TGI (%) |
|---|---|---|---|
| | (Day 0) | (Day 21) | (Day 21) |
| Vehicle group | 166 ± 24 | 653 ± 114 | N/A |
| Enzalutamide (20 mg/kg) | 166 ± 18 | 157 ± 37 | 101.9 |
| WX002 (30 mg/kg (D0-D3), 50 mg/kg (D4-D21)) | 166 ± 18 | 219 ± 39 | 89.1 |

| | | | |
|---|---|---|---|
| Conclusion: The compounds of the present disclosure exhibit significant antitumor effect on human prostate cancer LNCaP xenograft tumor model. | | | |

### Test example 4: In vitro test of regulation of AR protein level in human prostate cancer LNCaP cells

Experimental purpose: To test the degradation effect of compounds on androgen receptor AR in LNCaP cells by ICW method.

### Experimental method:

### Day 1

1. The cell culture medium, 0.025% trypsin, and phosphate buffered saline were preheated in a 37°C water bath.
2. Cells digested by trypsin were centrifuged at 1000 rpm for 5 minutes. The supernatant was discarded, and then the cells were resuspended in fresh culture medium.
3. The cells were inoculated in a COL-Coated CellCarrier-384 plate at a density of 4k/36 µL/well.
4. The 384 cell plate inoculated with cells was placed in an ultra-clean bench for 10 minutes.
5. The cell plate was cultured overnight in a CO₂ cell incubator.

### Day 2

1. The compound at a concentration of 10 mM was diluted to 3 mM (3 µL + 7 µL of dimethyl sulfoxide) before serial dilution.
2. The compound was serially diluted 3-fold (4 µL + 8 µL) in an Echo plate using Bravo.
3. ZPE and HPE control wells were set up, and the ZPE control wells were dimethyl sulfoxide.
4. The Echo plate was centrifuged at 1000 rpm for 1 minute, and then the diluted compound was transferred from the Echo plate to an intermediate plate using Echo with 200 nL/well.
5. To the intermediate plate was added the cell culture medium using Multidrop combi with 20 µL/well.
6. The intermediate plate was shaken on a horizontal shaker for 1 minute to mix the compound well with the culture medium.
7. The compound was transferred from the intermediate dilution plate to the cell plate using Bravo with 4 µL/well.
8. The cell plate was shaken on the horizontal shaker for 1 minute, and centrifuged at 800 rpm for 30 seconds.
9. The cell plate was returned to the CO₂ incubator, and cultured for another 24 hours.

### Day 3

1. 4% paraformaldehyde was removed from the refrigerator and brought to room temperature.
2. The cell plate was removed from the incubator.
3. To the cell plate was directly added 4% paraformaldehyde with 40 µL/well.
4. The plate was incubated at room temperature for 20 minutes.
5. The culture medium was manually shaken off from the cell plate, inverted in a centrifuge, and centrifuged at 800 rpm for 7 seconds to fully remove the residual culture medium.
6. To the cell plate was added 4% paraformaldehyde with 25 µL/well.
7. The plate was incubated at room temperature for 30 minutes.
8. The paraformaldehyde solution was manually shaken off from the cell plate, inverted in the centrifuge, and centrifuged at 800 rpm for 7 seconds.
9. 0.1% Trition was added with 25 µL/well.
10. The plate was incubated at room temperature for 10 minutes.
11. The 0.1% Trition solution was manually shaken off from the cell plate, inverted in the centrifuge, and centrifuged at 800 rpm for 7 seconds.
12. Intercept^{®} (PBS) blocking buffer was added with 25 µL/well.
13. The plate was incubated at room temperature for 1 hour.
14. The Intercept^{®} (PBS) blocking buffer solution was manually shaken off from the cell plate, inverted in the centrifuge, and centrifuged at 800 rpm for 7 seconds.
15. Primary antibody solution was added with 20 µL/well.The cell plate was placed in the centrifuge, and centrifuged at 800 rpm for 7 seconds.
16. The cell plate was sealed, and then incubated overnight at 4°C.

### Day 4

1. The primary antibody solution was manually shaken off from the cell plate, inverted in the centrifuge, and centrifuged at 800 rpm for 7 seconds.
2. The plate was washed three times with 110 µL of PBS.
3. The cell plate was inverted in the centrifuge, and centrifuged at 800 rpm for 7 seconds.
4. Secondary antibody and DAPI solution was added with 20 µL/well. The cell plate was placed in the centrifuge, and centrifuged at 800 rpm for 7 seconds.
5. The plate was incubated at room temperature for 1 hour.
6. The secondary antibody and DAPI solution was manually shaken off from the cell plate, inverted in the centrifuge, and centrifuged at 800 rpm for 7 seconds.
7. The cell plate was washed three times with 110 µL of PBS, and 30 µL of PBS remained in the plate.
8. The cell plate was placed in the centrifuge, and centrifuged at 800 rpm for 7 seconds.
9. The cell plate was scanned using Operetta.

### Experimental results

The test results are shown in Table 7.

**Table 7. Degradation effect of compounds on androgen receptor AR in LNCaP cells**

| # | Compound No. | DC50 (nM) | Dmax |
|---|---|---|---|
| 1 | WX002 | 8.9 | 72.01 |
| 2 | WX009 | 10.1 | 64.66 |
| 3 | Hydrochloride of WX014 | 22.4 | 76.22 |
| 4 | Hydrochloride of WX015 | 13.7 | 102.09 |
| 5 | WX016 | 14.0 | 95.66 |
| 6 | WX017 | 15.1 | 100.32 |

| | | | |
|---|---|---|---|
| Conclusion: The compounds of the present disclosure exhibit good degradation activity on AR. | | | |

### Test example 5: In cell western analysis of the expression level of AR protein in 293T AR F876L cells

Experimental purpose: In this experiment, the effects of compounds WX015, WX016, and WX017 on the expression level of AR protein with point mutation in cell line 293T AR F876L were detected by in cell western assay to evaluate the degradation effect of compounds on proteins with AR F876L point mutation.

### Experimental materials:

### 1. Cell line and culture method

**Table 8. Cell line and culture method**

| Cell line | Cell type | Growth characteristics | Culture method |
|---|---|---|---|
| 293T AR F876L | Embryo kidney cell | Adherent growth | DMEM + 10% FBS + 1% AA |

### 2. Culture media and reagents

**Table 9. Culture media and reagents**

| Culture media and reagents | Manufacturers | Cat. No. |
|---|---|---|
| DMEM | GIBCO | 11995-065 |
| PBS | Cytiva | SH30256.01 |
| FBS | Hyclone | SH30084.03 |
| Antibiotic-antimycotic | GIBCO | 15240-062 |
| 0.25% Trypsin | GIBCO | 25200072 |
| DMSO | SIGMA | D2650 |

### 3. Instruments

**Table 10. Instruments**

| Instruments | Supplier | Catalog # |
|---|---|---|
| Odyssey Gel Imaging System | LI-COR | CLx |
| CO₂ incubator | Thermo Scientific | Heracell 2401 |
| Automated cell counter | Countstar | IC-1000 |
| Inverted microscope | Nikon | ECLIPSE Ts2 |
| Biological safety cabinet | AIRTECH | BSC-1600 A3 |
| Decolorization shaker | Hm-Kylin | TS-1 |

### 4. Antibodies

**Table 11. Antibodies**

| Antibodies | Supplier & Cat. No. |
|---|---|
| Anti-mouse IgG (H+L) (DyLight^{™} 680 Conjugate) | CST-5470S |
| Androgen receptor (D6F11) XP^{®} rabbit mAb | CST-5153 |

### Experimental method:

### 1. Cell culture:

The cell line was cultured in a 37°C, 5% CO₂ incubator. Regular passage, and cells in logarithmic growth phase were taken for plating.

### 2. Cell plating:

1) Cells were stained with trypan blue and viable cells were counted.
2) The cell concentration was adjusted to an appropriate concentration with a density of 20000/well.
3) 90 µL of cell suspension per well was added to a 96-well culture plate, and cell-free culture medium was added to the blank control well.
4) The culture plate was cultured overnight in a 37°C, 5% CO₂ incubator with 100% relative humidity.

### 3. Preparation of compound storage plate and in cell western detection:

1) Preparation of 400X compound storage plate: The compound was serially diluted with DMSO from the highest concentration to the lowest concentration.
2) Preparation of 10X compound working solution: To a V-bottom 96-well plate was added 78 µL of cell culture medium, and 2 µL of compound was pipetted from a 400X compound storage plate and added to the cell culture medium of the 96-well plate. 2 µL of DMSO was added to vehicle control and blank control. After the compound or DMSO was added, blowing with a pipette and mixing well were carried out.
3) Administration: 10 µL of 10X compound working solution was added to the cell culture plate. To vehicle control and blank control was added 10 µL of DMSO-cell culture medium mixture. The final concentration of DMSO was 0.25%.
4) The 96-well cell plate was returned to the incubator and cultured for 48 hours.
5) The culture medium was removed, and the plate was washed once with PBS;
6) the plate was added with 8% formalin solution (diluted in PBS), and incubated overnight at 4°C.
7) 8% formalin solution was removed. The plate was added with 150 µL of Licor blocking buffer per well, and blocked on a shaker at room temperature for 1.5 hours.
8) The blocking solution was poured off. The plate was added with 50 µL of primary antibody diluted with Licor blocking buffer per well (no primary antibody was added to DMSO wells), incubated overnight at 4°C, and then washed five times with PBST (containing 0.1% Triton-X) for 5 minutes each time.
9) The plate was added with 50 µL of secondary antibody diluted with Licor blocking buffer per well, incubated at room temperature for 1 hour, washed three times with PBST (containing 0.1% Triton-X), washed twice with ddH2O, and then added with 100 µL of PBS per well for detection.
10) Detection: PBS was poured out of the well plate, and the fluorescence signal at 700 nm per well was detected by Li-COR odyssey two-color near-infrared imager.

### 4. Data analysis:

Relative quantification of the density intensity of immunoblot chemiluminescence bands was performed using Image Studio Ver 5.2 software.

### Experimental results

The test results are shown in Table 12.

**Table 12. Degradation effect of compounds on androgen receptor AR in 293T AR F876L cells**

| # | Compound No. | DC50 (nM) | Dmax |
|---|---|---|---|
| 1 | Hydrochloride of WX015 | 43 | 85 |
| 2 | WX016 | 18 | 86 |
| 3 | WX017 | 97 | 95 |

| | | | |
|---|---|---|---|
| Conclusion: The compounds of the present disclosure have a strong ability to degrade AR protein with F876L point mutation. | | | |

### Test example 6: Pharmacokinetic evaluation of compounds in mice

### Experimental purpose:

In this study, CB-17 SCID male mice were selected as experimental animals for the test compounds, and LCMS/MS method was used to quantify plasma concentrations of the test compound and the reference compound intravenously injected or orally administered to mice at different time points, so as to evaluate the pharmacokinetic profile of the test drug in mice.

### Experimental materials:

CB-17 SCID mice (male, 20 to 30 g, 7 to 10 weeks old, Beijing Vital River or Shanghai SLAC).

### Experimental operation:

A clarified solution or suspension of the test compound was injected into mice via the tail vein (without fasting), or intragastrically administered to mice (without fasting). For intravenous injection, blood was collected via the jugular vein puncture at 0 h (pre-administration), 0.083 h, 0.25 h, 0.5 h, 1 h, 2 h, 4 h, 8 h, and 24 h after administration, and placed in an anticoagulant tube added with EDTA-K2. The mixture was thoroughly vortexed and mixed, and centrifuged at 13000 rpm for 10 minutes at 4°C. For oral administration, blood was collected via the jugular vein puncture at 0 h (pre-administration), 0.5 h, 1 h, 2 h, 4 h, 6 h, 8 h, and 24 h after administration, and placed in an anticoagulant tube added with EDTA-K2. The mixture was thoroughly vortexed and mixed, and centrifuged at 13000 rpm for 10 minutes. Plasma concentrations were determined by LC-MS/MS method, and relevant pharmacokinetic parameters were calculated using WinNonlin^{™} Version 6.3 (Pharsight, Mountain View, CA) pharmacokinetic software with non-compartmental model linear-log trapezoidal method.

Experimental results: The test results are shown in Table 13.

**Table 13. Pharmacokinetic parameters of compounds of the present disclosure in mice**

| Pharmacokinetic parameters in mice | Intravenous injection (1 mg/mL) | | | Oral administration (10 mg/mL) | | | |
|---|---|---|---|---|---|---|---|
| | Plasma clearance rate (mL/min/kg) | Half-life (h) | Area under the drug-time curve (0-last, h*nmol/L) | Peak concentration (nmol/L) | Time to peak concentration (h) | Area under the drug-time curve (0-last, h*nmol/L) | Bioavailability F (%) |
| WX016 | 1.41 | 31.4 | 6192 | 1770 | 24 | 30541 | 49.3 |
| WX017 | 3.92 | 8.31 | 4651 | 1227 | 24 | 22390 | 48.1 |

| | | | | | | | |
|---|---|---|---|---|---|---|---|
| Conclusion: The compounds of the present disclosure exhibit good druggability in mice. | | | | | | | |

### Test example 7: Pharmacokinetic evaluation of compounds in rats

### Experimental purpose:

In this study, SD male rats were selected as experimental animals for the test compounds, and LCMS/MS method was used to quantify plasma concentrations of the test compound and the reference compound intravenously injected or orally administered to rats at different time points, so as to evaluate the pharmacokinetic profile of the test drug in rats.

### Experimental materials:

### SD rats (male, Beijing Vital River).

### Experimental operation:

A clarified solution or suspension of the test compound was injected into rats via the tail vein (without fasting), or intragastrically administered to rats (without fasting). For intravenous injection, blood was collected via the jugular vein puncture at 0 h (pre-administration), 0.25 h, 0.5 h, 1 h, 2 h, 4 h, 6 h, 8 h, 24 h, and 48 h after administration, and placed in an anticoagulant tube added with heparin sodium. The collected blood sample was placed on ice, and centrifuged to separate plasma within 1 hour (centrifugation conditions: 6000 g, 3 minutes, 2 to 8°C). For oral administration, blood was collected via the jugular vein puncture at 0 h (pre-administration), 0.25 h, 0.5 h, 1 h, 2 h, 4 h, 6 h, 8 h, 24 h, and 48 h after administration, and placed in an anticoagulant tube added with heparin sodium. The collected blood sample was placed on ice, and centrifuged to separate plasma within 1 hour (centrifugation conditions: 6000 g, 3 minutes, 2 to 8°C). Plasma concentrations were determined by LC-MS/MS method, and relevant pharmacokinetic parameters were calculated using WinNonlin^{™} Version 8.2.0 (Pharsight, Mountain View, CA) pharmacokinetic software with non-compartmental model linear-log trapezoidal method.

Experimental results: The test results are shown in Table 14.

**Table 14. Pharmacokinetic parameters of compounds of the present disclosure in rats**

| Pharmacokinetic parameters in rats | Intravenous injection (2 mg/mL) | | | Oral administration (10 mg/mL) | | | |
|---|---|---|---|---|---|---|---|
| | Plasma clearance rate (mL/min/kg) | Half-life (h) | Area under the drug-time curve (0-last, h*nmol/L) | Peak concentration (nmol/L) | Time to peak concentration (h) | Area under the drug-time curve (0-last, h*nmol/L) | Bioavailability F (%) |
| WX016 | 4.71 | 21.2 | 6949 | 451 | 13.3 | 14716 | 42.35 |
| WX017 | 6.86 | 10.2 | 5846 | 349 | 7.33 | 7420 | 25.38 |

| | | | | | | | |
|---|---|---|---|---|---|---|---|
| Conclusion: The compounds of the present disclosure exhibit good druggability in rats. | | | | | | | |

### Test example 8: Pharmacokinetic evaluation of compounds in Beagles

### Experimental purpose:

In this study, Beagles were selected as experimental animals for the test compounds, and LCMS/MS method was used to quantify plasma concentrations of the test compound and the reference compound intravenously injected or orally administered to Beagles at different time points, so as to evaluate the pharmacokinetic profile of the test drug in Beagles.

### Experimental materials:

Beagles (male, Jiangsu Yadong Institute of Experimental Animals Co., Ltd.).

### Experimental operation:

A clarified solution or suspension of the test compound was injected into Beagles via the tail vein (normal feeding), or intragastrically administered to Beagles (normal feeding). For intravenous injection, blood was collected via the forelimb vein puncture at 0 h (pre-administration), 0.25 h, 0.5 h, 1 h, 2 h, 4 h, 6 h, 8 h, 24 h, and 48 h after administration, and placed in an anticoagulant tube added with EDTA-K2. The collected blood sample was placed on ice, and centrifuged to separate plasma within 1 hour (centrifugation conditions: 6000 g, 3 minutes, 2 to 8°C). For oral administration, blood was collected via the forelimb vein puncture at 0 h (pre-administration), 0.25 h, 0.5 h, 1 h, 2 h, 4 h, 6 h, 8 h, 24 h, 48 h after administration, and placed in an anticoagulant tube added with EDTA-K2. The collected blood sample was placed on ice, and centrifuged to separate plasma within 1 hour (centrifugation conditions: 6000 g, 3 minutes, 2 to 8°C). Plasma concentrations were determined by LC-MS/MS method, and relevant pharmacokinetic parameters were calculated using WinNonlin^{™} Version 8.2.0 (Pharsight, Mountain View, CA) pharmacokinetic software with non-compartmental model linear-log trapezoidal method.

Experimental results: The test results are shown in Table 15.

**Table 15. Pharmacokinetic parameters of compounds of the present disclosure in Beagles**

| Pharmacokinetic parameters in Beagles | Intravenous injection (0.5 mg/mL) | | Oral administration (3 mg/mL) | | | |
|---|---|---|---|---|---|---|
| | Plasma clearance rate (mL/min/kg) | Area under the drug-time curve (0-last, h*nmol/L) | Peak concentration (nmol/L) | Time to peak concentration (h) | Area under the drug-time curve (0-last, h*nmol/L) | Bioavailability F (%) |
| WX016 | 0.66 | 2641 | 175 | 6 | 5442 | 34.3 |
| WX017 | 5.3 | 1713 | 178 | 4.67 | 4808 | 46.8 |

| | | | | | | |
|---|---|---|---|---|---|---|
| **[0416]** Conclusion: The compounds of the present disclosure exhibit good druggability in Beagles. | | | | | | |

## Claims

1. A compound of formula (II) or a pharmaceutically acceptable salt thereof, 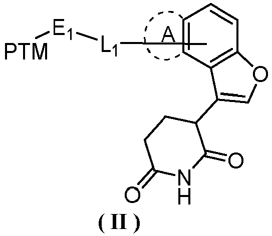 wherein
PTM is selected from 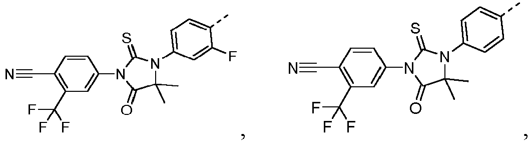 and 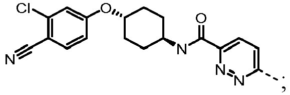
the structural moiety -E₁-L₁- is selected from 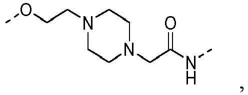 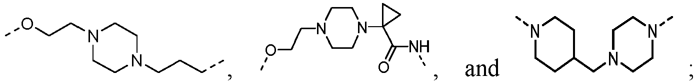
ring A is absent;
or ring A is selected from phenyl and 5-membered heteroaryl;
the 5-membered heteroaryl contains 1, 2, or 3 heteroatoms or heteroatom groups independently selected from -NH-, -O-, -S-, and N, respectively.

2. The compound or the pharmaceutically acceptable salt thereof according to claim 1, wherein the structural moiety 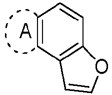 is selected from 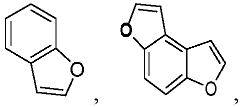 and

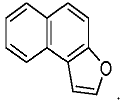

3. The compound or the pharmaceutically acceptable salt thereof according to claim 1, wherein the compound is selected from: 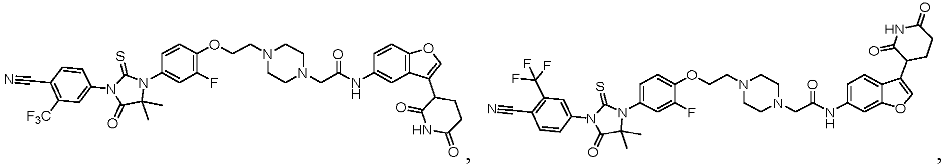 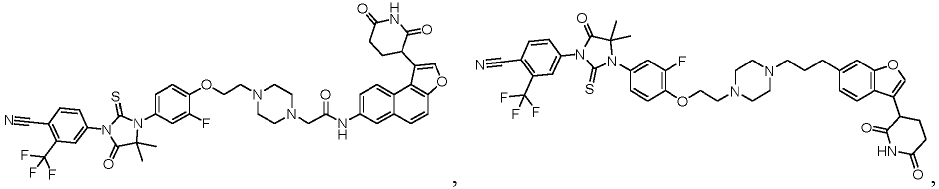 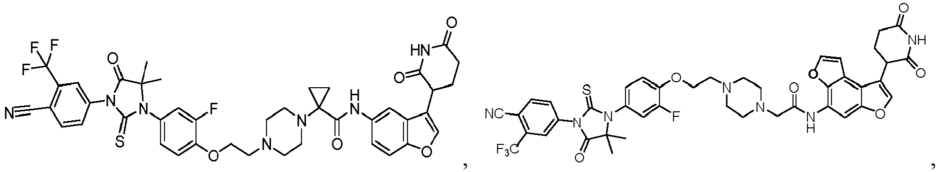 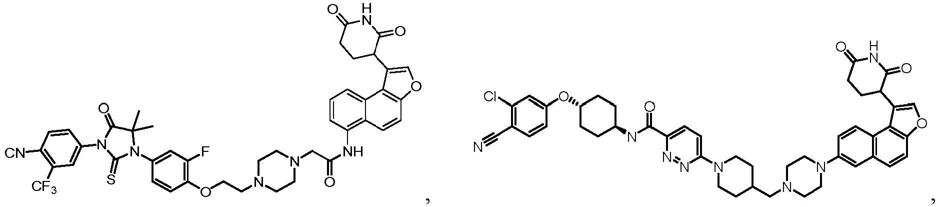 and

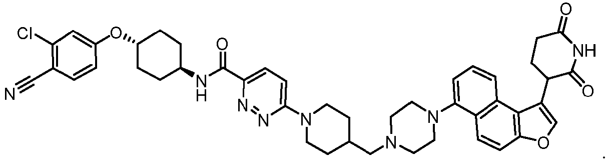

4. The compound or the pharmaceutically acceptable salt thereof according to claim 1, wherein the compound is selected from:

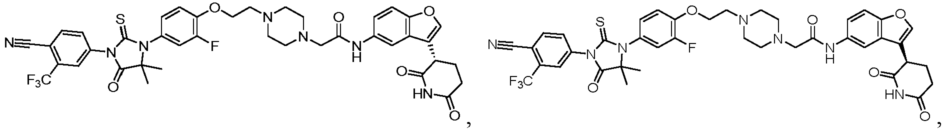

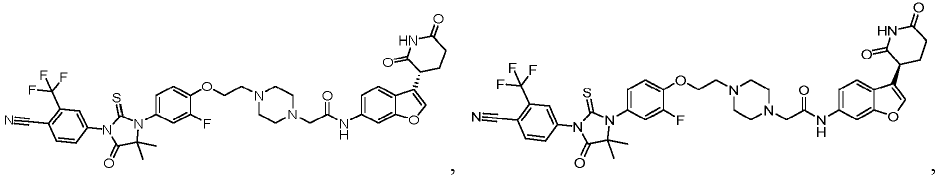

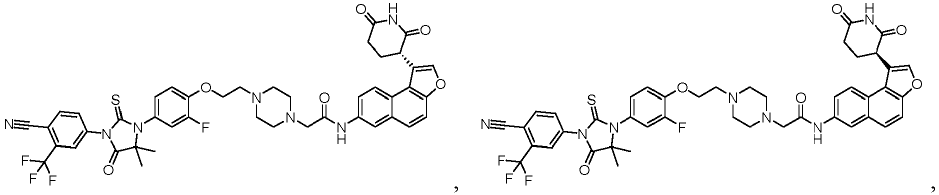

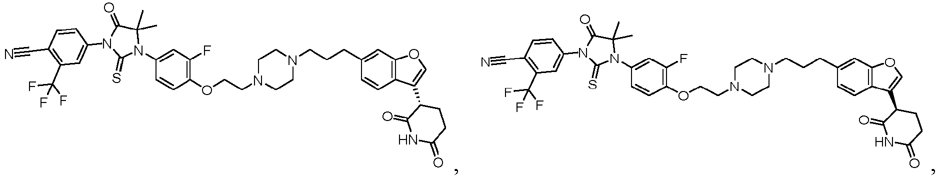

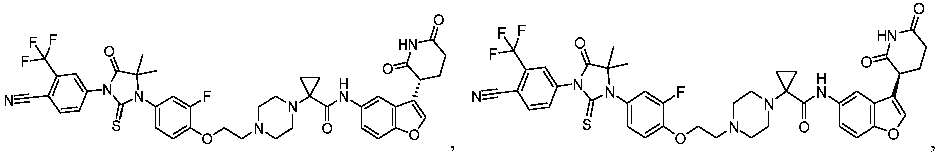

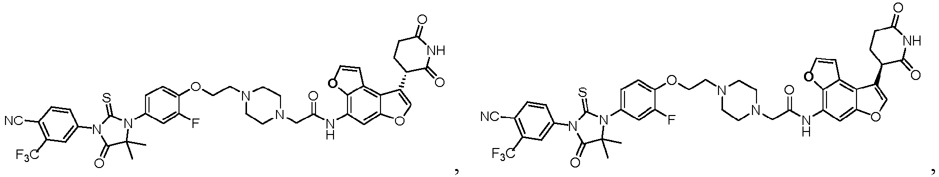

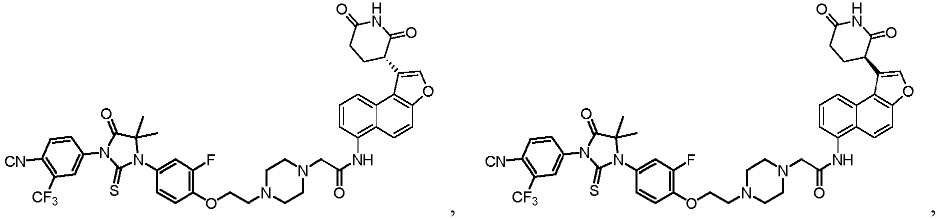

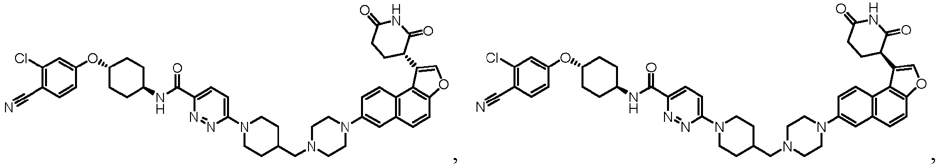

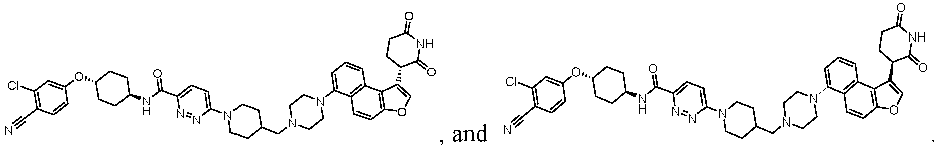

5. The compound or the pharmaceutically acceptable salt thereof according to any one of claims 1 to 4 for use in treating prostate cancer.

## Patentansprüche

1. Verbindung der Formel (II) oder pharmazeutisch akzeptables Salz davon, 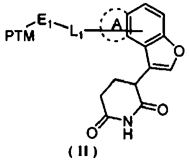 wobei
PTM ausgewählt ist aus 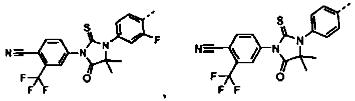 und 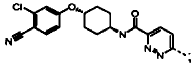
wobei die strukturelle Einheit -E₁-L₁- ausgewählt ist aus 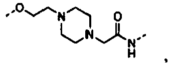 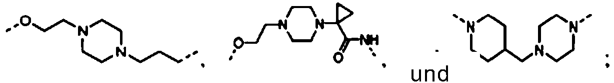
Ring A nicht vorhanden ist;
oder Ring A ausgewählt ist aus Phenyl und einem 5-gliedrigen Heteroaryl;
das 5-gliedrige Heteroaryl 1, 2 oder 3 Heteroatome oder Heteroatomgruppen enthält, die unabhängig voneinander aus -NH-, -O-, -S- bzw. N ausgewählt sind.

2. Verbindung oder das pharmazeutisch akzeptable Salz davon nach Anspruch 1, wobei die strukturelle Einheit 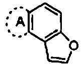 ausgewählt ist aus 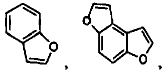 und

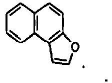

3. Verbindung oder das pharmazeutisch akzeptable Salz davon nach Anspruch 1, wobei die Verbindung ausgewählt ist aus: 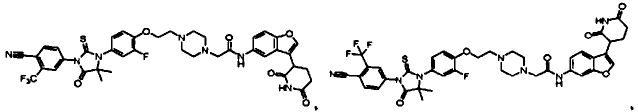 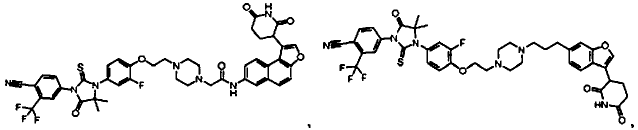 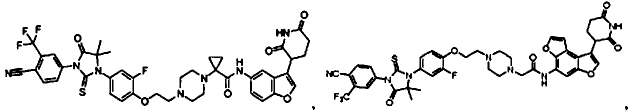 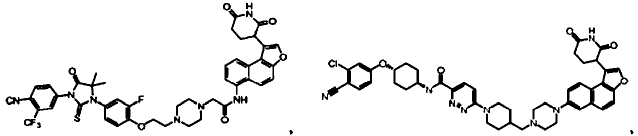 und

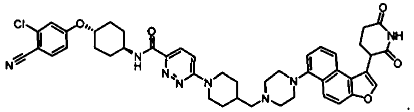

4. Verbindung oder das pharmazeutisch akzeptable Salz davon nach Anspruch 1, wobei die Verbindung ausgewählt ist aus:

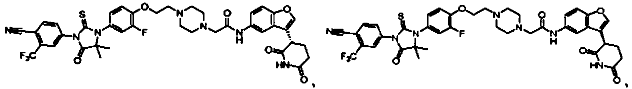

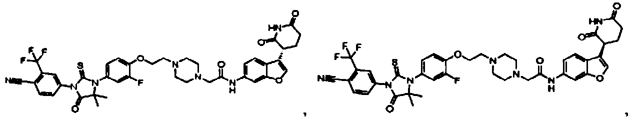

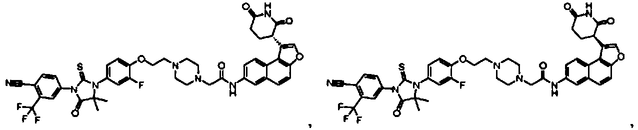

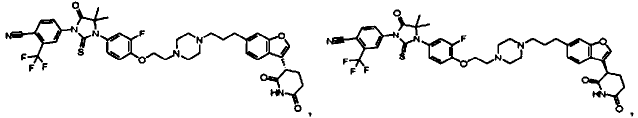

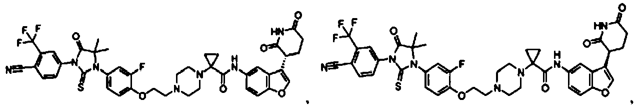

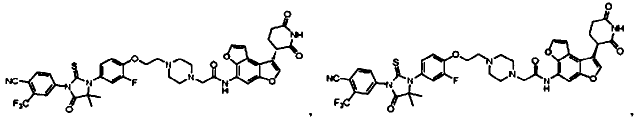

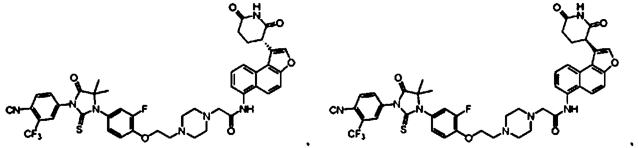

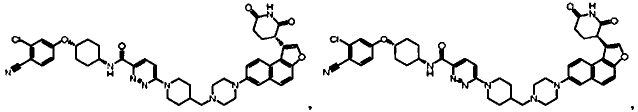

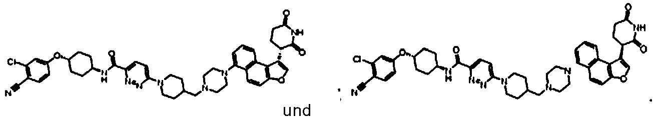

5. Verbindung oder pharmazeutisch akzeptables Salz davon nach einem der Ansprüche 1 bis 4 zur Verwendung bei der Behandlung von Prostatakrebs.

## Revendications

1. Composé de formule (II) ou sel pharmaceutiquement acceptable de celui-ci, 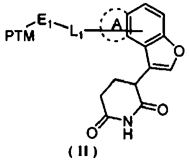 dans lequel
PTM est sélectionné parmi 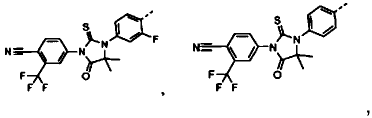 et 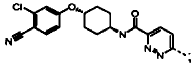
la fraction structurale -E₁-L₁- est sélectionnée parmi 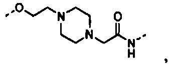 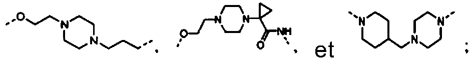
le cycle A est absent ;
ou le cycle A est sélectionné parmi phényle et hétéroaryle à 5 éléments ;
l'hétéroaryle à 5 éléments contient 1, 2 ou 3 hétéroatomes ou groupes d'hétéroatomes indépendamment sélectionnés parmi -NH-, -O-, -S- et N, respectivement.

2. Composé ou sel pharmaceutiquement acceptable de celui-ci selon la revendication 1, dans lequel la fraction structurale 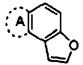 est sélectionnée parmi

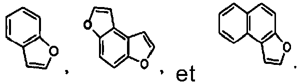

3. Composé ou sel pharmaceutiquement acceptable de celui-ci selon la revendication 1, dans lequel le composé est sélectionné parmi : 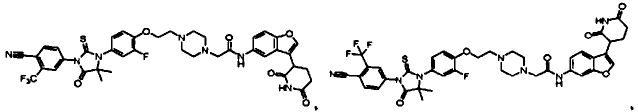 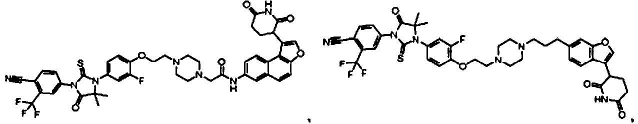 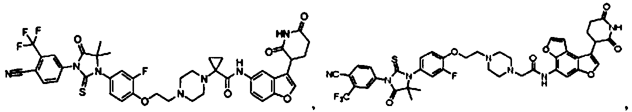 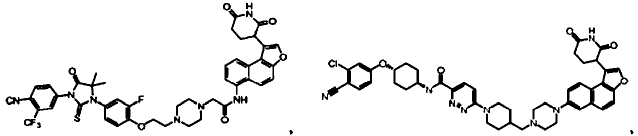 et

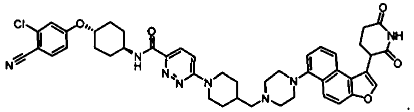

4. Composé ou sel pharmaceutiquement acceptable de celui-ci selon la revendication 1, dans lequel le composé est sélectionné parmi :

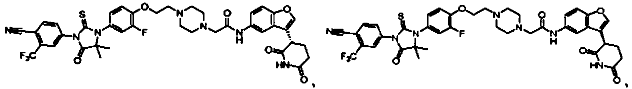

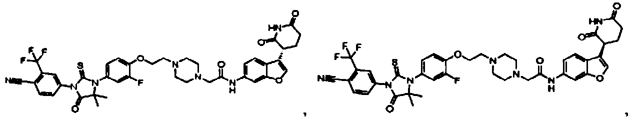

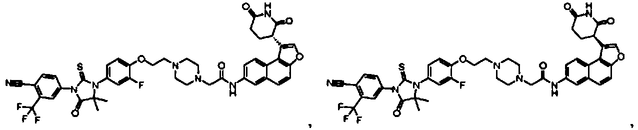

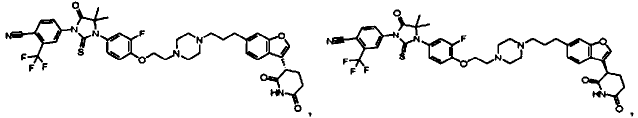

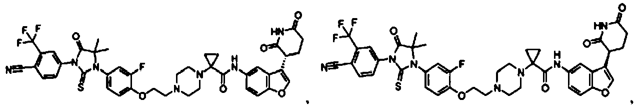

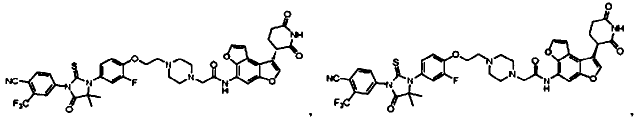

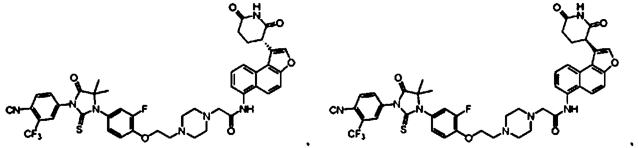

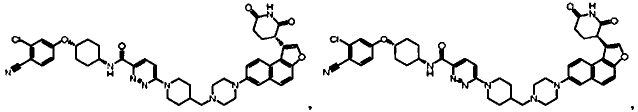

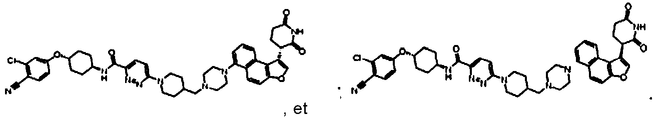

5. Composé ou sel pharmaceutiquement acceptable de celui-ci selon l'une quelconque des revendications 1 à 4 pour une utilisation dans le traitement d'un cancer de la prostate.
